# FASCICULE DE BREVET EUROPEEN

(11) **EP 1 532 134 B1**
(45) Date de publication et mention de la délivrance du brevet: **04.06.2008**
(21) Numéro de dépôt: 02807597.6
(22) Date de dépôt: 04.07.2002
(51) Int. Cl.: C07D 333/38, A61K 31/381, A61P 19/08

(54) **NOUVEAUX DERIVES ACYL HYDRAZINO DU THIOPHENE, LEUR PROCEDE DE PREPARATION, LEUR APPLICATION A TITRE DE MEDICAMENTS, COMPOSITIONS PHARMACEUTIQUES ET NOUVELLE UTILISATION**
NEUE THIOPHENACYLHYDRAZINODERIVATE, VERFAHREN ZU DEREN HERSTELLUNG, DEREN VERWENDUNG ALS ARZNEIMITTEL, PHARMAZEUTISCHE ZUSAMMENSETZUNGEN UND NEUE ANWENDUNGEN
NOVEL THIOPHENE ACYL HYDRAZINO DERIVATIVES, METHOD FOR PREPARING SAME, USE THEREOF AS MEDICINES, PHARMACEUTICAL COMPOSITIONS AND NOVEL USE

(43) Date de publication de la demande: 25.05.2005
(73) Titulaire: Aventis Pharma S.A., 92160 Antony (FR)
(72) Inventeur: BENARD, Didier, F-95560 Montsoult (FR); GOURVEST, Jean-François, F-77410 Claye-Souilly (FR)
(86) Numéro de dépôt international: PCT/FR2002/002335
(87) Numéro de publication internationale: WO 2004/007477

(56) Documents cités:
- EP-A- 0 261 755
- WO-A-00/00465
- WO-A-98/48799
- WO-A-99/59570
- GB-A- 2 195 634
- PESCHKE B ET AL: "Aminomethylthiophene-2-carboxylic Acids as Dipeptide Mimetic in New Growth Hormone Secretagogues" BIOORGANIC & MEDICINAL CHEMISTRY LETTERS, OXFORD, GB, vol. 7, no. 15, 5 août 1997 (1997-08-05), pages 1969-1972, XP004136368 ISSN: 0960-894X
- PARRA M ET AL: "SYNTHESIS AND MESOMORPHIC PROPERTIES OF AZO COMPOUNDS DERIVED FROM PHENYL- AND THIENYL-1,3,4-THIADIAZOLE" ZEITSCHRIFT FUR NATURFORSCHUNG, TEIL B: ANORGANISCHE CHEMIE, ORGANISCHE CHEMIE, VERLAG DER ZEITSCHRIFT FUR NATURFORSCHUNG. TUBINGEN, DE, vol. 52, no. 12, 1 décembre 1997 (1997-12-01), pages 1533-1538, XP000727157 ISSN: 0932-0776
- KRAYUSHKIN M.M. ET AL.: "Thermally Reversible Photochromic Dithienylethenes" INTERNATIONAL JOURNAL OF PHOTOENERGY, vol. 1, no. 3, 1999, pages 183-190, XP009003579
- Y.L. GOL'DFARB ET AL.: "Reactions of Aromatic and Heteroaromatic Compounds Carrying Electron-Acceptor substituents. 26. Acylaminomethylation of 2-Acylthiophenes, 2-Thiophenecarboxylic Acid, and its Esters" CHEM. HETEROCYCL. COMPD, vol. 22, no. 6, 1986, pages 625-629, XP009003553

## Description

La présente invention concerne de nouveaux dérivés acyl hydrazino du thiophène, leur procédé de préparation, les nouveaux intermédiaires obtenus, leur application à titre de médicaments, les compositions pharmaceutiques les renfermant et la nouvelle utilisation de tels dérivés acyl hydrazino du thiophène

L'invention a ainsi pour objet de nouveaux dérivés acyl hydrazino du thiophène possédant des propriétés inhibitrices d'enzymes métaboliques. De telles enzymes métaboliques sont notamment des kinases ou des protéases et notamment des cystéines protéases ou des sérine protéases.

On peut rappeler qu'on a constaté que des cystéines protéases de crustacés induisent chez les vertébrés des effets biologiques identiques à ceux d'une hormone la calcitonine ce qui laisse suggérer une évolution commune de ces enzymes et de cette hormone : cette constatation est à la base du développement de nouveaux traitements de maladies du métabolisme du cartilage et de l'os et particulièrement de l'ostéoporose et la maladie de Paget.

Les enzymes métaboliques telles que des protéases ou des kinases sont des enzymes largement distribuées dans le règne animal. A titre d'exemples non exhaustifs, on peut citer comme références bibliographiques, les documents suivants pour les protéases: 'Methods in Enzymology XLII (1975)' et 'Journal of Medicinal Chemistry' vol. 43 n° 3 ('D. Leung, G. Abbenante et D.P. Fairlie') et le document suivant pour les kinases: 'Methods in Enzymology, Vol 80(1981)(Academic Press Inc.)'

Parmi les protéases capables de catalyser sélectivement l'hydrolyse des liaisons polypeptidiques, on peut citer les quatre principales classes : les aspartate protéases, les sérine protéases, les métallo-protéases et les cystéine protéases.

Les cystéine protéases constituent une famille de protéases qui possède un groupement thiol dans le site actif. De telles protéases existent chez les bactéries, les virus, les microorganismes eucaryotiques, les plantes et les animaux. De telles cystéines protéases sont très nombreuses et on cite ci-après, de façon non exhaustive, des exemples de cystéines protéases telles que des cystéines protéases de plantes comme la papaïne, la ficine, l'aleuraine, l'orizaine, et l'actinidine ; des cystéines protéases de mammifères comme par exemple les cathepsines B, H, J, L, N, S, T, C, V, W, K ou O, 02, l'enzyme de conversion de l'interleukine (ICE), les calpaïnes I et II, l'hydrolase bléomycine ; des cystéines protéases virales comme par exemple picornian 2A et 3C, les endopeptidases aphthvirus, cardiovirus, comovirus, potyvirus I et II, adénovirus, togavirus ou encore les cystéines protéases de la polio ou des rhinovirus. Les cystéines protéases sont connues également pour être essentielles pour la vie de certains parasites.

Parmi les cystéines protéases, on peut citer notamment les cathepsines et notamment les cathepsines K, B, L et S et la papaine.

On peut citer les documents suivants : WO 98/48799, WO 96/40737 et également 'Rawlings et al, Biochem. J. 290 :205-218 1993

De telles enzymes kinases ou protéases sont impliquées dans des processus de catabolisation et de communication inter et intracellulaire : elles jouent un rôle important dans un grand nombre de maladies de domaines différents tels que notamment le domaine cardiovasculaire, l'oncologie, le système nerveux central, l'inflammation, les désordres osseux, les affections du périodonte et également les maladies infectieuses parasitaires, fongiques ou virales. C'est pourquoi ces protéines sont des cibles de grand intérêt pour la recherche pharmaceutique.

Des inhibiteurs de telles protéases peuvent donc être utiles dans des domaines thérapeutiques nombreux et divers.

Les produits de la présente invention peuvent ainsi notamment être utiles dans la prévention ou le traitement de maladies dans lesquelles de telles enzymes métaboliques sont impliquées comme certaines maladies cardiovasculaires, maladies du système nerveux central, maladies inflammatoires, maladies de l'os telles que par exemple l'ostéoporose, l'ostéoarthrite ou encore les gingivites, les maladies infectieuses nécessitant notamment pour leur thérapie des anti-infectieux ou encore certains cancers.

De tels produits de la présente invention peuvent ainsi notamment être utilisés pour le traitement de maladies dans lesquelles de telles protéases sont impliquées, et notamment les maladies associées à une perte importante d'os ou de cartilage telles que définies ci-après.

Le tissu osseux est le siège d'un remodelage permanent continu qui permet d'assurer l'homéostasie phosphocalcique et de maintenir les qualités mécaniques des os.

Quelle que soit la nature des l'os (long, court, plat, ...)le remodelage se déroule selon une séquence de 4 phases successives :
1. Activation : comprend la différenciation des pré ostéoclastes par des hormones ou des signaux physiques
2. Résorption osseuse : assurée par les ostéoclastes matures, dédutée par une adhésion de ces cellules à la matrice osseuse, provoquant l'organisation de la membrane apicale en sealing zone délimitant un micro environnement (lacune de Howship) où sont libérés des protons produits par la cellule, responsable de la déminéralisation de la partie minérale des os, ainsi que des enzymes protéolytiques libérées au travers de la membrane, responsable de la dégradation des protéines de la matrice.
3. Inversion : caractérisée par le détachement des ostéoclastes, remplacés par les ostéoblastes.
4. Reconstruction : néoformation de la matrice (ostéoïde) et minéralisation, assurées par les ostéoblastes.

La durée d'un cycle de remodelage chez l'homme adulte normal est d'environ 3 mois.

Plusieurs enzymes protéolytiques interviennent dans la dégradation des protéines des la matrice. C'est le cas notamment pour les cathepsines K, B, L, et S ainsi que pour certaines metalloprotéases.

Les cathepsines sont définies comme des protéines lysosomales dont la majorité sont des enzymes « papaïne-like ». Plusieurs séquences du génome humain, encodant pour des cathepsines à cystéine, ont été identifiées à ce jour (ex. : B,H,L,S, C, K, O, F, V, X, W). Toutes ces enzymes contiennent une paire cystéine-histidine-asparagine dans leur site catalytique, formant une paire thiolate-imidazolium nécessaire à l'activité enzymatique. Elles sont capables d'hydrolyser divers substrats comme les peptides, les amides, les esters, les thiols esters et les thiono esters.

La famille des cathepsines à cystéine comprend divers enzymes protéolytiques impliquées dans plusieurs processus physiologiques, et peuvent ainsi jouer des rôles importants dans diverses pathologies.

La cathepsine K (préalablement dénommée O ou 02) a été identifiée à partir d'une banque de d'ADNc de lapin, puis localisée au niveau des ovaires et des ostéoclastes humains (dans les cellules matures adhérentes(Tezuka K, Tezuka Y, Maejima S: J.Biol.Chem. (1994) 269: 1106-1109)). Elle est exprimée majoritairement dans ces derniers (Brömme D, Okamoto K, Wang B, Biroc S : J.Biol.Chem. (1996) 271 : 2126-2132) et est ainsi considérée comme la protéase clé de ce processus, par rapport à sa localisation (Yamaza T, Goto T, Kamiya T, Kobayashi Y, Sakai H, Tanaka T : Bone (1998) 23 : 499-509) et parce que c'est une des quelques protéases ayant pour substrat naturel le collagène natif, constituant 90% des protéines de la matrice osseuse (Garnero P, Borel 0, Byrjalsen I, Ferreras M, Drake FH, McQueney MS, Foged NT, Delmas P, Délaisse JM : J.Biol.Chem (1998) 273 : 32347-32352) ; les 10% restant étant constitués de nombreuses protéines non collagéniques parfois substrats de la cathepsine K (ostéopontine, thrombospondine, fibronectine et vitronectine), parfois régulatrices de l'activité protéolytique (protéoglycanes).

Des souris transgéniques, KO pour le gène encodant la cathepsine K, ont un phénotype ostéopétrotique, incluant un accroissement de la masse osseuse et des os épais de mauvaise qualité. Dans ce modèle, la résorption osseuse est quasi absente. Seule subsiste la déminéralisation. Ce phénotype se compare à celui de patients atteints de Pycnodysostose (maladie de Toulouse-Lautrec), une maladie génétique aboutissant à la production de cathepsine K inactive responsable de l'hypoplasie faciale et de l'arrêt prématuré de la croissance des os longs avec ostéoscléroses sévères (Saftig P, Hunziker E, Wehmeyer O, Jones S, Boyde A, Rommerskirch W, Moritz JD, Schu P, Von Figura K : Proc.Natl.Acad.Sci. USA (1998) 95 : 13453-13458). L'utilisation d'oligonucléotides antisens de la cathepsine K (S-ODN) a permis d'inhiber la résorption osseuse médiée par les ostéoclastes dans un test de pit formation « in vitro » (Inuit FW, J.Biol. Chem (1997) 272 :8109-8112).

L'implication de la cathepsine K dans le processus de résorption osseuse fait de cette enzyme une cible privilégiée pour le traitement de pathologies, telle l'ostéoporose, dues à un déséquilibre du turn-over osseux en faveur de la résorption, cible pour de nouvelles molécules inhibitrices de l'activité enzymatique.

La présente invention concerne donc de nouveaux dérivés acyl hydrazino du thiophène inhibiteurs de cystéine protéases plus particulièrement de la famille des cathepsines B, K et notamment de la famille des cathepsine K.

La présente invention a ainsi pour objet les produits de formule (I): 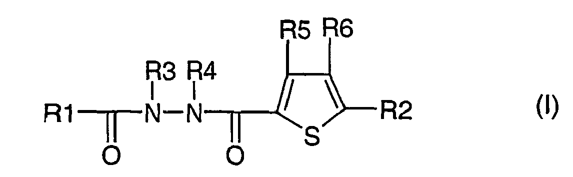 dans laquelle:
R1 représente un radical alkyle ou alcoxy linéaires ou ramifiés renfermant au plus 8 atomes de carbone substitués soit par un radical monocyclique ou constitué de cycles condensés, carbocyclique ou hétérocyclique saturé ou insaturé renfermant au plus 14 chaînons et contenant un ou plusieurs hétéroatomes identiques ou différents, O, N, NH ou S, un tel radical monocyclique ou constitué de cycles condensés étant lui-même éventuellement substitué comme indiqué ci-après, soit par un radical amino lui-même éventuellement substitué par un radical -C(O)-O- R7 ou -C(O)-R7,
R2 représente :
a) un radical alkyle linéaire ou ramifié renfermant au plus 8 atomes de carbone éventuellement substitué par un ou plusieurs radicaux choisis parmi les radicaux -NH-C(O)-O-R7, les radicaux -NH-C(O)-alk-NH-C(O)-O-R7 et les radicaux monocycliques ou constitués de cycles condensés, carbocycliques ou hétérocycliques saturés ou insaturés renfermant au plus 14 chaînons et contenant un ou plusieurs hétéroatomes identiques ou différents, O, N, NH ou S, de tels radicaux monocycliques ou constitués de cycles condensés étant eux-mêmes éventuellement substitués come indiqué ci-après,
b) un radical phényle éventuellement substitué par un radical alcoxy linéaire ou ramifié renfermant au plus 6 atomes de carbone ou un radical arylalcoxy, ces radicaux alcoxy et arylalcoxy étant eux-mêmes éventuellement substitués come indiqué ci-après,
c) un radical naphtyle éventuellement substitué come indiqué ci-après,
d) un radical amino éventuellement substitué par un ou deux radicaux identiques ou différents choisis parmi les radicaux alkyles linéaires ou ramifiés renfermant au plus 6 atomes de carbone, les radicaux -C(O)-alk-NH-C(O)-O-R7 et les radicaux arylalkyle eux-mêmes éventuellement substitués par un radical alcoxyphénoxy dans lequel le radical alcoxy linéaire ou ramifié renferme au plus 6 atomes de carbone, ces radicaux alkyle, arylalkyle, alcoxy, et alcoxyphénoxy étant eux-mêmes éventuellement substitués come indiqué ci-après,
e) -(CH₃)C=N-O-R⁸,
R3 et R4, identiques ou différents, représentent un atome d'hydrogène, un radical alkyle, arylalkyle ou aryle, dans lesquels les radicaux alkyle sont linéaires ou ramifiés renfermant au plus 6 atomes de carbone et les radicaux aryle renferment au plus 10 atomes de carbone, ces radicaux alkyle, arylalkyle et aryle éventuellement substitués par un atome d'halogène, un radical hydroxyle ou un radical alkyle ou alcoxy linéaires ou ramifiés renfermant au plus 4 atomes de carbone,
R5 et R6, identiques ou différents, sont choisis parmi l'atome d'hydrogène, le radical hydroxyle et les radicaux alkyle et alcoxy linéaires ou ramifiés renfermant au plus 6 atomes de carbone, ces radicaux alkyle et alcoxy étant eux-mêmes éventuellement substitués comme indiqué ci-après
R7 représente un radical alkyle linéaire ou ramifié renfermant au plus 6 atomes de carbone éventuellement substitué par un radical aryle ou hétéroaryle (indazolyle), éventuellement substitués,
R8 représente un atome d'hydrogène ou un radical alkyle linéaire ou ramifié renfermant au plus 6 atomes de carbone éventuellement substitué par un radical aryle (phényle), étant entendu que tous les radicaux alkyle, alcoxy, monocyclique ou constitué de cycles condensés, aryle, hétéroaryle, arylalkyle, arylalcoxy et alcoxyphénoxy indiqués ci-dessus comme étant éventuellement subtitués, sont éventuellement substitués par un ou plusieurs radicaux choisis parmi les atomes d'halogène ; les radicaux hydroxyle; alkyle, alkényle, alkylthio ou alcoxy renfermant au plus 6 atomes de carbone; cycloalkyle renfermant au plus 6 chaînons; acyle renfermant au plus 7 atomes de carbone ; cyano ; nitro ; carboxy libre, salifié ou estérifié ; tétrazolyle; indazolyle ; -NH2, -NH(alk), -N(alk)(alk) ; -S02-NH-CO-NHR5 dans lequel R5 représente alk ou phényle; -C(O)-NH2 , -C(O)-NH(alk), -C(O)-N(alk)(alk), -NH-C(O)-(alk), -N(alk)-C(O)-(alk); et phényle lui même éventuellement substitué par un radical alkyle ou alcoxy renfermant au plus 4 atomes de carbone,
étant entendu que alk représente un radical alkyle linéaire ou ramifié renfermant au plus 4 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) et dans ce qui suit :
- le terme radical alkyle linéaire ou ramifié désigne les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés,
- le terme radical alcoxy linéaire ou ramifié désigne les radicaux méthoxy, éthoxy, propoxy, isopropoxy, butoxy linéaire, secondaire ou tertiaire, pentoxy ou hexoxy ainsi que leurs isomères de position linéaires ou ramifiés
- le terme atome d'halogène désigne les atomes de chlore, de brome, d'iode ou de fluor et de préférence l'atome de chlore, de brome ou de fluor,
- Le terme radical carbocyclique ou hétérocyclique monocyclique ou constitués de cycles condensés saturé ou insaturé renfermant au plus 14 chaînons contenant un ou plusieurs hétéroatomes identiques ou différents choisis parmi O, N, NH ou S, regroupe les définitions qui suivent:

- le terme radical carbocyclique saturé désigne notamment un radical cycloalkyle
- le terme radical cycloalkyle désigne les radicaux cyclopropyle, cyclobutyle, cyclopentyle et cyclohexyle et tout particulièrement les radicaux cyclopentyle et cyclohexyle,
- le terme radical hétérocyclique monocyclique désigne un radical saturé ou insaturé constitué notamment de 5 ou 6 chaînons tel que l'un ou plusieurs des chaînons représente un atome d'oxygène, de soufre ou d'azote : un tel radical hétérocyclique désigne ainsi un radical carbocyclique interrompu par un ou plusieurs hétéroatomes choisis parmi les atomes d'oxygène, d'azote ou de soufre étant entendu que les radicaux hétérocycliques peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents. On peut citer notamment le radical dioxolane, dioxane, dithiolane, thiooxolane, thiooxane, morpholinyle, pipérazinyle, pipérazinyle substitué par un radical alkyle, linéaire ou ramifié, renfermant au plus 4 atomes de carbone, pipéridyle, thiényle tel que 2-thiényle et 3-thiényle, furyle tel que 2- furyle, pyrimidinyle, pyridyle tel que 2-pyridyle, 3-pyridyle et 4-pyridyle pyrimidyle, pyrazolinyle, pyrrolyle, thiazolyle, isothiazolyle, diazolyle, thiadiazolyle, triazolyle, tétrazolyle libre ou salifié thiadiazolyle, thiatriazolyle, oxazolyle, oxadiazolyle, 3- ou 4-isoxazolyle. On peut citer tout particulièrement les radicaux morpholinyle, thiényle tel que 2-thiényle et 3-thiényle, furyle tel que 2-furyle , tétrahydrofuryle, thiényle, tétrahydrothienyle, pyrrolyle, pyrrolinyle, pyrazolinyle, isoxazolyle, pyridyle et pyrrolidinyle.
- le terme radical hétérocyclique constitué de cycles condensés désigne un radical saturé ou insaturé renfermant au plus 14 chaînons tel que l'un ou plusieurs des chaînons représente un atome d'oxygène, de soufre ou d'azote et notamment des groupes hétérocycliques condensés contenant au moins un hétéroatome choisi parmi le soufre, l'azote et l'oxygène, par exemple benzothiényle tel que 3-benzothiényle, benzothiazolyle, quinolyle, isoquinolyle, tetralone, benzofuryle, benzopyrrolyle, benzimidazolyle, benzoxazolyle, thionaphtyle, indolyle ou purinyle.
- le terme aryle désigne des radicaux monocycliques ou constitués de cycles condensés carbocycliques non saturés tels que définis ci-dessus et désigne ainsi notamment les radicaux phényle et naphtyle et notamment le radical phényle.
- le terme hétéroaryle désigne des radicaux monocycliques ou constitués de cycles condensés hétérocycliques non saturés tels que définis ci-dessus et désigne ainsi notamment les radicaux indazolyle, thiényle, benzo-thiényle, furyle, pyrannyle, isobenzofurannyle, pyrrolyle, imidazolyle, pyrazolyle, pyridyle, pyrazinyle, pyrimidinyle, pyridazinyle, indolyle et ses isomères de position de l'atome d'azote, indazolyle, quinolyle et ses isomères de position de l'atome d'azote, quinazolinyle et ses isomères de position des deux atomes d'azote, isothiazolyle, isoxazolyle, furazannyle.
- le terme arylalcoxy désigne des radicaux dans lesquels aryle et alcoxy ont les définitions indiquées ci-dessus et représente notamment les radicaux phénylalcoxy dans lesquels alcoxy renferme au plus 4 atomes de carbone,
- le terme alcoxyphénoxy désigne des radicaux dans lesquels alcoxy a la définition indiquée ci-dessus et représente notamment alcoxy renferme au plus 4 atomes de carbone,
- le terme arylalkyle désigne des radicaux dans lesquels aryle et alkyle ont les définitions indiquées ci-dessus et représente notamment les radicaux phénylalkyle dans lesquels alkyle renferme au plus 4 atomes de carbone
- le terme alkylphényle désigne un radical phényle substitué par un ou plusieurs radicaux alkyle tels que définis ci-dessus linéaires ou ramifiés de préférence renfermant au plus 4 atomes de carbone
- les termes NH(alk) et N(alk)(alk) désigne un radical amino substitués respectivement par un ou deux radicaux alkyle, de tels radicaux alkyle ou alk étant linéaires ou ramifiés et renfermant de préférence au plus 4 atomes de carbone
- le terme acylamino désigne les radicaux -C(O)-NH2,
- C(O)-NH(alk) et -C(O)-N(alk)(alk) : dans ces radicaux ainsi que dans les radicaux NH-C(O)-(alk), -N(alk)-C(O)-(alk) -et - C(O)-alk-NH-C(O)-O-R7, les termes alk, NH(alk), N(alk)(alk), ont les significations indiquées ci-dessus
- le terme acyle désigne un radical R-C(O)- dans lequel R représente un radical choisi parmi l'atome d'hydrogène, les radicaux alkyle linéaire ou ramifié renfermant au plus 6 atomes de carbone, un radical phényle ou un radical pyrrolidinyle : le terme acyle désigne ainsi notamment les radicaux formyle, les radicaux acétyle, propionyle, butanoyle, pentanoyl, hexanoyl, benzoyle et pyrrolidinylcarbonyle
- le terme alkényle désigne des radicaux linéaire ou ramifié renfermant au plus 6 atomes de carbone : on peut citer notamment les radicaux vinyle, 1-propényle, allyle, butényle, 3-méthyle-2-butényle
- le terme alkylthio désigne des radicaux linéaire ou ramifié renfermant au plus 6 atomes de carbone tels que notamment les radicaux méthylthio, éthylthio, propylthio, isopropylthio, butylthio, isobutylthio, sec-butylthio, tert-butylthio, pentylthio, isopentylthio, hexylthio ou encore isohexylthio ainsi que leurs isomères de position linéaires ou ramifiés : parmi ces radicaux alkylthio, on choisit de préférence parmi ceux cités ci-dessus, ceux qui renferment au plus 4 atomes de carbone
- le terme acide aminé désigne les acides aminés dits naturels ou dits non naturels connus de l'homme du métier qui peuvent être trouvés dans des documents usuels tels que par exemple le document 'Amino Acids (1999), 16(3-4), 345-379.

Le ou les radicaux carboxy des produits de formule (I) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple, dans les groupements chlorométhyle, hydroxypropyle, méthoxyméthyle, propionyloxyméthyle, méthylthiométhyle, diméthylaminoéthyle, benzyle ou phénéthyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

Oh peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.

On peut noter que lorsque R1 représente alkyle substitué par amino lui-même éventuellement substitué, R1 représente notamment un reste d'acide aminé décarboxylé éventuellement substitué et plus précisément un reste de Leu décarboxylée éventuellement substituée.

La présente invention a ainsi pour objet les produits de formule (I) telle que définie ci-dessus répondant à la formule (Ia) : 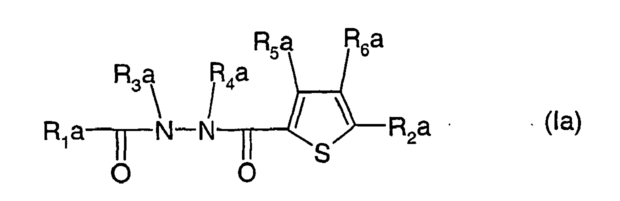 dans laquelle:
R1a représente un radical alkyle ou alcoxy linéaires ou ramifiés renfermant au plus 8 atomes de carbone substitués par un radical phényle, un radical constitué de cycles condensés carbocyclique saturé ou insaturé renfermant au plus 14 chaînons et un radical amino lui-même éventuellement substitué par un radical -C(O)-O- R7a ou -C(O)-R7a, ce radical phényle et ces radicaux constitués de cycles condensés carbocyclique saturé ou insaturé étant éventuellement substitués comme indiqué ci-après,
R2a représente :
a) un radical alkyle linéaire ou ramifié renfermant au plus 6 atomes de carbone éventuellement substitué par un ou plusieurs radicaux choisis parmi les radicaux NH-C(O)-O-R7a et -NH-C(O)-alk-NH-C(O)-O-R7, le radical phényle et les radicaux hétérocycliques saturés ou insaturés renfermant au plus 10 chaînons,
b) un radical phényle éventuellement substitué par un radical alcoxy linéaire ou ramifié renfermant au plus 6 atomes de carbone ou un radical benzyloxy,
c) un radical naphtyle,
d) un radical amino éventuellement substitué par un ou deux radicaux identiques ou différents choisis parmi les radicaux alkyles linéaires ou ramifiés renfermant au plus 4 atomes de carbone, les radicaux -C(O)-alk-NH-C(O)-O-R7a et le radical benzyle lui-même éventuellement substitué par un radical alcoxyphénoxy dans lequel le radical alcoxy renferme au plus 4 atomes de carbone,
e) -(CH₃)-C=N-O-R⁸,
R3a et R4a, identiques ou différents, représentent un atome d'hydrogène, un radical alkyle linéaire ou ramifié renfermant au plus 6 atomes de carbone ou un radical phényle éventuellement substitué par un radical hydroxyle ou alcoxy renfermant au plus 4 atomes de carbone
R5a et R6a, identiques ou différents, sont choisis parmi l'atome d'hydrogène, le radical hydroxyle et les radicaux alkyle ou alcoxy linéaires ou ramifiés renfermant au plus 6 atomes de carbone éventuellement substitués par un radical phényle ou indazolyle,
R7a représente un radical alkyle linéaire ou ramifié renfermant au plus 4 atomes de carbone éventuellement substitué par un radical phényle ou un radical indazolyle, R8a représente un atome d'hydrogène ou un radical alkyle linéaire ou ramifié renfermant au plus 4 atomes de carbone éventuellement substitué par un radical phényle,
étant entendu que alk représente un radical alkyle linéaire ou ramifié renfermant au plus 4 atomes de carbone,
lesdits produits de formule (Ia) étant sous toutes les formes isomères possibles racémiques, énantiomères et diastéréoisomères, ainsi que les sels d'addition avec les acides minéraux et organiques ou avec les bases minérales et organiques desdits produits de formule (Ia).

La présente invention a plus particulièrement pour objet les produits de formule (I) telle que définie ci-dessus répondant à la formule (Ib) : 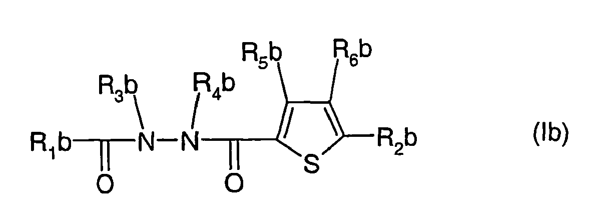 dans laquelle:
R1b représente un radical alkyle ou alcoxy linéaires ou ramifiés renfermant au plus 6 atomes de carbone substitué par un radical choisi parmi les radicaux phényle, fluoren (ex 1 et 20) et amino lui-même éventuellement substitué par un radical -C(O)-O-R7b ou -C(O)-R7b,
R2b représente :
a) un radical alkyle linéaire ou ramifié renfermant au plus 6 atomes de carbone éventuellement substitué par un ou plusieurs radicaux choisis parmi les radicaux -NH-C(O)-O-R7b et -NH-C(O)-alk-NH-C(O)-O-R7b et les radicaux phényle, pyridyle, pyridinyle, pipéridinyle, benzofurannyle, morpholinyle, quinolinyle, indolyle, benzimidazolyle et indazolyle,
b) un radical phényle éventuellement substitué par un radical alcoxy linéaire ou ramifié renfermant au plus 4 atomes de carbone ou un radical benzyloxy,
c) un radical naphtyle,
d) un radical amino éventuellement substitué par un ou deux radicaux identiques ou différents choisis parmi les radicaux alkyles linéaires ou ramifiés renfermant au plus 4 atomes de carbone, les radicaux -C(O)-alk-NH-C(O)-O-benzyle et le radical benzyle lui-même éventuellement substitué par un radical alcoxyphénoxy dans lequel le radical alcoxy renferme au plus 4 atomes de carbone,
e) -(CH₃)C=N-O-R⁸,
R3b et R4b représentent un atome d'hydrogène,
R5b et R6b, identiques ou différents, représentent un atome d'hydrogène, un radical hydroxyle, un radical alkyle ou alcoxy linéaires ou ramifiés renfermant au plus 4 atomes de carbone éventuellement substitué par un radical phényle,
R7b représente un radical alkyle linéaire ou ramifié renfermant au plus 4 atomes de carbone éventuellement substitué par un radical phényle ou un radical indazolyle, R8b représente un atome d'hydrogène ou le radical benzyle, étant entendu que alk représente un radical alkyle linéaire ou ramifié renfermant au plus 4 atomes de carbone,
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

La présente invention a tout particulièrement pour objet les produits de formules (I), (Ia) et (Ib) telles que définies ci-dessus,dans lesquelles R3 ,R3a, R4, R4a, R5, R5a, R5b, R6, R6a et R6b représentent un atome d'hydrogène,
lesdits produits de formules (I), (Ia) et (Ib) étant sous toutes les formes isomères possibles racémiques, énantiomères et diastéréoisomères, ainsi que les sels d'addition avec les acides minéraux et organiques ou avec les bases minérales et organiques desdits produits de formules (I), (Ia) et (Ib).

La présente invention a encore plus particulièrement pour objet les produits de formule (I) telle que définie ci-dessus, répondant aux formules suivantes:
- le 2-[(2S)-4-méthyl-1-oxo-2-[[(phénylméthoxy)carbonyl] amino]pentyl]-hydrazide de l'acide 5-[(2S)-1-[[4-méthyl-1-oxo
- 2-[[(phénylméthoxy)carbonyl]amino]pentyl]amino]éthyl]-2-thiophènecarboxylique
- le 2-[[(9H-fluoren-9-yl)méthoxy]carbonyl]-hydrazide de l'acide 5-[1-(hydroxyimino)éthyl]-2-thiophènecarboxylique
- le 2-[(2S)-4-méthyl-1-oxo-2-[[(phénylméthoxy)carbonyl] amino]pentyl]-hydrazide de l'acide 5-[2-(phénylméthoxy) phényl]-2-thiophènecarboxylique
- le 2-[(2S)-4-méthyl-1-oxo-2-[[(phénylméthoxy)carbonyl] amino]pentyl]-hydrazide de l'acide 5-[[[3-(4-méthoxyphènoxy) phényl]méthyl](phénylméthyl)amino]-2-thiophènecarboxylique
- le 2-[(2S)-4-méthyl-1-oxo-2-[[(phénylméthoxy)carbonyl]-amino] pentyl]-hydrazide de l'acide 5-[1-[(phénylméthoxy) imino]éthyl]-2-thiophènecarboxylique
- le 2-[[(9H-fluoren-9-yl)méthoxy]carbonyl]- hydrazide de l'acide 5-[1-[[(phénylméthoxy)carbonyl]amino]éthyl]-2-thiophènecarboxylique
- le 2-[(2S)-4-méthyl-1-oxo-2-[[(phénylméthoxy)carbonyl]-amino] pentyl]-hydrazide de l'acide 5-[1-[[(phénylméthoxy)carbonyl] amino]éthyl]-2-thiophènecarboxylique
- le 2-[(2S)-4-méthyl-1-oxo-2-[[(phénylméthoxy)carbonyl]-amino] pentyl]- hydrazide de l'acide 5-[3-méthyl-1-[(phénylméthoxy) carbonyl]amino]butyl]-2-thiophènecarboxylique
- le 2-[(2S)-4-méthyl-1-oxo-2-[[(phénylméthoxy)carbonyl]-amino] pentyl]-hydrazide de l'acide 5-[3-méthyl-1-[[(phénylméthoxy) carbonyl]amino]butyl]-2-thiophènecarboxylique isomère A
- le [(2S)-4-méthyl-1-oxo-2-[[(phénylméthoxy)carbonyl]amino] pentyl]-hydrazide de l'acide 5-[3-méthyl-1-[[(phénylméthoxy) carbonyl]amino]butyl]-2-thiophènecarboxylique Isomere B

La présente invention a encore pour objet un procédé de préparation des produits de formule (I), telle que définie ci-dessus, caractérisé en ce que l'on part d'un composé de formule (II): 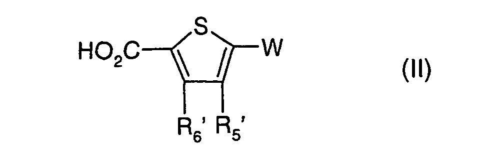 dans lequel W représente un atome d'hydrogène, un atome d'halogène ou un radical nitro, R5' et R6' ont les significations indiquées ci-dessus respectivement pour R5 et R6, dans lesquelles les éventuelles fonctions réactives sont éventuellement protégées par des groupements protecteurs, produit de formule (II) que selon la valeur de W l'on peut soumettre aux réactions suivantes :
a) lorsque W représente un atome d'hydrogène, l'on peut soumettre le produit de formule (II) aux réactions des voies i) ou ii) :
   voie i) on peut soumettre le composé de formule (II) dans lequel W représente un atome d'hydrogène à l'action d'un dérivé bromé de formule (III) :

   R2'-Br (III)

   dans laquelle R2' a la signification indiquée ci-dessus pour R2 dans laquelle les éventuelles fonctions réactives sont éventuellement protégées par des groupements protecteurs, pour obtenir un produit de formule (IV) : 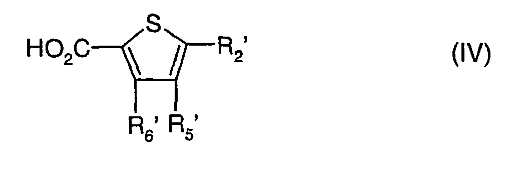 dans laquelle R2', R5' et R6' ont les significations indiquées ci-dessus,
   que lorsque R2' représente un radical -CH2- phényle, pour donner le produit de formule (IV1) : 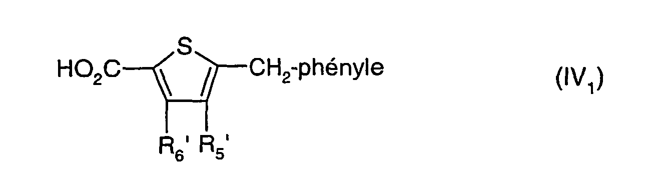 dans laquelle R5' et R6' ont les significations indiquées ci-dessus, l'on peut soumettre à une seconde réaction avec le composé de formule (III) pour obtenir le composé de formule (IV2) : 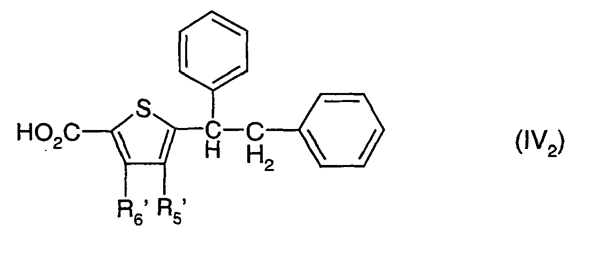 dans laquelle R5' et R6' ont les significations indiquées ci-dessus,
   produits de formules (IV), (IV1) et (IV2) que l'on peut soumettre à une réaction d'estérification pour obtenir les produits correspondants de formules (V), (V1) et (V2) : 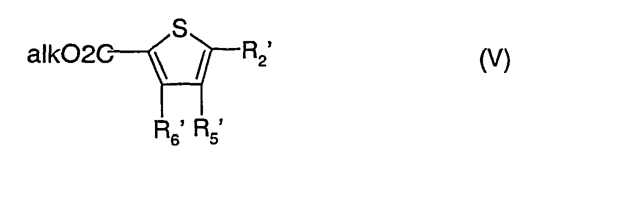 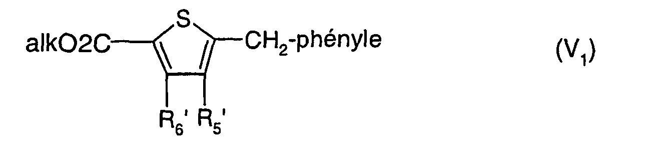 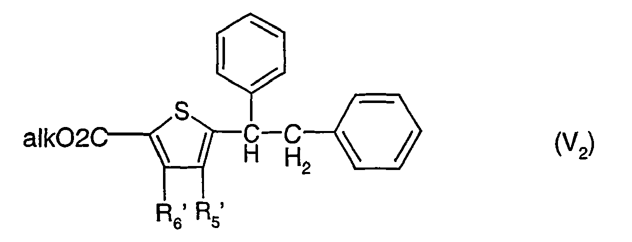 dans laquelle R2', R5' et R6' ont les significations indiquées ci-dessus et alk représente un radical alkyle linéaire ou ramifié renfermant au plus 4 atomes de carbone, voie ii) on peut soumettre le composé de formule (II) dans lequel W représente un atome d'hydrogène à une réaction d'estérification avec un alcool de formule (VI) :

   Rc-OH (VI)

   dans laquelle Rc représente un radical alkyle linéaire ou ramifié renfermant au plus 6 atomes de carbone, pour obtenir des produits de formule (VII): 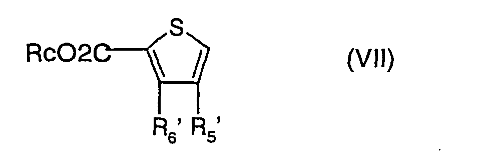 dans laquelle Rc, R5' et R6' ont les significations indiquées ci-dessus,
   que l'on soumet à une aldéhyde de formule (VIII) :

   Rd-CHO (VIII)

   dans laquelle Rd représente un atome d'hydrogène, un radical alkyle tel que défini ci-dessus ou un radical monocyclique ou constitué de cycles condensés, carbocyclique ou hétérocyclique saturé ou insaturé renfermant au plus 14 chaînons et contenant un ou plusieurs hétéroatomes identiques
   ou différents, O, N, NH ou S, eux-mêmes éventuellement substitués,
   pour obtenir des produits de formule (IX) : 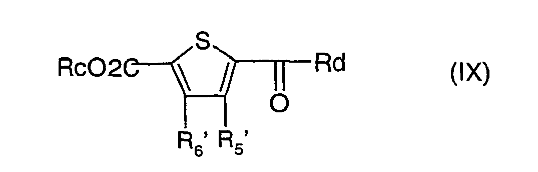 dans laquelle Rc, Rd, R5' et R6' ont les significations indiquées ci-dessus,
   produits de formule (IX) que l'on fait réagir avec une amine de formule (X):

   H2N-CH2-ORe (X)

   dans laquelle Re représente un radical arylalkyle éventuellement substitué,
   pour obtenir des produits de formule (XI) : 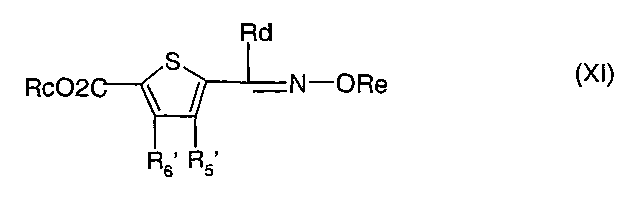 dans laquelle Rc, Rd, Re, R5' et R6' ont les significations indiquées ci-dessus,
   produit de formule (XI) que l'on peut soumettre à une réaction de réduction sélective,
   pour obtenir des produits de formule (XII) : 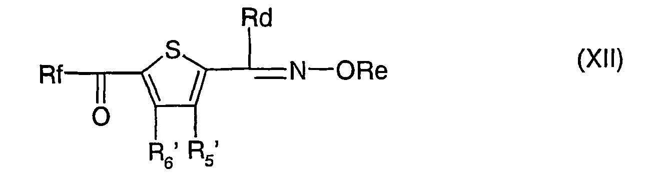 dans laquelle Rd, Re, R5' et R6' ont les significations indiquées ci-dessus, et Rf représente un groupement de réduction sélective de ORc produits de formules (XI) et (XII) que l'on peut soumettre à une réaction de réduction pour obtenir les produits correspondants de formule (XIII): 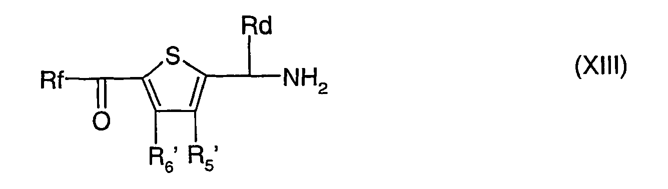 dans laquelle Rd, Rf, R5' et R6' ont les significations indiquées ci-dessus,
   produits de formule (XIII) que l'on peut soumettre à une réaction de saponification pour obtenir les produits correspondants de formule (XIV): 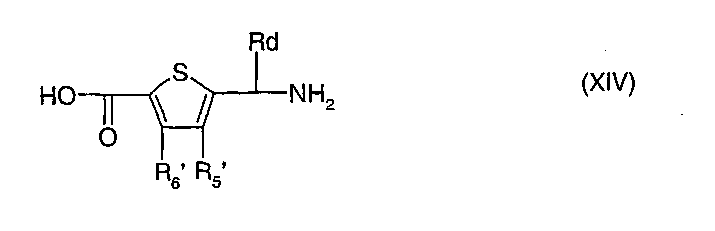 dans laquelle Rd, R5' et R6' ont les significations indiquées ci-dessus,
   produit de formule (XIV) que l'on peut faire réagir avec un produit de formule (XV): 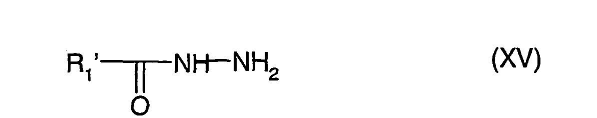 dans laquelle R1' a la signification indiquée ci-dessus pour R1 dans laquelle les éventuelles fonctions réactives sont éventuellement protégées par des groupements protecteurs, pour obtenir des produits de formule (I'x): 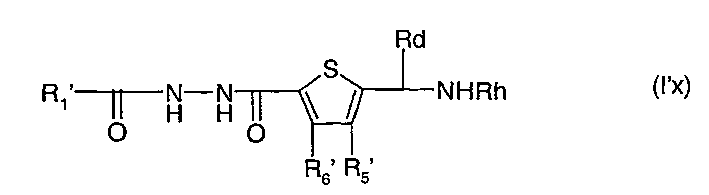 dans laquelle R1', Rd, R5' et R6' ont les significations indiquées ci-dessus et Rh représente -C(O)-O-R7 ou -C(O)-alk-NH-C(O)-O-R7 avec R7 tel que défini ci-dessus,
b) lorsque W représente un atome d'halogène, l'on peut soumettre le produit de formule (II) à une réaction d'estérification avec un alcool de formule (VI) telle que définie ci-dessus pour obtenir des produits de formule (XVI): 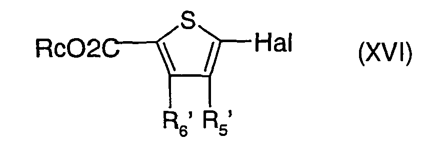 dans laquelle Rc, R5' et R6' ont les significations indiquées ci-dessus et Hal représente un atome d'halogène,
   produit de formule (XVI) que l'on peut soumettre :
   soit à l'action d'un dérivé boré de formule (XVII) :

   R2'-B(OH)2 (XVII)

   dans laquelle R2' a la signification indiquée ci-dessus, pour obtenir un produit de formule (XVIII) : 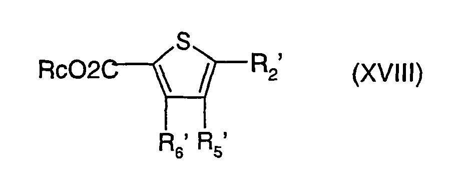 dans laquelle Rc, R2', R5' et R6' ont les significations indiquées ci-dessus,
   soit à l'action d'un produit de formule (XIX):

   NH-RaRb (XIX)

   dans laquelle Ra et Rb identiques ou différents représentent un radical alkyle ou aryle tels que définis ci-dessus éventuellement substitués,
   pour obtenir un produit de formule (XX) : 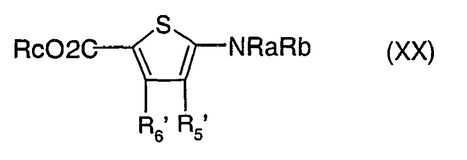 dans laquelle Rc, Ra, Rb, R5' et R6' ont les significations indiquées ci-dessus,
c) lorsque W représente un radical nitro, l'on peut soumettre le produit de formule (II) à une réaction d'estérification avec un alcool de formule (VI) tel que défini ci-dessus pour obtenir des produits de formule (XXI) : 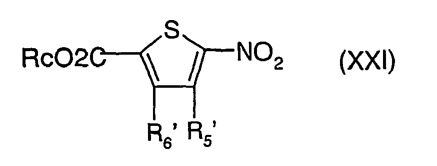 dans laquelle Rc, R5' et R6' ont les significations indiquées ci-dessus,
   que l'on peut soumettre à une réaction avec un dérivé halogéné de formule (XXII):

   RaRbHal (XXII)

   dans laquelle Ra et Rb ont les significations indiquées ci-dessus et Hal représente un atome d'halogène,
   pour obtenir un produit de formule (XX) telle que définie ci-dessus,
   produits de formules (IV), (IV1), (IV2), (V), (V1), (V2), (schéma I) (XVIII), (XX) telles que définies ci-dessus que l'on peut faire réagir avec la diamine NH2-NH2 pour obtenir un produit de formule (XXIII) : 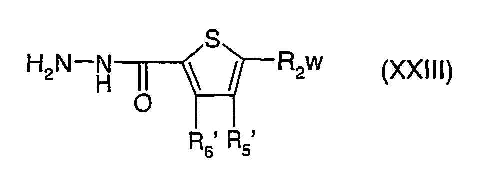 dans laquelle R5' et R6' ont les significations indiquées ci-dessus et R2w représente -CH2-phényle,
   -(CH)phényle-CH2(phényle), R2' ou NRaRb telles que définies ci-dessus,
   que l'on peut soumettre à une réaction avec un produit de formule (XXIV) :

   R1'-COOH (XXIV)

   dans laquelle R1' a la signification indiquée ci-dessus, pour obtenir un produit de formule (Iy) : 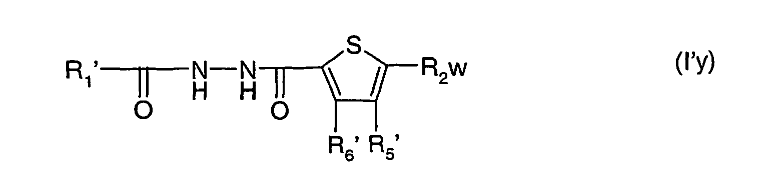
dans laquelle R1', R2w, R5' et R6' ont les significations indiquées ci-dessus,
produits de formule (Ix') et (Iy')qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

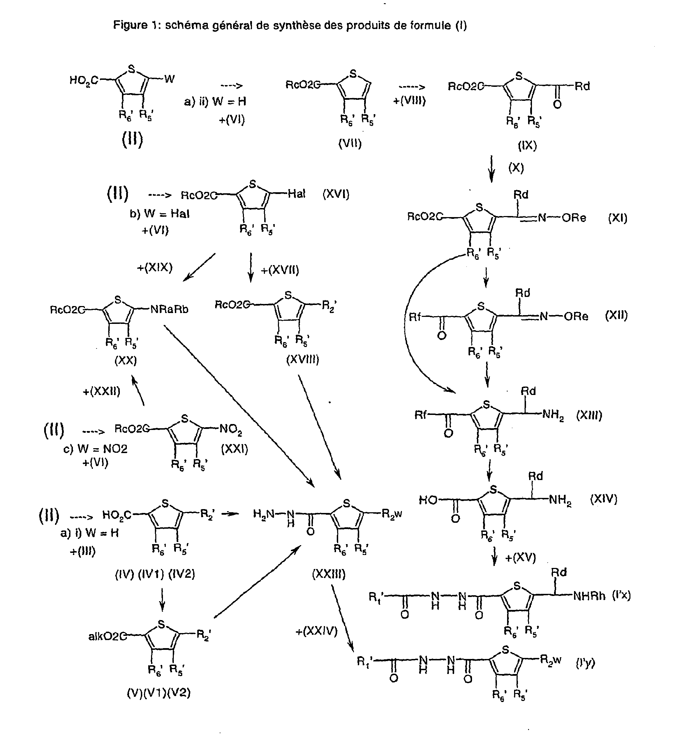

La figure 1 ci-après représente le schéma de synthèse des produits de formule (I) selon le procédé défini ci-dessus. Dans des conditions préférentielles de mise en oeuvre de l'invention, le procédé décrit ci-dessus peut-être réalisé de la façon suivante :

Le produit de formule (II) dans lequel W représente un atome d'hydrogène peut être soumis à l'action du dérivé halogéné de formule (III) pour donner un produit de formule (IV) dans un solvant tel que le THF en présence de LDA.

Le produit de formule (III) représente un dérivé halogéné tel que par exemple alkylhal ou arylalkylHal dans lequel Hal représente un atome d'halogène et aryl et alkyl représentent respectivement un radical aryle et alkyle tels que définis ci-dessus : (III) représente ainsi par exemple phényl-CH2-Br ou un bromure d'alkyle tel que le bromure d'isopropyle. On peut soumettre de nouveau le produit de formule (IV) obtenu à l'action du même composé de formule (III) dans les mêmes conditions.

Ainsi lorsque (III) représente phényl-CH2-Br, on peut soumettre de nouveau le produit de formule (IV1) obtenu à l'action du même composé de formule (III) dans les mêmes conditions et on obtient ainsi le produit de formule (IV2) telle que définie ci-dessus.

Les produits de formules (IV1) et (IV2) sont soumis à une réaction d'estérification dans les conditions usuelles telles que par exemple dans un alcool tel que l'éthanol ou le méthanol catalysé par H2SO4 ou par couplage avec le dicyclohexylcarbodiimide (DCC) ou ses dérivés ou le diazométhane dans le chlorure de méthylène, pour obtenir les produits correspondants de formules (V), (V1) et (V2) telles que définies ci-dessus.

Le produit de formule (II) dans lequel W représente un atome d'hydrogène peut être soumis à l'action d'un alcool de formule (VI) tel que par exemple le méthanol ou l'éthanol dans un solvant tel que SOC12 ou dans les conditions indiquées ci-dessus pour donner un produit de formule (VII) tel que défini ci-dessus. On fait réagir le produit de formule (VII) ainsi obtenu avec un aldéhyde de formule (VIII) par exemple dans le THF pour obtenir un produit de formule (IX) tel que défini ci-dessus.

Dans le composé de formule (VIII) tel que défini ci-dessus, Rd représente notamment un atome d'hydrogène, un radical alkyle ou aryle éventuellement substitués notamment par un radical hydroxyle, phényle ou hydroxyphényle.

La réaction du produit de formule (IX) avec l'amine de formule (X) est réalisée dans la pyridine ou encore dans le dichlorométhane en présence de triéthylamine pour donner un produit de formule (XI) tel que défini ci-dessus.

Le produit de formule (XI) ainsi obtenu est soumis à une réaction de couplage en présence de DCC ou d'un de ces dérivés dans CH2Cl2 ou DMF pour donner un produit de formule (XII) tel que défini ci-dessus.

Les produits de formules (XI) et (XII) peuvent être soumis à une réaction de réduction dans l'acide trifluoroacétique en présence de Zn pour donner un produit de formule (XIII) tel que défini ci-dessus.

Le produit de formule (XIII) est soumis à une réaction de saponification dans les conditions usuelles par exemple dans un mélange THF/eau/base, la base pouvant être de la lithine (LiOH) ou de la soude et on obtient ainsi le produit de formule (XIV).

On fait réagir le produit de formule (XIV) avec un produit de formule (V) tel que défini ci-dessus dans CH2Cl2 en présence de DMF avec un agent de couplage tel que le DCC ou un de ses dérivés pour obtenir un produit de formule (I'x) telle que définie ci-dessus.

Le produit de formule (II) dans lequel W représente un atome d'halogène peut être soumis à une réaction d'estérification avec un alcool de formule (VI) telle que définie ci-dessus pour obtenir un produit de formule (XVI) en opérant par exemple dans les mêmes conditions que pour obtenir (VII) à partir de (II) et (VI). On peut noter que l'atome d'halogène est de préférence l'atome de brome.

On peut faire réagir le produit de formule (XVI) ainsi obtenu avec un dérivé boré de formule (XVII) par exemple dans le toluène en présence de Pd(P(phényl)3)4 pour obtenir un produit de formule (XVIII) tel que défini ci-dessus.

On peut également faire réagir le produit de formule (XVI) ainsi obtenu avec une amine de formule (XIX) telle que définie ci-dessus dans la DMF de préférence sous pression à 165°C pour obtenir un produit de formule (XX) tel que défini ci-dessus.

Le produit de formule (II) dans lequel W représente un radical nitro peut être soumis à une réaction d'estérification avec un alcool de formule (VI) telle que définie ci-dessus pour obtenir un produit de formule (XXI) en opérant par exemple dans les mêmes conditions que pour obtenir (VII) à partir de (II) et (VI).

On peut faire réagir le produit de formule (XXI) ainsi obtenu avec un produit de formule (XXII) telle que définie ci-dessus dans le DMF pour obtenir le produit de formule (XX) tel que défini ci-dessus.

On peut alors faire réagir les produits de formules (IV), (IV1), (IV2), (V), (V1), (V2), (XVIII) ou (XX) avec l'hydrazine sous forme d'hydrate dans un solvant tel que par exemple la DMF dans un alcool tel que par exemple l'éthanol pour obtenir les produits correspondants de formule (XXIII) telle que définie ci-dessus.

On peut alors faire réagir les produits de formule (XXIII) ainsi obtenus avec un acide de formule (XXIV) par exemple dans le dichlorométhane et la DMF pour obtenir un produit de formule (Iy') telle que définie ci-dessus.

Le produit de formule (XXIV) R1'-COOH peut notamment représenter un reste d'acide aminé tel que par exemple Phe ou Leu éventuellement substitués.

Selon les valeurs de R1', R5', R6', Rd, Rh et R2W , les produits de formules (Ix') et (Iy') ainsi obtenus constituent ou non des produits de formule (I).

Les produits de formule (Ix') et (Iy') peuvent constituer des produits de formule (I) dans laquelle les fonctions éventuellement réactives sont protégées et qui donnent donc après réaction de déprotection de ces fonctions des produits de formule (I) telle que définie ci-dessus.

Les produits de formule (Ix') et (Iy') peuvent également donner des produits de formule (I), ou être transformés en d'autres produits de formule (I) en étant soumis à une ou plusieurs des réactions a) à k) indiquées ci-dessus et détaillées ci-après.

Ainsi les diverses fonctions réactives que peuvent porter certains composés des réactions définies ci-dessus peuvent, si nécessaire, être protégées: il s'agit par exemple des radicaux hydroxyle, acyle, carboxy libres ou encore amino et monoalkylamino qui peuvent être protégés par les groupements protecteurs appropriés.

La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, benzylox-ycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante
- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (IV) et (I') telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuels groupements alkylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio et du réactif tel que notamment un peracide. L'obtention de la fonction sulfone peut être favorisée par un mélange du produit renfermant un groupement alkylthio avec un excès du réactif tel que notamment un peracide.
d) La réaction de transformation d'une fonction cétone en oxime peut être réalisée dans les conditions usuelles connues de l'homme de métier, telle que notamment une action en présence d'une hydroxylamine éventuellement O-substituée dans un alcool tel que par exemple l'éthanol, à température ambiante ou en chauffant.
e) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique. Les éventuelles fonctions carboxy libre des produits décrits ci-dessus peuvent être, si désiré, réduites en fonction alcool notamment par de l'hydrure de bore.
f) Les éventuelles fonctions alcoxy telles que notamment méthoxy des produits décrits ci-dessus peuvent être, si désiré, transformées en fonction hydroxyle dans les conditions usuelles connues de l'homme du métier et notamment selon les conditions décrites ci-après dans la partie expérimentale
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
   J. Organometallic Chemistry., 33, 337 (1971) KOZIMA S.& coll. On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate. Il est entendu que les réactions décrites ci-dessus peuvent être effectuées comme indiqué ou encore, le cas échéant, selon d'autres méthodes usuelles connues de l'homme du métier.
i) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine. On trouvera une liste de différents groupements protecteurs utilisables par exemple dans le brevet BF 2 499 995.
j) Les produits décrits ci-dessus peuvent, si désiré, faire l'objet de réactions de salification par exemple par un acide minéral ou organique ou par une base minérale ou organique selon les méthodes usuelles connues de l'homme du métier.
k) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après. Les produits de formule (I) tels que définis ci-dessus ainsi que leurs sels d'addition avec les acides présentent d'intéressantes propriétés pharmacologiques.

Les produits de la présente invention peuvent ainsi être doués de propriétés inhibitrices d'une ou plusieurs enzymes métaboliques telles que définies ci-dessus notamment de certaines protéines kinases ou protéases.

Plusieurs inhibiteurs de kinases ont été décrits comme la butyrolactone, le flavopiridol et la 2(2-hydroxyéthylamino)-6-benzylamino-9-méthylpurine appelée olomoucine.

Plusieurs inhibiteurs de protéases ont été également décrits, notamment dans 'Journal of Médicinal Chemistry, vol. 43 - n° 3 (D. Leung, G. Abbenante et D.P. Fairlie). Selon une liste non exhaustive, on peut citer par exemple comme inhibiteurs de thrombine : l'argatroloan, le napsagatran, l'inogatran, l'efegatran, le CVS-1123 ainsi que le melagatran.

On peut également citer comme puissants inhibiteurs du facteur Xₐ le DX-9065ₐ, le YM-60828 et le ZK 807-191 ou encore le FX 2212.

On peut citer comme inhibiteurs d'élastase le ICI-200800, le MR 889, le L-658,758, le MDL 101,146, le ZD 8321 ou encore différents dérivés hétérocycliques tels que des pénicillines, des pénèmes, des β-lactames, des isocoumarines, des benzisothiazolones, des alkylazetidinones.

Comme inhibiteurs de tryptases, on peut citer l'APC-366, comme inhibiteurs de "complément convertases" on peut citer le sepimostat mésylate (FUT-187) ou le nafamostat mésylate (FUT 175).

On peut également citer comme puissant inhibiteur de la cathepsine K, le Cbz-Leu-Leu-Leu-CHO, le 1,3-bis(acylamino)-2-propanone ou des dérivés du 1,5-diacylcarbohydrazide ; comme inhibiteurs de caspases, on peut citer des dérivés 5-aminopyrimidine-6-one, des dérivés phenyl kétométhyl éther ou amino méthylène cétone ; comme inhibiteurs de calpaïnes, on peut citer des aldéhydes peptidiques tels que Cbz-Val-Phe-CHO et calpeptine.

Les produits de la présente invention tels que définis ci-dessus, possèdent des propriétés inhibitrices de kinases ou de protéases et notamment de cystéines protéases. Les produits de la présente invention tels que définis ci-dessus, possèdent ainsi notamment des propriétés inhibitrices de cathepsines B, H, J, L, N, S, T, C, V, W, K ou O, 02 et tout particulièrement de cathepsine K.

Les produits de la présente invention ou leurs sels pharmaceutiquement acceptables sont donc notamment utiles pour la prévention ou le traitement de maladies nécessitant l'utilisation d'inhibiteurs de kinases ou de protéases, notamment d'inhibiteurs de cystéines protéases, particulièrement d'inhibiteurs de cathepsines B, H, J, L, N, S, T, C, V, W, K ou O, 02 et tout particulièrement d'inhibiteurs de cathepsine K.

Les niveaux, la régulation et l'activité d'un certain nombre de protéines kinases ou protéases jouent un rôle dans plusieurs pathologies humaines. L'activité d'une protéine kinase peut notamment être associée à des récepteurs possédant des domaines transmembranaires ou à des protéines intracellulaires.

Certaines kinases ou protéases peuvent jouer un rôle dans l'initiation, le développement et l'achèvement des évènements du cycle cellulaire et ainsi, des molécules inhibitrices de telles kinases ou protéases sont susceptibles de limiter des proliférations cellulaires non désirées telles que celles observées dans les cancers, psoriasis, croissance de champignons, de parasites (animaux, protistes): de telles molécules inhibitrices de ces kinases ou protéases sont ainsi également susceptibles d'intervenir dans la régulation de maladies neurodégénératives telles que la maladie d'Alzheimer.

Certains produits de formule (I) de la présente invention peuvent ainsi être doués de propriétés antimitotiques. Certains produits de formule (I) telle que définie ci-dessus peuvent comme inhibiteurs de kinase ou protéase avoir notamment la propriété d'inhiber la résorption osseuse médiée par les ostéoclastes. Ils peuvent donc être utiles pour le traitement thérapeutique ou prophylactique de maladies qui sont causées au moins en partie par une augmentation non désirée de la résorption osseuse, par exemple l'ostéoporose. Certains produits de formule (I) de la présente invention peuvent ainsi par exemple inhiber l'adhésion des ostéoclastes sur la surface de l'os et ainsi la résorption osseuse par les ostéoclastes.

Les maladies de l'os dont le traitement ou la prévention nécessitent l'emploi des composés de formule (I) ou de leurs prodrugs, sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, la maladie de Paget, l'ostéopénie induite par l'immobilisation. En outre les composés de formule (I) peuvent être utilisés pour soulager, empêcher ou traiter les désordres de l'os qui sont causés par les traitements, par les glucocorticoides, les thérapies liées à la prise de stéroides ou de corticostéroïdes ou par les déficiences d'hormones sexuelles mâles ou femelles.

Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.

Certains produits de formule (I) de la présente invention peuvent posséder en plus de leurs propriétés inhibitrices spécifiques de kinases ou protéases, des effets cellulaires intéressants tels que des propriétés antiprolifératives et notamment des effets sur l'apoptose.

On sait par des travaux décrits dans la littérature tel que dans WO 97/20842, que des rapports existent entre le cycle cellulaire et l'apoptose. Parmi les voies conduisant à l'apoptose, certaines sont dépendantes de kinases ou protéases.

Les produits de la présente invention sont notamment utiles pour la thérapie de tumeurs.

Les produits de l'invention peuvent également ainsi augmenter les effets thérapeutiques d'agents anti-tumoraux couramment utilisés.

Les produits de formule (I) de la présente invention possèdent donc tout particulièrement des propriétés antimitotiques et anti-neurodégénératives.

Certains produits de la présente invention peuvent être inhibiteurs d'effets vasoconstricteurs et hypertenseurs et ainsi produire un effet anti-ischémique, ou encore s'opposer à des effets stimulants au niveau de certains types cellulaires notamment les cellules musculaires lisses, les fibroblastes, les cellules neuronales et les cellules osseuses.

Les produits selon la présente invention peuvent ainsi être utilisés dans le traitement de maladies telles que les maladies prolifératives, le cancer, la resténose, l'inflammation; les allergies, les maladies cardiovasculaires ou certaines maladies infectieuses.

Les produits de la présente invention peuvent également être utilisés dans le traitement de certains désordres gastro-intestinaux, gynécologiques et en particulier pour un effet relaxant au niveau de l'utérus.

Ces propriétés justifient leur application en thérapeutique et l'invention a particulièrement pour objet à titre de médicaments, les produits de formule (I) telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a ainsi particulièrement pour objet à titre de médicaments, les produits de formule (I) telle que définie ci-dessus , ainsi que les sels d'addition avec les acides minéraux et organiques ou avec les bases minérales et organiques pharmaceutiquement acceptables desdits produits de formule (I).

L'invention a plus particulièrement pour objet à titre de médicaments, les produits de formule (Ia) ou (Ib) telles que définies ci-dessus , ainsi que les sels d'addition avec les acides minéraux et organiques ou avec les bases minérales et organiques pharmaceutiquement acceptables desdits produits de formule (Ia) ou (Ib).

L'invention a tout particulièrement pour objet, à titre de médicaments, les produits décrits ci-après dans les exemples et notamment les produits de formule (I) telle que définie ci-dessus, répondant aux formules suivantes:
- le 2-[(2S)-4-méthyl-1-oxo-2-[[(phénylméthoxy)carbonyl] amino]pentyl]-hydrazide de l'acide 5-[(2S)-1-[[4-méthyl-1-oxo
- 2-[[(phénylméthoxy)carbonyl]amino]pentyl]amino]éthyl]-2-thiophènecarboxylique
- le 2-[[(9H-fluoren-9-yl)méthoxy]carbonyl]-hydrazide de l'acide 5-[1-(hydroxyimino)éthyl]-2-thiophènecarboxylique
- le 2-[(2S)-4-méthyl-1-oxo-2-[[(phénylméthoxy)carbonyl] amino]pentyl]-hydrazide de l'acide 5-[2-(phénylméthoxy) phényl]-2-thiophènecarboxylique
- le 2-[(2S)-4-méthyl-l-oxo-2-[[(phénylméthoxy)carbonyl] amino]pentyl]-hydrazide de l'acide 5-[[[3-(4-méthoxyphènoxy) phényl]méthyl](phénylméthyl)amino]-2-thiophènecarboxylique
- le 2-[(2S)-4-méthyl-1-oxo-2-[[(phénylméthoxy)carbonyl]-amino] pentyl]-hydrazide de l'acide 5-[1-[(phénylméthoxy) imino]éthyl]-2-thiophènecarboxylique
- le 2-[[(9H-fluoren-9-yl)méthoxy]carbonyl]- hydrazide de l'acide 5-[1-[[(phénylméthoxy)carbonyl]amino]éthyl]-2-thiophènecarboxylique
- le 2-[(2S)-4-méthyl-1-oxo-2-[[(phénylméthoxy)carbonyl]-amino] pentyl]-hydrazide de l'acide 5-[1-[[(phénylméthoxy)-carbonyl] amino]éthyl]-2-thiophènecarboxylique
- le 2-[(2S)-4-méthyl-1-oxo-2-[[(phénylméthoxy)carbonyl]-amino] pentyl]- hydrazide de l'acide 5-[3-méthyl-1-[(phénylméthoxy) carbonyl]amino]butyl]-2-thiophènecarboxylique
- le 2-[(2S)-4-méthyl-1-oxo-2-[[(phénylméthoxy)carbonyl]-amino] pentyl]-hydrazide de l'acide 5-[3-méthyl-1-[[(phénylméthoxy) carbonyl]amino]butyl]-2-thiophènecarboxylique isomère A
- le [(2S)-4-méthyl-1-oxo-2-[[(phénylméthoxy)carbonyl]amino] pentyl]-hydrazide de l'acide 5-[3-méthyl-1-[[(phénylméthoxy) carbonyl]amino]butyl]-2-thiophènecarboxylique Isomere B ainsi que les sels d'addition avec les acides minéraux et organiques ou avec les bases minérales et organiques pharmaceutiquement acceptables desdits produits de formule (I).

Les produits de formule (I) de la présente invention peuvent donc notamment être utilisés sous forme de médicaments pour inhiber un mécanisme non désiré provoquant une pathologie sous l'effet d'une ou plusieurs protéase(s) ou kinase(s) : la méthode peut ainsi consister en l'administration au patient dont le traitement requiert l'inhibition de l'effet de telle protéase ou kinase, d'une quantité inhibitrice d'un tel médicament selon la présente invention.

Les médicaments objet de l'invention peuvent ainsi être utilisés dans le traitement et la prévention d'affections cardiovasculaires associées à une altération de la vasomotricité ou de la volémie, de l'infarctus du myocarde et ses conséquences, l'insuffisance cardiaque, l'insuffisance rénale, l'angine de poitrine, l'hyperaldostéronisme, hypertension artérielle et ses conséquences , le traitement et la prévention des complications en particulier les hypertrophies cardiaques et vasculaires ainsi que le développement de fibroses au niveau des organes cibles, prévention et traitement de l'hypertension, de désordres métaboliques, endocrinologiques et neurologiques, des chocs endotoxiques, des hémorragies 'subarachnoid', de l'arythmie, de l'asthme, de l'insuffisance rénale aiguë ('acute renal failure'), de 'preeclampsia' et de diabètes.

Les médicaments objet de l'invention peuvent ainsi être utilisés dans le traitement de spasmes vasculaires, dans le traitement des suites d'une hémorragie cérébrale, dans le traitement des spasmes coronariens, des spasmes vasculaires périphériques. Ces médicaments peuvent notamment être utilisés dans le traitement de l'insuffisance cardiaque congestive, dans la prévention des resténoses post-angioplastie, la prévention des fibroses cardiaques et vasculaires, dans le traitement de l'athérosclérose et de certaines formes d'hypertension comme l'hypertension pulmonaire ainsi que dans le traitement de l'asthme.

Ces médicaments objet de l'invention pourraient également être utilisés pour le traitement du glaucome et de différents types de spasmes viscéraux, ainsi qu'à titre de substances protectrices neuronales ou encore dans la prévention des resténoses post-angioplastie.

Notamment les médicaments objet de l'invention peuvent être utilisés pour leurs effets anti-hypertrophique et anti-fibrotique aux niveaux cardiaque et vasculaire. Tout particulièrement, ils peuvent être utilisés pour le traitement et la prévention des désordres cardiovasculaires associés au diabète.

Les médicaments objet de la présente invention peuvent également trouver une application dans le traitement de l'ostéoporose et en tant que protecteurs neuronaux.

Les médicaments objet de l'invention peuvent également être utilisés dans le traitement des troubles de la mémoire et des fonctions cognitives ainsi que de l'anxiété.

Les médicaments, objet de l'invention, peuvent également trouver, comme antimitotiques, leur emploi dans la chimiothérapie des cancers, ou encore dans le traitement de psoriasis, de parasitoses telles que celles dues à des protistes ou à des champignons ou encore dans le traitement de la maladie d'Alzheimer ou dans le traitement de l'apoptose neuronale.

L'invention s'étend aux compositions pharmaceutiques contenant à titre de principe actif l'un au moins des médicaments tels que définis ci-dessus.

De telles compositions pharmaceutiques de la présente invention peuvent également, le cas échéant, renfermer des principes actifs d'autres médicaments antimitotiques tels que notamment ceux à base de taxol, cis-platine, les agents intercalants de l'ADN et autres.

Ces compositions pharmaceutiques peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 à 5 g par jour chez l'adulte, ou de préférence de 0,1 à 2 g par jour.

L'invention a tout particulièrement pour objet les compositions pharmaceutiques telles que définies ci-dessus caractérisées en ce qu'elles renferment un ou plusieurs produit(s) de formule (I) telle que définie ci-dessus: de telles compsitions pharmaceutiques sont ainsi caractérisées en ce qu'elles renferment un ou plusieurs inhibiteurs de kinases ou de protéases telles que définies ci-dessus et qu'elles sont utilisées comme médicament pour les applications thérapeutiques indiquées ci-dessus.

L'invention a particulièrement pour objet l'utilisation des produits de formule (1) telle que définie ci-dessus pour la préparation de compositions pharmaceutiques destinées au traitement ou à la prévention d'affections nécessitant une inhibition d'une ou plusieurs enzymes métaboliques telles que définies ci-dessus.

L'invention a ainsi particulièrement pour objet l'utilisation des produits de formule (I) telle que définie ci-dessus pour la préparation de compositions pharmaceutiques destinées au traitement de maladies résultant d'anomalies dans les niveaux, la régulation ou l'activité d'un certain nombre d'enzymes métaboliques. De telles anomalies jouent un rôle dans certaines pathologies humaines qui intéressent particulièrement la présente invention.

La présente invention concerne donc notamment l'utilisation des produits de formule (I) de la présente invention comme inhibiteurs de protéases ou kinases. La présente invention concerne ainsi l'inhibition de certaines de telles enzymes protéases ou kinases et revendique l'emploi de produits de la présente invention comme inhibiteurs dans les cas où l'inhibition de telle protéase ou kinase est indiquée. L'invention a ainsi tout particulièrement pour objet l'utilisation des produits de formule (I) telle que définie ci-dessus ou de sels pharmaceutiquement acceptables desdits produits de formule (I) pour la préparation de médicaments destinés à la prévention ou au traitement de médicaments destinés à la prévention ou au traitement de maladies dans lesquelles des enzymes métaboliques telles que des protéases ou des kinases sont impliquées.

La présente invention a ainsi pour objet l'utilisation de produits de formule (I) telle que définie ci-dessus ou de sels pharmaceutiquement acceptables desdits produits de formule (I) pour la préparation de médicaments destinés à la prévention ou au traitement de maladies liées à un comportement physiologique anormal au niveau de la sécrétion et/ou de l'activité de cystéines protéases telles que notamment les cathepsines B, H, J, L, N, S, T, C, V, W, K ou O, 02 ou la papaïne.

La présente invention a plus précisément pour objet l'utilisation de produits de formule (I) telle que définie ci-dessus ou de sels pharmaceutiquement acceptables desdits produits de formule (I) pour la préparation de médicaments destinés à la prévention ou au traitement de maladies liées à un comportement physiologique anormal au niveau de la sécrétion et/ou de l'activité de la cathepsine K.

La présente invention a notamment pour objet l'utilisation de produits de formule (I) telle que définie ci-dessus ou de sels pharmaceutiquement acceptables desdits produits de formule (I) comme inhibiteurs de cystéine protéase cathepsine K.

La présente invention a ainsi notamment pour objet l'utilisation de produits l'utilisation de produits de formule (I) telle que définie ci-dessus ou de sels pharmaceutiquement acceptables desdits produits de formule (I) pour la préparation de médicaments destinés à la prévention ou au traitement de maladies nécessitant l'utilisation d'inhibiteurs de cystéine protéase cathepsine K.

La présente invention a particulièrement pour objet l'utilisation de produits de formule (I) telle que définie ci-dessus ou de sels pharmaceutiquement acceptables desdits produits de formule (I) pour la préparation de médicaments destinés à la prévention ou au traitement de maladies du métabolisme du cartilage et de l'os, les maladies prolifératives, les cancers, les maladies cardiovasculaires, la resténose, les maladies du système nerveux central, les maladies du système immunitaire, les allergies, les maladies infectieuses, inflammatoires et les maladies autoimmunes.

L'invention a ainsi tout particulièrement pour objet l'utilisation des produits de formule (I) telle que définie ci-dessus pour la préparation de compositions pharmaceutiques destinées au traitement de l'hypertension artérielle, de l'insuffisance cardiaque, des resténoses post-angioplastie,. de spasmes vasculaires, des suites d'une hémorragie cérébrale et des insuffisances rénales.

L'invention a ainsi tout particulièrement pour objet l'utilisation des produits de formule (1) telle que définie ci-dessus pour la préparation de compositions pharmaceutiques destinées au traitement de l'infarctus du myocarde, à la prévention des resténoses post-angioplastie et à la prévention des fibroses cardiaques et vasculaires. L'invention a ainsi tout particulièrement pour objet l'utilisation des produits de formule (1) telle que définie ci-dessus pour la préparation de compositions pharmaceutiques destinées au traitement de l'insuffisance rénale.

L'invention a ainsi tout particulièrement pour objet l'utilisation des produits de formule (1) telle que définie ci-dessus pour la préparation de compositions pharmaceutiques destinées au traitement et à la prévention des désordres cardiovasculaires associés au diabète.

L'invention a notamment pour objet l'utilisation des produits de formule (I) telle que définie ci-dessus pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

La présente invention a plus particulièrement pour objet l'utilisation telle que définie ci-dessus caractérisée en ce que les maladies à prévenir ou à traiter sont choisies parmi les maladies du métabolisme du cartilage et de l'os, les maladies prolifératives, les cancers et les maladies neurodégénératives comme la maladie d'Alzheimer.

La présente invention a tout particulièrement pour objet l'utilisation telle que définie ci-dessus caractérisée en ce que les maladies à prévenir ou à traiter sont choisies parmi les maladies du métabolisme du cartilage et de l'os et les cancers osseux.

La présente invention a notamment pour objet l'utilisation telle que définie ci-dessus caractérisée en ce que les maladies à prévenir ou à traiter sont notamment l'ostéoporose, les maladies gingivales incluant gingivite et périodontosis, l'arthrite telle que notamment l'ostéoarthrite et l'arthrite rhumatoïde, la maladie de Paget, l'hypercalcémie et les cancers osseux.

La présente invention a ainsi pour objet l'utilisation telle que définie ci-dessus caractérisée en ce que la maladie à prévenir ou à traiter est notamment l'ostéoporose.

La préparation des produits de formule (I) telle que définie ci-dessus met en oeuvre des composés de départ décrits ci-dessus de formules diverses telles que les composés de formule (II) dans laquelle W représente un atome d'hydrogène, un atome d'halogène ou un radical nitro, les alcools de formules (III) et (VI), les aldéhydes de formule (VIII), des amines de formule (X), des diamines de formule (XV) (R1'-CO-NR3-NR4-), des dérivés borés de formule (XVII) (R2'-B-(OH)2), des diamines de formule (XIX), des réactifs de formule (XXII) et des acides de formule (XXIV) (R1'-COOH).

Parmi de tels produits de départ, certains sont connus et peuvent être obtenus commercialement. Certains produits de départ peuvent être préparés selon les méthodes usuelles connues de l'homme du métier.

On peut encore notamment préparer certains produits de départ à partir de produits de commerciaux par exemple en les soumettant à une ou plusieurs des réactions décrites ci-essus en a) à k), réalisées dans les conditions également décrites ci-dessus.

Les produits de départ de formule (XXIV) peuvent être des acides aminés naturels ou non naturels commerciaux ou préparés selon les méthodes usuelles connues de l'homme du métier.

On peut citer notamment les acides aminés suivants : Glu, Gly, Ala, Val, Phe ou Tyr, qui sont éventuellement sous une forme protégée par exemple par un groupe Fmoc, alloc ou terbutyle.

On peut notamment à partir d'acides aminés dits naturels ou non naturels fixer un radical Fmoc sur la fonction amine N-terminale de l'acide aminé concerné : une telle réaction est notamment décrite dans l'article dont la référence suit : 'Mueller, A. ;Vogt,C. ; Sewald,N. ; Synthesis (1998),(6),837-841'.

On trouve également dans le commerce un grand nombre d'acides aminés sous forme protégée fmoc et qui peuvent alors constituer des produits de départ pour le procédé de la présente demande. On peut citer notamment Fmoc-Leu, Fmoc-L-Glu(Alloc); Fmoc-Gly; Fmoc-Ala; Fmoc-Val; Fmoc-Phe ou encore Fmoc-Tyr(OtBu).

La partie expérimentale ci-après donne des exemples de tels produits de départ.

Les exemples suivants illustrent l'invention sans toutefois la limiter.

### Partie expérimentale:

### Exemple 1: 2-[(2S)-4-méthyl-1-oxo-2-[[(phénylméthoxy)-carbonyl] amino]pentyl]-hydrazide de l'acide 5-[(2S)-1-[[4-méthyl-1-oxo -2-[[(phénylméthoxy)carbonyl]amino]pentyl]-amino]éthyl]-2-thiophènecarboxylique

### Stade 1 : Acide 5-[1-(hydroxyimino)éthyl]-2-thiophène carboxylique

A une solution de 15 ml de pyridine sous argon, contenant 2,25 g d'acide 5-acetyl-2-thiophènecarboxylique, on ajoute 1,84 g de chlorhydrate d'hydroxylamine. Le milieu est porté à 100°C pendant huit heures puis concentré à sec, repris par une solution de 20 ml de HCl 2N, extrait par 50 ml d'acétate d'éthyle, séché sur MgSO₄, filtré et évaporé sous vide. On purifie sur silice avec pour éluant CH₂Cl₂/MeOH/CH₃COOH :
95/5/0,5 et obtient ainsi 0,450 g du produit attendu.
RMN (1H,DMSO) :
2,17(s,3H) ; 2,28(s,3H) ; 7,37(d, 1H) ; 7,49(d,1H) ; 7,55(d, 1H) ; 7,70(d, 1H) ; 11,57(s, 1H) ; 12, 13 (sl, 1H) ; 13, 08 (sl, 1H) .

### Stade 2 : [(2,2-diméthyléthoxy)carbonyl]-hydrazide de l'acide 5-[1-(hydroxyimino)éthyl]-2-thiophènecarboxylique

A une solution de 2ml de DMF et 3 ml de CH₂Cl₂ contenant le produit obtenu au stade 1 ci-dessus, on ajoute vers 0-5°C (bain glace sel) 0,28 g de HOBT puis 0,39 g d'EDC.

Le milieu réactionnel est ramené à température ambiante et conservé 30 minutes sous agitation.

La Boc-hydrazine en solution dans 1 ml de CH₂Cl₂ est ajoutée vers 0-5°C. Le milieu est conservé pendant 3 heures à température ambiante. Après évaporation sous vide, le résidu est repris par 50 ml d'acétate d'éthyle, lavé par 25 ml d'une solution aqueuse (pH = 4). La solution d'acétate d'éthyle est décantée puis lavée par 201 d'une solution aqueuse. La phase organique est ensuite séparée, séchée et évaporée sous vide. Le résidu est purifié par chromatographie sur silice avec pour éluant une solution dichlorométhane/méthanol : 90/10. On obtient ainsi 0,460 g du produit attendu.
RMN(1H,DMSO)
1,47(s,9H) ; 2,17(s,3H) ; 2,27(s,3H) ; 7,37(d,1H) ; 7,50(d,1H) ; 7,71(d,1H) ; 7,77(d,1H) ; 8,95(sl,1H) ; 10, 24 (sl, 1H) ; 10,27(s,1H) ; 11, 51 (s, 1H) ; 12,0(s,1H).

### Stade 3 : di trifluoroacetate de hydrazide de l'Acide 5-(1-aminoethyl)-2-thiophènecarboxylique

On procède à une réduction de l'oxime et déprotection du carbamate comme suit.

A une solution sous argon à température ambiante contenant 0,2 g du dérivé oxime obtenu au stade 2 ci-dessus, on ajoute 0,44 g de Zn. La suspension obtenue est conservée 1 heure puis filtrée sur célite. La solution filtrée est concentrée puis reprise par 50 ml d'éther éthylique. Le précipité obtenu est filtré. On obtient ainsi 0,20 g du produit attendu.

### Stade 4 : 2-[(2S)-4-méthyl-1-oxo-2-[[(phénylméthoxy)carbonyl] amino]pentyl]-hydrazide de l'acide5-[(2S)-1-[[4-méthyl- 1-oxo-2-[[(phénylméthoxy)carbonyl]amino]pentyl]amino]éthyl]-2-thiophènecarboxylique

A une solution de 2 ml de DMF et 2 ml de CH₂Cl₂ contenant 0,35 g de dérivé Z-Leu, on ajoute 0,18 g de HOBT puis 0,26 g d'EDC à 0°C. Le mélange est ensuite ramené à température ambiante pendant 30 minutes, et on ajoute 0,24g de dérivé thiophène obtenu au stade 3 ci-dessus en solution dans 2 ml de DMF. Ce mélange est conservé à température ambiante pendant 12 heures, puis évaporé sous vide, repris par 50 ml d'acétate d'éthyle, lavé par 25 ml d'une solution HCl 2N puis par 25 ml d'une solution saturée NaCl. On sèche sur MgSO₄, filtre et purifie par chromatographie en éluant par une solution de dichlorométhane/méthanol : 95/5. On récupère ainsi 0,070 g du produit attendu.
RMN(1H,DMSO)
0,86(m,12H) ; 1,32 à 1,78(m,6H) ; 4,06(m,1H) ; 4,16(m,1H) ; 5,03(s,4H) ; 5,14(m,1H) ; 6,96(d,1H) ; 7,0(d,1H) ; 7,35(m,10H) ; 7,66(d,1H) ; 7,67(d,1H); 7,39(d,1H) ; 7,52(d, 1H) ; 8,52(d,1H) ; 8,56 (d, 1H) ; 10, 04 (sl, 1H) ; 10, 32 (sl, 1H) .

### Exemple 2 : 2-[[(9H-fluoren-9-yl)méthoxy]carbonyl]-hydrazide de l'acide 5-[1-(hydroxyimino)éthyl]-2-thiophènecarboxylique

A une solution de 5 ml de CH₂Cl₂ et 3 ml de DMF à 0°C (bain glace -sel) contenant 0,73 g du produit obtenu au stade 1 de l'exemple 1, on ajoute 0,53 g de HOBT puis 0,75 g d'EDC. Le mélange est conservé après retour à T.A pendant 20 minutes. On ajoute à cette solution 1 g de Fmoc-hydrazine en solution dans 5 ml de DMF à 0-5° C (bain glace-sel). Le milieu est ensuite ramené à T.A et conservé tel que pendant 6 heures, puis évaporé à sec, repris par 50 ml d'acétate d'éthyle et 25 ml d'H₂O. La solution d'acétate d'éthyle est décantée, séchée sur MgSO₄, concentrée, purifiée par chromatographie sur silice en éluant par une solution CH₂Cl₂/MeOH/CH₃COOH :
98/2/0,5 pour donner 1,42 g du produit attendu.
RMN(1H,DMSO) :
2,17(s,3H) ; 2,28(s,3H) ; 4,23(m,3H) ; 4,28(s1,1H); 4,40(d1,1H) ; 7,05 à 7,70(m,2H) ; 7,35(t1,2H) ; 7,45(t1,2H) ; 7,75(d1,2H) ; 7,91(d1,2H) ; 9,09(s,1H) ; 9,44(s,1H) ; 10, 39(s,1H) ; 10,62(s,1H) ; 11,52(s,1H), 12,04(s,1H).

### Exemple 3 : 2- [(2S)-1-oxo-2-[[(phénylméthoxy)carbonyl]-amino]pentyl]-hydrazide de l'acide 5-(4-méthoxyphényl)-2-thiophènecarboxylique

Stade 1 : 5-bromo-2-thiophènecarboxylate d' ethyle

On agite 10 g d'acide 5-bromo-2-thiophènecarboxylique (48,29mM 1 équivalent) dans 17,6 ml de SOC12 (24mM 5 équivalents) pendant 1 heure 30 à 100°C. On évapore à sec en reprenant avec 2 fois 20ml de toluène. Le produit brut obtenu est agité 2 heures au reflux dans 40 ml d'EtOH. On évapore à sec, reprend dans CH₂Cl₂ et lave à l'eau, puis sèche sur MgSO₄ et évapore à sec. On obtient ainsi 10,95 g (96%) de produit attendu sous forme de liquide marron.

Stade 2 : 5-(4-méthoxyphényl)-2-thiophènecarboxylate d'ethyle A une solution de 2,0 g d'ester bromé (8,5mM 1 équivalent) obtenu au stade 1 ci-dessus dans 48 ml de toluène et 74 ml d'EtOH, on ajoute 2,58 g (17,01mM 2 équivalents) d'acide boronique et 18 ml de carbonate de sodium 2M et on dégaze 5 minutes avec N₂. Puis on ajoute 1,21 g de Pd(PØ₃)₄ (1,05mM-0,124 équivalents). On agite pendant 3 heures à 80°C (T.ext). On ajoute 200 ml d'H₂O, extrait avec 100 ml de AcOEt, sèche sur MgSO₄ et évapore à sec. On obtient ainsi 5,28 g de produit brut que l'on purifie sur colonne de silice avec pour éluant cyclohexane : 98/ACOEt : 2 puis 95/5. On obtient ainsi 2,22 g de produit attendu.

Stade 3 : hydrazide de l'acide 5-(4-méthoxyphényl)-2-thiophènecarboxylique

On agite 200 ml d'ester (0,762mM 1 équivalent) obtenu au stade 2 ci-dessus au reflux dans 5 ml de DMF et 1,1 ml d'hydrate d'hydrazine (22,8mM, 30éq) pendant 24 heures puis on évapore à sec.

Après purification sur colonne de silice avec pour éluant (CH₂Cl₂ : 5 AcOEt : 5 puis CH₂Cl₂:9/MeOH:1/NH₄OH:0,1, on obtient 245 mg de produit attendu.

Stade 4 : 2- [(2S)-1-oxo-2-[[(phénylméthoxy)carbonyl]amino] pentyl]-hydrazide de l'acide 5-(4-méthoxyphényl)-2-thiophènecarboxylique

A une solution de 97,0 mg de ZLeu-OH (0,366Mm 1,3 équivalent) dans 5 ml de DMF sur siliporite et refroidie à 0°C, on ajoute 70 mg de cétohydrazide (0,282mM 1 équivalent) obtenu au stade 3 ci-dessus, 70 mg de EDC (0,366mM 1,3 équivalent) et 49 mg de HOBT (0,366mM 1,3 équivalent).

On agite 2 heures à 0°C et 2 heures à TA puis on évapore le DMF, reprend dans 20 ml de Et₂O, lave avec 20 ml d'une solution saturée de Na₂CO₃, lave avec 20 ml d'eau, sèche sur MgSO₄ et évapore à sec.

On obtient 268 mg de produit brut que l'on purifie sur colonne de silice avec pour éluant Cyclohexane:5/AcOEt:5), et on obtient ainsi 73 mg (68%) de produit attendu.
IR :
NH: - 3403 ; ~ 3289 cm⁻¹
C=0: 1708 ; 1633 cm⁻¹
Hétérocycles, aromatiques et Amide II : 1611, 1573, 1539, 1505 cm⁻¹;
RMN (H,CDCl3)
0,93 (cm) 6H : (CH₃)₂-CH
1,56 à 1,78 (cm) : CH₂-CH(CH₃)₂
3,82 (s) 3H : OCH₃
4,46 (m) 1H : NH-CH
5,03, 5,13 : (2H) AB : OCH₂ø
5,57 (d) 1H: NH-CH
6,87, 7,47 (4H): AA'BB'
~7,30 (m) phényle
7,08 (1) 1H : H₄
7,58 (1) 1H : H₃
9,14, 9,31 : 2H mobiles

### Exemple 4 : 2- [(2S)-1-oxo-3-phényl-2-[[(phénylméthoxy)-carbonyl] amino]propyl]-hydrazide de l'acide 5-(4-méthoxyphényl) -2-thiophènecarboxylique

On opère comme au stade 4 de l'exemple 3 à partir de 188 mg (0,757mM, 1éq)de cétohydrazine obtenu au stade 3 de l'exemple 3, 218 mg (1,13mM 1,5 équivalent) de EDC, 340 mg (1,13mM 1,5 équivalent) de ZPheOH et 154 mg (1,13mM 1,5éq) de HOBT. On obtient ainsi 934 mg de produit brut que l'on purifie sur silice avec pour éluant CH₂Cl₂ :9/AcOEt:1.

On obtient ainsi 225 mg (73%) de produit attendu sous forme de 2 isomères C et D en proportions respectives de 85% et 15%.
RMN (1H, DMSO)
Pour C, on localise:
2,82(m), 3,08(m): phényl-CH₂-CH
4,38(td) : phényl- CH₂-CH
3,81 (s) : CH₃-O-phényl
7,02, 7,67: AA'BB'
7,46 (d), 7,83 (d): Ha et Hb

Pour D, on localise :
4,11(sl): phényl-CH₂-CH
3,05 (m), 2,84 (m) : phényl-CH₂-CH
Pour C et D, on localise :
4, 95 (m) : phényl-CH₂-O
7,10 à 7,40(m): H aromatiques
7,69 (masqué) : NH-CH-CH2-
10,28(sl), 10,48(sl): H mobiles

### Exemple 5 : 2-[(2S)-4-méthyl-1-oxo-2-[[(phénylméthoxy)-carbonyl] amino]pentyl]-hydrazide de l'acide 5-[2-(phénylméthoxy) phényl]-2-thiophènecarboxylique

### Stade 1 : Acide 2-(phénylméthoxy)phényl- Boronique

1) A une solution de 8,4 g de dérivé bromé (N8) (31,9mM 1 équivalent) dans 50 ml de THF, on ajoute à -78°C à la seringue 19,93 ml de BuLi (1,6M dans l'hexane) (31,9mM 1 équivalent) et on agite 30 minutes à -78°C. On obtient ainsi une solution contenant le dérivé lithié que l'on ajoute avec une ampoule à brome à -78°C dans une solution de 30 g de B(OiPr)₃ (159,5mM 5 équivalents) dans 50 ml de THF. Puis on agite en laissant remonter à TA pendant 4 heures. On verse sur 20 ml de HCL 3N, extrait 2 fois avec 50 ml d'AcOEt, lave avec eau, sèche sur MgSO₄ et évapore à sec. On obtient 8,53 g de produit brut que l'on purifie sur silice avec pour éluant cyclohexane:9/AcOEt:1. On obtient ainsi 3,6 g (47%)de produit pur.

### Stade 2 : 5-[2-'(phénylméthoxy)phényl]-2-Thiophènecarboxylate d'ethyle

On procède dans les mêmes conditions expérimentales décrites au stade 2 de l'exemple 3 à partir de 1,0 g (4,23mM 2 équivalents) d'acide boronique obtenu au stade 1 ci-dessus, 500 mg (2,11 mM 1 équivalent) de bromo thiophène obtenu au stade 1 de l'exemple 3, 307 mg (0,266mM 0,125éq) de Pd(Pø₂)₄, 12 ml de toluène, 18 ml de EtOH et 4,6 ml de Na₂CO₃ 2M. On obtient ainsi 1,79 g de produit brut que l'on purifie sur silice avec pour éluant (cyclohexane:99/AcOEt:1). On obtient ainsi 760 mg de produit attendu.

### Stade 3 : Hydrazide de l'acide 5-[2-(phénylméthoxy)phényl]-2-Thiophènecarboxylique

A une solution de 713 mg d'ester thiophène obtenu au stade 2 ci-dessus (2,1mM 1 équivalent) dans 20 ml d'EtOH, on ajoute 4,2 g d'hydrate d'hydrazine (84mM 40éq) et on agite à reflux pendant 48 heures. On évapore à sec et l'on purifie sur silice avec pour éluant CH₂Cl₂:5/ACOEt:5 puis CH₂Cl₂:9/MeOH:1/NH₄OH:0,1. On obtient ainsi 540 mg de produit attendu.

### Stade 4 : 2-[(2S)-4-méthyl-1-oxo-2-[[(phénylméthoxy)carbonyl] amino]pentyl]-hydrazide de l'acide5-[2-(phénylméthoxy)phényl] -2-thiophènecarboxylique

On procède dans les mêmes conditions opératoires que celles décrites au stade 4 de l'exemple 3 à partir de 95 mg (0,293mM 1 équivalent) de Cétohydrazide obtenu au stade 3 ci-dessus, 117 mg (0,439mM 1,5éq) de ZLeuOH, 8,4 mg (0,439mM 1,5éq) de EDC et 59 mg (0,439mM 1,5éq) de HOBT. On obtient ainsi 196 mg de produit brut que l'on purifie sur silice avec pour éluant (CH₂Cl₂:8/AcOEt:2). On obtient ainsi 140 mg de produit attendu.
RMN (H, CDCl₃₎
0,93(se)6H: (CH₃)₂-CH-CH2-CH-NH
1,61(masqu.), 1,73(m)2H : (CH₃)-CH-CH₂-CH-NH
4,43(m)1H: (CH₃)-CH-CH₂-CH-NH
5,45(d1)1H : (CH₃)-CH-CH₂-CH-NH
5, 08, 5, 14, 5,22(sl)4H: les O-CH₂-phényl
6,97(tl)2H: H₃ et H₄
7,25(m): H₂
7,63(dd)1H: H₅
7,44(d), 7,60 (d): Ha et Hb
7,21 à 7,46(m)10H aromatiques
8,80(sl, 9,16(sl): 2H NH

### Exemple 6: 2-[(2S)-2-[[(1,1-diméthyl)éthoxy]carbonyl]amino]-4-méthyl-1-oxo-pentyl]-hydrazide de l'acide 5-[2-(phénylméthoxy) phényl]-2-thiophènecarboxylique

On procède dans les mêmes conditions opératoires que celles déjà décrites au stade 4 de l'exemple 3 à partir de 336 mg (1,035mM 1 équivalent)de Cétohydrazide obtenu au stade 3 de l'exemple 5, 387 mg (1,55mM 1,5éq) de Boc-Leu-OH , 298 mg (1,55mM 1,5éq) de EDC et 210 mg (1,55mM 1,5éq) de HOBT. On obtient 635 mg de produit brut que l'on purifie sur colonne de silice avec pour éluant CH₂Cl₂:9/AcOEt:1. On obtient ainsi 439 mg de produit attendu.
RMN (1H,CDCl₃)
0, 95 (d), 0, 97 (d) 6H : (CH₃)₂-CH-CH₂
1,59(m), 1,75(m) 3H : (CH₃)₂-CH-CH₂
1,46(s) 9H O-C(CH₃)₃
4,33(sl) 1H : NH-CH-CO
5,03(dl) 1H : NH-CH-CO
5,23(AB) 2H : O-CH2-phényle
7,46(d), 7,60(d) : 2H Ha et Hb
6,99 (d), 7,65(m), 7,25 (masqué) 4H : H₂,H₃,H₄ et H₅
7,33(t) 1H Hc'
7,37(tl) 2H Hb'
7,41(m) 2H Ha'

### Exemple 7 : 2-((2S)-2-amino-4-méthyl-1-oxo-pentyl)-hydrazine de l'acide 5-(2-(phénylméthoxy)phényl) 2 thiophène carboxylique

On procède dans les mêmes conditions opératoires que celles décrites à l'exemple 10 à partir du produit de l'exemple 6 au lieu du produit de l'exemple 9 et on obtient ainsi le produit attendu.

### Exemple 8: 2-[(2S)-4-méthyl-1-oxo-2-[[(phénylméthoxy)-carbonyl] amino]pentyl]-hydrazide de l'acide 5-(1-naphthalényl) -2-thiophènecarboxylique

Stade 1 : 5-(1-naphthalenyl)-2-Thiophènecarboxylate d'éthyle On opère dans les mêmes conditions opératoires précédemment décrites au stade 2 de l'exemple 3 à partir de 1,83 g de bromoester ester 5-bromo-2-thiophenecarboxylate d' ethyle, obtenu au stade 1 de l'exemple 3 (7,78mM 1 équivalent), 2,67 g (15,56mM 2éq) de Naphtalène boronic acid N11, 1,124 g (0,97mM 0,125 éq) de Pd(Pø₃)₄, 44 ml de toluène, 67 ml d'EtOH et 17 ml de Na₂CO₃ 2M. On obtient ainsi 4,88 g de produit brut et que l'on purifie sur colonne de silice avec pour éluant Cyclohexane:98/AcOEt:2. On obtient ainsi 486 mg (22%)de produit purifié.

Stade 2 : Hydrazide de l'acide 5-(1-naphthalenyl)-2-Thiophènecarboxylique

On procède dans les mêmes conditions opératoires que celles décrites précédemment au stade 3 de l'exemple 3 à partir de 430 mg d'ester thiophène (1,52mM 1 équivalent) obtenu au stade 1 ci-dessus, 2,28 g (45,6mM 30éq) d'Hydrate d'hydrazine et 15 ml d'EtOH. On purifie après évaporation sur silice avec pour éluant CH₂Cl₂:5/AcOEt:5 puis CH₂Cl₂:9/MeOH:1/NH₄OH:0,1. On obtient ainsi 306 mg (75%) de produit attendu.

Stade 3 : 2-[(2S)-4-méthyl-1-oxo-2-[(phénylméthoxy)carbonyl] amino]pentyl]-hydrazide de l'acide 5-(1-naphthalényl)-2-thiophènecarboxylique

On opère dans les mêmes conditions opératoires que celles précédemment décrites au stade 4 de l'exemple 3 à partir de 90 mg (0,336mM 1 équivalent) de cétohydrazide thiophène obtenu au stade 2 ci-dessus, 133 mg (0,502mM 1,5 équivalent) de Zleu OH, 96 mg (0,502mM 1,5éq) de EDC, 68 mg (0,502mM 1,5éq) de HOBT et 5 ml de DMF. On obtient ainsi 302 mg de produit brut que l'on purifie sur silice avec pour éluant Cyclohexane:6/AcOEt:4. On obtient ainsi 150 mg de produit attendu.
RMN(1H, CDCl3)
0,94(d), 0,96(d): les CH3 de iPr
1,75(m) : CH de iPr
1,75 ; 1,64 : CH2-CH-NH-COO-
4,47(m): CH2-CH-NH-COO-
5,54 : CH2-CH-NH-COO-
5,15(d,J=12) ; 5,06(d,J=12) : OCH2-C=
7,31(m) : H aromatiques
7,16(d,5=4): H₄
7,75(d,5=4): H₃
7,41 à 7,51 :4H ; 7,86(m) :H₂' et H₅'; 8,10(d,5=8,5) :H₈'; H de naphtyl ;
9,20(sl), 9,31(sl) : NH-NH
IR dans CHCl₃
NH : 3400, 3288 cm⁻¹
C=O : 1707, 1635 cm⁻¹
Hétérocycle+Aromatique+Amides II = 1593, 1534, 1509 ;
1500cm⁻¹

### Exemple 9 : 2-[(2S)-2-[[[(1,1-diméthyl)éthoxy]carbonyl]-amino]-4-méthyl-1-oxo-pentyl]-hydrazide de l'acide 5-(1-naphthaléayl)-2-thiophènecarboxylique

On procède dans les mêmes conditions opératoires que celles précédemment décrites au stade 4 de l'exemple 3 à partir de 247 mg du produit obtenu au stade 2 de l'exemple 8 et en utilisant Boc-Leu 0H au lieu de ZleuOH. On obtient ainsi 890 mg de produit brut que l'on purifie sur silice avec pour éluant cyclohexane:7/AcOEt:3.

On obtient ainsi 337 mg (76%) de produit attendu.
RMN (H, CDCl3)
0,96(d), 0,98(d) : 6H CH-CH₂-CH-(CH₃)₂
1,76(m) : CH-CH₂-CH-(CH₃)₂
1,76, 1,62 : CH-CH₂-CH-(CH₃)₂
4,39(s1) : 1H CH-CH₂-CH-(CH₃)₂
5,14(d1) : 1H NH-CH-CH₂
7,17(d) 7,75(d) : 2H Ha et Hb
1,46(s) : 9H O-C(CH3)₃
7,37 à 7,53 (m) : 4H H₂, H₃, H₅ et H₆
7,85(dd) 7,89(dd) : 2H H₁ et H₄
8,12 (dl) : 1H H₇
9,18(sl) : 2H H mobiles
IR dans CHCl₃
NH : 3420, 3288cm⁻¹
C=O : 1703, 1690, 1635cm⁻¹
Hétérocycle + Aromatique: 1592, 1540, 1508cm⁻¹

### Exemple 10: 2-[(2S)-2-amino-4-méthyl-1-oxo-pentyl]-hydrazide de l'acide 5-(1-naphthalényl)-2-thiophènecarboxylique

A une solution de 275 mg de produit de l'exemple 9(-N-BOC) dans 4,5 ml de CH₂Cl₂, on ajoute 4,5 ml de CF₃CO₂H et on agite 3 heures à TA. On évapore à sec, reprend dans 10ml de CH₂Cl₂, lave avec 10ml de NH₄OH à 10%, sèche sur MgSO₄ et évapore à sec. On obtient ainsi 177 mg (81%) de produit attendu.
RMN (H,CDCl3)
0,97(d) 1,00(d): 6H les CH₃-CH-CH2-CH
1,80(m) : CH₃-CH-CH2-CH
1,50(m), 1,80(m) : CH₃-CH-CH2-CH
3,62(dd) : 1H CH₃-CH-CH2-CH
7,17(d), 7,74(d) : 2H Ha et Hb
7,35 à 7,53 (m) : 4H H₃, H₄, H₆ et H₇
7,86(m): 2H H₂ et H₅
8,13(d) : 1H H₈
IR dans CHCl₃
NH/NH2 (complexe) : 3365, 3221cm⁻¹
C=O : 1687, 1635cm⁻¹
Hétérocycle + Aromatique: 1592, 1540, 1508cm⁻¹

### Exemple 11: 2-[(2S)-2-[[[(1H-benzimidazol-2-yl)méthyl]-carbonyl]amino]-4-méthyl-1-oxo-pentyl]-hydrazide de l'acide 5-(1-naphthalényl)-2-thiophènecarboxylique

On procède dans les mêmes conditions opératoires que celles décrites au stade 4 de l'exemple 3 à partir de 68 mg (0,178mM 1 équivalent) d'amine thiophène obtenu à l'exemple 10, 47 mg (0,267mM 1,5éq) de l'acide carboxylique du 2-((1H-benzimidazol-2-yl)méthyl), 51 mg (0,267mM 1,5éq) de EDC et 36 mg (0,267mM 1,5éq) de HOBT. On obtient ainsi 118 mg de produit brut que l'on purifie sur silice avec pour éluant CH₂Cl₂ : 97,5 / MeOH : 2,5. On obtient ainsi 19 mg (20%) de produit attendu.
RMN (1H,DMSO)
0,87(d), 0,94(d) : 6H CH₃-CH-CH2-CH-NH
1,73(m) : 1H CH₃-CH-CH2-CH-NH
1,58(t) : 2H CH₃-CH-CH2-CH-NH
4,48(q) : 1H CH₃-CH-CH2-CH-NH
8,51(d) : 1H CH₃-CH-CH2-CH-NH
3,86(2xs) : 2H CH₂-CO
7,07(t) : 1H H₁₁
7, 32 (t) : 1H H₁₀
7,40(d), 7,94(d) : 2H Ha et Hb
7,46(d) : 1H H₉
7,56 à 7,66 : 4H H₃, H₄, H₆, H₇
8,03(m) : 2H H₂ et H₅
8,14 (m) : 1H H₈
10,21(sl), 10,51(s1) : 2H NH-NH
12,77 (sl) : 1H NH-C=N

### Exemple 12 : 2-[(2S)-4-méthyl-1-oxo-2-[[(phénylméthoxy)-carbonyl] amino]pentyl]-hydrazide de l'acide 5-(phénylméthyl) -2-thiophènecarboxylique

### Stade 1 : 5-(phénylméthyl)-2-thiophènecarboxylate d'ethyle produit G

5-(1,2-diphénylethyl)-2-thiophènecarboxylate d'ethyle produit H

On procède à une alkylation puis une estérification comme suit.

### 1) Alkylation

A une solution de 41 ml de LDA 2M dans le THF (82mM 2,1éq) et refroidie à -78°C, on ajoute 12 ml de tétraméthylène diamine (82mM ;2,1éq) et 5 g de thiophène acide dans 30 ml de THF (39mM, 1 équivalent) puis après 20 minutes, 8 g de Bromophényl (46,8mM, 1,2 équivalent) et on agite 2 heures en laissant remonter à TA. On ajoute 300 ml d'eau (à T<20°C), lave avec 100 ml d'Et₂0, ajoute de l'acide citrique jusqu'à pH≈4, extrait avec 100 ml d'Et₂0, lave avec 100 ml de NaCl, sèche sur MgSO₄ et évapore à sec. On obtient 6,87 g de produit brut.

### 2) Estérification.

On agite le produit brut 8 heures dans 68 ml d'EtOH et 13,7 ml d'H₂SO₄ au reflux à 120°C (T.ext). On évapore EtOH, extrait avec 100 ml d'Et₂0, lave avec 50 ml de Na₂CO₃, sèche sur MgSO₄ et évapore à sec. On obtient ainsi 6,76 g.de produit brut que l'on purifie sur silice avec pour éluant nHex : 99 / AcOEt : 1. On obtient ainsi 3,34 g de produit attendu sous forme d'un mélange de produit G (mono alkyle) : 2,5 g (26%) et produit H (dialkyle) : 0,385 g (3%)

### Stade 2 : Hydrazide de l'acide 5-(phénylméthyl)-2-thiophènecarboxylique

On procède dans les mêmes conditions que celles déjà décrites au stade 3 de l'exemple 3 à partir de 1,0 g (4,06Mm, 1 équivalent) de thiophène ester obtenu comme produit G au stade 1 ci-dessus, 2,5 ml (173mM 43éq) d' Hydrate d'hydrazine et 40 ml d'EtoH. On purifie sur silice avec pour éluant CH₂Cl_{2:}5 /AcOEt:5 puis CH₂Cl₂:9/MeOH:1NH4OH:0,1. On obtient ainsi 987 mg de produit attendu.

### Stade 3 : 2-[(2S)-4-méthyl-1-oxo-2-[[(phénylméthoxy)carbonyl] amino]pentyl]-hydrazide de l'acide 5-(phénylméthyl)-2-thiophènecarboxylique

On procède dans les mêmes conditions que celles déjà décrites au stade 4 de l'exemple 3, à partir de 400 mg (1,72Mm, 1 équivalent) de Cétohydrazide obtenu au stade 2 ci-dessus, 685 mg (2,58mM, 1,5 équivalent) de ZLeuOH, 495 mg (2,58mM 1,5éq) de EDC, 349 mg (2,58 mM 1,5éq) de HOBT et 15 ml de DMF. On purifie sur silice avec pour éluant Cyclohexane:6/ AcOEt:4. On obtient ainsi 779 mg de produit attendu(Rdt de 95%).
RMN (H, CDcl3)
0,90 (s1) 6H les CH₃-CH-CH₂-CH-NH
1, 66 (masq) CH₃-CH-CH₂-CH-NH
1, 57 (m), 1, 66 (masq) CH₃-CH-CH₂-CH-NH
4, 40 (m) CH₃-CH-CH₂-CH-NH
5,50(dl) CH₃-CH-CH₂-CH-NH
4,10(s)2H = C-CH₂-phényle
5,00, 5,10 AB 2H O-CH₂-phényle
6,71 (d), 7,46 (d) Ha et Hb
7,21(m), 7,29(m) 10H aromatiques
8,93(s1), 9,21(s1) 2H les H mobiles
IR CHCl₃
NH : 3420, 3286 cm-1
C=O : 1706, 1630 cm-1
Hétérocycle + Aromatique + Amide II : 1587, 1540, 1509, 1497 cm⁻¹.

### Exemple 13 : 2-[(2S)-4-méthyl-1-oxo-2-[[(phénylméthoxy)-carbonyl]amino] pentyl]-hydrazide de l'acide 5-[(diméthyl)-amino]-2-thiophènecarboxylique

Stade 1 : 5-(diméthylamino)-2-thiophènecarboxylate d'ethyle On agite à 165°C (T.ext) pendant une nuit 2,0 g d'ester bromé obtenu au stade 1 de l'exemple 3 (8,5 mM 1 équivalent) dans 60 ml de DMF et 60 ml de diméthylamine à 33% dans l'éthanol. On évapore les solvants, reprend dans CH₂Cl₂, lave avec ? ml de NaHCO₃ à 10%, sèche sur MgSO₄ et évapore à sec. On obtient ainsi 1,668 g de produit brut que l'on purifie sur silice avec pour éluant cyclohexane:8/AcOEt:2. On obtient ainsi 632 mg (37%) de produit attendu.

Stade 2 : Hydrazide de l'acide 5-(diméthylamino)-2-thiophènecarboxylique

On procède dans les mêmes conditions opératoires que celles décrites précédemment au stade 3 de l'exemple 3. On dissout 200 mg d'ester thiophène (1mM 1éq) obtenu au stade 1 ci-dessus dans 10 ml d'EtOH. On ajoute 1,5 ml d'hydrate d'hydrazine (30 mM 30 équivalents) et agite au reflux pendant 2 heures. On évapore à sec et on obtient ainsi 195 g du produit attendu.

Stade 3 : 2-[(2S)-4-méthyl-1-oxo-2-[[(phénylméthoxy)carbonyl] amino]pentyl]-hydrazide de l'acide 5-[(diméthyl)amino]-2-thiophènecarboxylique

On opère dans les mêmes conditions opératoires que celles décrites précédemment au stade 4 de l'exemple 3, à partir de 100 mg (0,540mM 1 équivalent) de Cétohydrazide obtenu au stade 2 ci-dessus, 186 mg (0,701mM 1,3éq) de ZleuOH, 135 mg (0,701 mM 1,3éq) de EDC, 95 mg (0,701 mM 1,3éq) et de HOBT et 5 ml de DMF. On obtient ainsi 671 mg de produit brut que l'on purifie sur silice avec pour éluant cyclohexane:5/AcOEt:5. On obtient ainsi 141 mg (61%) de produit attendu.
RMN (H, CDCl₃)
0,92(m) : 6H les (CH₃)₂-CH
1,53 à 1,78 : (CH₃)₂CH-CH₂
2,99(s) : 6H les NCH₃
4,40(m) : 1H CH-NH
5,50(d) : CH-NH
5,03, 5,13 : 2H AB OCH₂Ø
5,77(d) : 1H H₃
7,32(sl) : 5H de phényle
7,40(d) : 1H H₄
8,49(sl), 9,14(sl) : 2H mobiles
IR (H,CHCl₃)
NH : 3408, 3290 cm⁻¹
C=O : 1710, 1623 cm⁻¹
Hétérocycle + Aromatique+ Amide II : 1546, 1501 cm⁻¹

### Exemple 14: 2-[(2S)-1-oxo-3-phényl-2-[[(phénylméthoxy)-carbonyl] amino]propyl]-hydrazide de l'acide 5-((diméthyl)-amino] -2-thiophènecarboxylique

On procède dans les mêmes conditions opératoires que celles utilisés précédemment au stade 4 de l'exemple 3 à partir de 81 mg (0,437mM 1 équivalent)de cétohydrazide thiophène obtenu au stade 2 de l'exemple 13, 197 mg (0,656mM 1,5éq) de Z Phe OH, 126 mg (0,656mM 1,5éq) de EDC, 89 mg (0,656mM, 1,5éq) de HOBT et de 5 ml de DMF. On obtient ainsi 452 mg de produit brut que l'on purifie sur silice avec pour éluant cyclohexane:5/ACOEt:5. On obtient ainsi 130 mg (64%) de produit attendu.
RMN (H,CDCl₃)
2,88 (dd), 3,14(masq.) : phényle-CH₂-CH
3,00 (s) : 6H les CH₃-N-
4,44 (m) : 1H phényle -CH₂-CH-NH
4,99 (s) : 2H phényle -CH₂-O
5,94(d), 7,61 (d) : 2H les Hₐ et H_{b}
7,13 à 7,46(m) : 10H aromatiques
9,79(sl), 9,91(sl): H mobiles

### Exemple 15 : 2-[(2S)-4-méthyl-1-oxo-2-[[(phénylméthoxy)-carbonyl] amino]pentyl]-hydrazide de l'acide 5-[(phénylméthyl)amino]-2-thiophènecarboxylique

### Stade 1 : 5-nitro-2-thiophènecarboxylate d'ethyle

Une solution de 8,72 g d'acide 5-nitro-2-thiophène carboxylique(50,4mM 1 équivalent) dans 18 ml de SOCL₂ (252mM 5éq) est chauffée au reflux pendant 3 heures. On évapore SOCl₂ en reprenant avec 2 fois 50 ml de toluène. On reprend dans 35 ml d'EtOH et chauffe au reflux pendant 3 heures. On évapore à sec et lave avec 25 ml de NaHCO₃ à 10%, extrait avec 50 ml de AcOEt, sèche sur MgSO₄ et évapore à sec. On obtient ainsi 9,69 g (96%)de produit attendu.

### Stade 2 : 5-amino-2-Thiophènecarboxylate d'ethyle

On agite 3 heures à 75°C une suspension de 1,0 g (4,97mM 1 équivalent)du produit obtenu au stade 1 ci-dessus, 1,64 g de Fer en poudre (29,82mM 6 équivalents) dans 8 ml d'acide acétique glacial. On ajoute 10 ml d'eau, filtre, neutralise à pH≈8 avec Na₂CO₃, extrait avec 25 ml de AcOEt, sèche sur MgSO₄ et évapore à sec. On obtient ainsi 917 mg de produit brut que l'on purifie sur silice avec pour éluant CH₂Cl₂:9/AcOEt:1. On obtient ainsi 619 mg (73%) de produit attendu.

### Stade 3 : 5-[di(phénylméthyl)amino]-2-thiophènecarboxylate d'ethyle Produit E

### 5-[(phénylméthyl)amino]-2-thiophènecarboxylate d'ethyle Produit F

On agite pendant 36 heures à 60°C une solution de 470 mg d'amine thiophène obtenu (2,75mM 1 équivalent) obtenu au stade 2 ci-dessus, 2,6-lutidine (3,84 mM 1,4 équivalent) et BrCH₂Ø bromométhylphényle (3,57mM 1,3 équivalent) dans 10 ml de DMF. On ajoute 10 ml d'eau, extrait avec 25 ml d'Et₂0, sèche sur MgSO₄ et évapore à sec. On obtient ainsi 940 mg de produit brut que l'on purifie sur silice avec pour éluant cyclohexane:8/AcOEt:2. On obtient ainsi 182 mg (19%) de produit E et 269 mg(37%) de produit F.
RMN (H,CDCl₃) Produit E
1,31(t) : 3H CH₃-CH₂-O-CO
4,26(q) : 2H CH₃-CH₂-O-CO
4,56(sl) : 4H les phényle-CH₂-N
5,91(d), 7,50(d) : 2H les Hₐ et H_{b}
7,20 à 7,26(m) : 4H ; 7,27 à 7,38(m) 6H : H aromatiques
RMN (H,CDCl₃) Produit F
1,33(t) : 3H CH₃-CH₂-O-CO
4,27(q) : 2H CH₃-CH₂-O-CO
4,36(d) : 2H phényle-CH₂-NH
4,75(sl) : 1H phényle-CH₂-NH
5,97(d), 7,49 (d) : 2H Hₐ et H_{b}
7,36(m) : 5H aromatiques

### Stade 4 : hydrazide de l'acide 5-[(phénylméthyl)amino]-2-thiophènecarboxylique

On opère dans les mêmes conditions que celles déjà décrites au stade 3 de l'exemple 3 à partir de 150 mg (0,57 mM 1 équivalent)d'ester thiophène produit E obtenu au stade 3 ci-dessus, 1,1 g (23mM 40éq) d' hydrate d'hydrazine et 5 ml d'EtOH. On purifie sur silice avec pour éluant CH₂Cl₂:5/AcOEt:5 puis CH₂Cl₂:9/MeOH:1/NH₄OH:0,1). On obtient ainsi 119 mg (84%) de produit attendu.

### Stade 5 : 2-[(2S)-4-méthyl-1-oxo-2-[[(phénylméthoxy)carbonyl] amino]pentyl]-hydrazide de l'acide 5-[(phénylméthyl)amino]-2-thiophènecarboxylique

On opère dans les mêmes conditions que celles déjà décrites au stade 4 de l'exemple 3 à partir de 119 mg (0,481 mM 1 équivalent)de cétohydrazide obtenu au stade 4 ci-dessus, 191 mg (0,721mM 1,5éq) de ZleuOH, 138 mg (0,721mM 1,5éq) de EDC, 98 mg (0,721mM 1,5éq) de HOBT et 5 ml de DMF. On obtient ainsi 254 mg de produit brut que l'on purifie sur silice avec pour éluant (CH₂Cl₂:7/AcOEt:3). On obtient ainsi 162 mg (68%) de produit attendu.
RMN (H, CDCl₃)
0,91 (m) : 6H les (CH₃)₂-CH-CH2-CH-NH
1,69(masq.) : (CH₃)₂-CH-CH2-CH-NH
1,58(m) : (CH₃)₂-CH-CH2-CH-NH
4,38(m) : 1H (CH₃)₂-CH-CH2-CH-NH
5,48(dl) : 1H (CH₃)₂-CH-CH2-CH-NH
4,30(d) : 2H phényle-CH₂-NH
4,80(t) : 1H phényle-CH₂-NH
5,01, 5,11 : AB 2H phényle-CH₂-O
7,30 à 7,40(m) : 10H aromatiques
5, 90 (d), 7,33 (masq.) : Hₐ éth_{b}
8,53(dl) 9,10(dl) : 2H les H mobiles
IR CHCl₃
-NH : 3409, 3288 cm⁻¹
>=O : 1708, 1626 cm⁻¹
Système Conj.+ Aromatique + Amide II : 1585, 1485 cm⁻¹

### Exemple 16: 2-[(2S)-4-méthyl-1-oxo-2-[[(phénylméthoxy)-carbonyl] amino]pentyl]-hydrazide de l'acide 5-[bis(phénylméthyl)amino] -2-thiophènecarboxylique

### Stade 1 : hydrazide de l'acide 5-[di(phénylméthyl)amino]-2-thiophènecarboxylique

On procède comme au stade 4 de l'exemple 15 à partir de 180 mg (0,512mM 1 équivalent)d'ester thiophène produit F obtenu au stade 3 de l'exemple 15, 3 ml (60Mm 120 équivalents) de NH₂NH₂,H₂O et 16 ml d' EtOH. Le produit brut est purifié sur silice avec pour éluant (CH₂Cl₂:5/AcOEt:5 puis CH₂Cl₂:9/MeOH : 1/NH40H:0,1). On obtient ainsi 96 mg (56%) de produit attendu.

### Stade 2 : 2-[(2S)-4-méthyl-1-oxo-2-[[(phénylméthoxy)carbonyl] amino]pentyl]-hydrazide de l'acide 5-[bis(phénylméthyl) amino]-2-thiophènecarboxylique

On procède dans les mêmes conditions opératoires que celles déjà décrites au stade 4 de l'exemple 3 à partir de 96 mg (0,284mM léquivalent) de cétohydrazide obtenu au stade 1 ci-dessus, 113 mg (0,426mM 1,5 équivalent) de ZLeuOH, 82 mg (0,426mM 1,5éq) de EDC, 58 mg (0,426mM 1,5éq) de HOBT et 5ml de DMF. On obtient ainsi 205 mg de produit brut que l'on purifie sur silice avec pour éluant (CH2C12:9/AcOEt:1). On obtient ainsi 129 mg (78%) de produit attendu.
RMN (H,CDCl₃)
0,90(sl) : 6H (CH₃)₂-CH-CH2-CH-NH
1,69 (masqué) : 1H (CH₃)₂-CH-CH2-CH-NH
1,56(m) : 2H (CH₃)₂-CH-CH2-CH-NH
4,37(m) : 1H (CH₃)₂-CH-CH2-CH-NH
5,44(dl) : 1H (CH₃)₂-CH-CH2-CH-NH
4,52(sl) : 4H N-CH₂-Ø
5,02(d), 5,11(d) : 2H O-CH₂- Ø
5,85(d) : 1H H4
7,20(dd) : 4H Hₐ
7,26 à 7,35 : 11H H_{b}, H_{c}, H_{d}, Hₑ, H_{f}
7,32(masqué) : 1H H_{g}
8,36(sl) 9,05(sl) : 2H H mobiles

### Exemple 17 : 2-[(2S)-4-méthyl-1-oxo-2-[[(phénylméthoxy)-carbonyl] amino]pentyl]-hydrazide de l'acide 5-[[[3-(4-méthoxyphènoxy) phényl]méthyl](phénylméthyl)amino]-2-thiophènecarboxylique

### Stade 1 : 5-[[3-[(4-méthoxy)phénoxy]phényl] (phénylméthyl)amino]-2-thiophènecarboxylate d'ethyle

On procède à l'alkylation de l'amine benzylique comme suit. On agite une solution de 110 mg (0,420mM 1 équivalent)d'ester thiophène produit E obtenu au stade 3 de l'exemple 15, 160 mg (0,547mM 1,3éq)de bromure de 3-((4-méthoxyphénoxy) phényl)méthyl et 63 mg (0,586mM 1,4éq) de 2,6-lutidine dans 5 ml de DMF pendant 24 heures à 80°C. On ajoute 25 ml d'Et₂O, lave avec 25 ml d'eau, sèche sur MgSO₄ et évapore à sec. On obtient 333 mg de produit brut que l'on purifie sur silice avec pour éluant Cyclohexane:9/AcOEt:1. On obtient ainsi 108 mg (54%)de produit attendu.

### Stade 2 : Hydrazide de l'acide 5-[[3-[(4-méthoxy)phénoxy] phényl](phénylméthyl)amino]-2-thiophènecarboxylique

On procède dans les mêmes conditions opératoires que celles déjà décrites au stade 3 de l'exemple 3 à partir de 93 mg (0,196mM 1 équivalent)d'ester thiophène obtenu au stade 1 ci-dessus, 1,4 ml (28,8mM 147éq) d'hydrate d'hydrazine et 5 ml d'EtOH. On purifie sur colonne de silice avec pour éluant (CH₂Cl₂:5/AcOEt:5 puis CH₂Cl_{2:}9/MeOH:1/NH₄OH:0,1. On obtient ainsi 49 mg (54%) de produit attendu.

### Stade 3 : 2-[(2S)-4-méthyl-1-oxo-2-[[(phénylméthoxy)carbonyl] amino]pentyl]-hydrazide de l'acide 5-[[[3-(4-méthoxyphènoxy) phényl]méthyl](phénylméthyl)amino]-2-thiophènecarboxylique

On procède dans les mêmes conditions que celles déjà décrites au stade 4 de l'exemple 3 à partir de 48 mg (0,104mM 1 équivalent)de cétohydrazide obtenu au stade 2 ci-dessus, 42 mg (0,157mM 1,5 équivalent) de ZLeuoH, 30 mg (0,157mM 1,5éq) de EDC, 21 mg (0,157mM 1,5éq) de HOBT et 5 ml de DMF. On obtient ainsi 81 mg de produit brut que l'on purifie sur silice avec pour éluant CH₂Cl_{2:}5/AcOEt:5. On obtient ainsi 60 mg (81%) de produit attendu.
RMN (H,CDCl₃)
0,91(sl) : 6H (CH₃)₂-CH-CH₂-CH-NH
1,56(m) : 1H (CH₃)₂-CH-CH₂-CH-NH
1,69(m) : 2H (CH₃)₂-CH-CH₂-CH-NH
4,36(m) : 1H (CH₃)₂-CH-CH₂-CH-NH
5,36(dl) : 1H (CH₃)₂-CH-CH₂-CH-NH
3,80(s) : 3H CH₃-O-phényle
4,47(s), 4,51(s) : 4H les N-CH₂-phényle
5,04, 5,16 : AB 2H phényle-CH₂-O
5,85(d), 7,31 (masqué) : 2H Hₐ et H_{b}
6,82, 6,94 : AA BB' O-phényle-O-
6,76 à 6,96(m) : 3H, 7,15 à 7,34(m)11H : H aromatiques 8,24(s1), 8,91(s1) : 2H les NH

### Exemple 18: 2-[(2S)-4-méthyl-1-oxo-2-[[(Phénylméthoxy)-carbonyl]amino] pentyl]-hydrazide de l'acide 5-[(1,2-diphényl)éthyl]-2-thiophènecarboxylique

### Stade 1 : hydrazide de l'acide 5-(1,2-diphénylethyl)-2-thiophènecarboxylique

On procède comme au stade 2 de l'exemple 12 à partir de 315 mg (0,936mM 1 équivalent)d'ester thiophène, 2,4 g (48mM 52 équivalents) d'Hydrate d'hydrazine et 15 ml d'EtOH. On purifie sur silice avec pour éluant CH₂Cl_{2:}5/AcOEt:5 puis CH₂Cl₂:9/MeOH:1/NH₄OH:0,1). On obtient ainsi 135 mg (50%) de produit attendu.

### Stade 2 : 2-[(2S)-4-méthyl-1-oxo-2-[[(phénylméthoxy)carbonyl] amino]pentyl]-hydrazide de l'acide 5-[(1,2-diphényl)éthyl]-2-thiophènecarboxylique

On opère dans les mêmes conditions que celles déjà décrites au stade 4 de l'exemple 3 à partir de 120 mg (0,372mM 1 équivalent)de Cétohydrazide obtenu au stade 1 ci-dessus, 148 mg (0,558mM 1,5 équivalent) de ZLeuOH, 107 mg (0,558mM l,5éq) de EDC, 75 mg (0,558mM 1,5éq) de HOBT et 5 ml de DMF. On purifie sur colonne de silice avec pour éluant CH₂Cl₂:9/AcOEt:1. On obtient ainsi 161 mg (76%) de produit attendu.
RMN (H, CDCl3)
0,90 (CH₃)-CH-CH₂-CH-NH
1,39(m) (CH₃)-CH-CH₂-CH-NH
1,57(m) (CH₃)-CH-CH₂-CH-NH
4, 39 (masquée) (CH₃)-CH-CH₂-CH-NH
5,44 (CH₃)-CH-CH₂-CH-NH
3, 34 (m) Ø-CH₂-CH
4,40(t) Ø-CH₂-CH
5, 06 (AB) OCH₂ Ø
6,88(d), 7,40 les H du thiophène
7,10 à 7,35 H aromatiques
8,73(s), 9,22(s) CO-NH NH-CO

### Exemple 19 : 2-[(2S)-4-méthyl-1-oxo-2-[[(phénylméthoxy)-carbonyl]amino] pentyl]-hydrazide de l'acide 5-[1-[(phénylméthoxy) imino]éthyl]-2-thiophènecarboxylique

### Stade 1 : Acide 5-[1-(phénylméthoxyimino)ethyl]-2-thiophènecarboxylique

A une solution de 7 ml de pyridine contenant 0,5 g d'acide N31 est ajouté 0,94 g de benzylhydroxylamine. Le milieu est porté à 100° C pendant 20 heures. La solution est ensuite évaporée sous vide, reprise par 25 ml d'une solution HCl 2N et 50 ml d'acétate d'éthyle. Celle-ci est décantée, séchée sur MgS04, filtrée et concentrée. On récupère 0,430 g du produit attendu utilisé tel que pour la suite de la synthèse. Stade 2 : 2-[(2S)-4-méthyl-1-oxo-2-[[(phénylméthoxy)carbonyl] amino]pentyl]-hydrazide de l'acide 5-[1-[(phénylméthoxy) imino]éthyl]-2-thiophènecarboxylique

On procède dans les mêmes conditions opératoires que celles décrites au stade 4 de l'exemple 3 pour le couplage acide et ZLeu. On récupère ainsi 0,5 g du produit attendu purifié par chromatographie avec pour éluant une solution dichlorométhanol/méthanol (95/5).
RMN(1H,DMS0) :
1,49(s,9H) ; 2,19(s,3H) ; 2,27(s,3H) ; 5,18(s,2H) ; 5,30(s,2H) ; 6,65(sl,1H) ; 7,09(d,1H) ; 7,29 à 7,45(m,5H) ; 7,32(s1,1H) ; 7,50(d,1H).

### Exemple 20: 2-[[(9H-fluoren-9-yl)méthoxy]carbonyl]- hydrazide de l'acide 5-[1-[[(phénylméthoxy)carbonyl]amino]éthyl]-2-thiophènecarboxylique

### Stade 1 : 2-[[(9H-fluoren-9-yl)méthoxy]carbonyl] hydrazide de l'acide 5-(1-aminoethyl)-2-thiophènecarboxylique

On procède comme au stade 3 de l'exemple 1 à partir de ? g du produit de l'exemple 2. Après traitement du milieu réactionnel, on obtient 0,64 g de sel de l'amine. Celui-ci est engagé immédiatement dans l'étape suivante.

### Stade 2 : 2-[[(9H-fluoren-9-yl)méthoxy]carbonyl]- hydrazide de l'acide 5-[1-[[(phénylméthoxy) carbonyl]amino]éthyl]-2-thiophènecarboxylique

A une solution de 5 ml de THF contenant 0,64 g de sel trifluoroacétate de l'amine obtenu au stade 1 ci-dessus, on ajoute sous azote 0,22 g de ?. Le milieu réactionnel est porté à environ 0-5° C (bain glace-sel). On y ajoute alors goutte à goutte 0,22 g de chloroformiate de benzyle. Après ½ heure à cette température, on revient à T.A. pendant 4 heures. On évapore sous vide, reprend par 25 ml de CH₂Cl₂ et lave par 15 ml d'une solution HCl 1N. La solution de CH₂Cl₂ est décantée, concentrée. Le résidu obtenu est purifié par chromatographie en éluant par une solution dichlorométhane/méthanol (96/4). On obtient ainsi 0,40 g du produit attendu.
RMN(1H,CDCl₃) :
1,56(d,3H) ; 4,26(t,1H) ; 4,47(d,2H) ; 5,13(m,2H) ; 6,91(m,2H) ; 7,34(m,9H) ; 7,59(d,2H) ; 7,75 (d, 2H) ; 8,17(s1,1H).

### Exemple 21: 2-[(2S)-4-méthyl-1-oxo-2-[[(phénylméthoxy)-carbonyl]amino] pentyl]-hydrazide de l'acide 5-[1-[[(phénylméthoxy)carbonyl] amino]éthyl]-2-thiophènecarboxylique

### Stade 1 : hydrazide de l'acide 5-[1-[[(phénylméthoxy) carbonyl]amino]ethyl]-2-thiophènecarboxylique

On procède à une déprotection du carbonate fmoc comme suit. A une solution de 10 ml d'acétonitrile à température ambiante contenant 0,35 g du dérivé thiophène obtenu à l'exemple 20, on ajoute 2 ml de pipéridine. Le milieu est conservé 2 heures sous agitation à température ambiante puis concentré sous vide. Le résidu est purifié par chromatographie en éluant avec une solution CH₂Cl₂/MeOH (95/5). On obtient ainsi 0,150 g du produit attendu.

### Stade 2 : 2-[(2S)-4-méthyl-1-oxo-2-[[(phénylméthoxy)carbonyl] amino]pentyl]-hydrazide de l'acide 5-[1-[[(phénylméthoxy) carbonyl]amino]éthyl]-2-thiophènecarboxylique

On procède à un couplage acide-amine, dans les conditions opératoires décrites au stade 4 de l'exemple 3. On récupère ainsi 0,050 g du produit attendu purifié par chromatographie en éluant par une solution dichlorométhane /MeOH (96/4).

### Exemple 22: 2-[(2S)-4-méthyl-1-oxo-2-[[(phénylméthoxy)-carbonyl]amino] pentyl]- hydrazide de l'acide 5-[3-méthyl-1-[(phénylméthoxy) carbonyl]amino]butyl]-2-thiophènecarboxylique

Stade 1 : 5-(1-hydroxy-3-méthylbutyl)-2-thiophènecarboxylate de méthyle

A une solution de 22,5 ml de LDA (2M dans THF) (44,87 nM2,3éq) et refroidie à -78°C, on ajoute 2,5 g (19,5Mm, 1éq) d'acide 2-thiophènecarboxylique en solution dans 20 ml de THF, ajoute 1,84 g d'isovaléraldéthyde (21,46mM,1éq) et agite 2 heures à TA. On ajoute H₂0 (150ml), lave avec 50 ml de Et₂0, ajoute de l'acide citrique jusqu'à pH≈4, extrait avec 50 ml de CH₂Cl₂ et sèche sur MgSO4. On obtient ainsi 3,37 g de produit brut, que l'on reprend dans 50 ml de CH₂Cl₂. On ajoute 60 ml de CH₂N₂/CH₂Cl₂ (12 g/1) (1,65 équivalent), agite à TA pendant 3 heures et évapore à sec. On obtient ainsi 1,67 g de produit brut que l'on purifie sur silice avec pour éluant nHex:9/AcOEt:1. On obtient ainsi 855 mg (37%) de produit attendu.

Stade 2 : 5-(3-méthyl-1-oxo-butyl)-2-thiophènecarboxylate de méthyle

On agite au reflux pendant 5 heures une suspension de 787 mg de thiophène ester obtenu au stade 1 ci-dessus, 2,36 g de Mn02 (3 parties en poids) et 15 ml de CH2C12. On filtre et évapore à sec. On obtient ainsi 722 mg de produit brut.

Stade 3 : 5-(3-méthyl-1-hydroxyimino-butyl)-2-thiophéne carboxylate de méthyle

A une solution de 520 mg de thiophène (2,29 mM 1 équivalent) obtenu au stade 2 ci-dessus dans 5 ml de pyridine, on ajoute 319 mg de chlorhydrate d'hydroxylamine (4,59mM 2éq) et agite 2 heures au reflux. On évapore la pyridine, ajoute 50 ml de HCl 2N, extrait avec 50 ml de AcOEt, sèche sur MgS04 et évapore à sec. On obtient ainsi 554 mg de produit attendu.

Stade 4 : 5-(1-amino-3-méthyl-butyl)-2-thiophènecarboxylate de méthyle

On agite 1 heure à TA une suspension de 550 mg de thiophène hydroxyamine (2,28mM 1 équivalent) obtenu au stade 3 ci-dessus, 1,49 g de Zinc en poudre (22,8mM 10 équivalents) dans 10 ml de CF3CO2H. On évapore CF3CO2H, reprend dans 25 ml de MeOH, filtre et évapore à sec. On reprend dans 50 ml de CH₂Cl₂, extrait avec 25 ml de HCl 1N, amène à pH≈8 avec NaHCO₃ à 10%, extrait avec 50 ml de AcOEt, sèche sur MgSO₄ et évapore à sec. On obtient ainsi 316 mg (61%) de produit attendu.

Stade 5 : 5-[1-[[(phénylméthoxy)carbonyl]amino]-3-méthylbutyl]-2-thiophènecarboxylate de méthyle

A une solution de 315 mg de l'amine thiophène (1,39mMl 1éq) obtenu au stade 4 ci-dessus dans 10 ml de THF et refroidie à O°C, on ajoute 238 mg de TEA (2,365mM 1,7 équivalent) et 403 mg de ClCO₂CH₂Ø (2,365mM 1,7 éq). On évapore le THF, reprend dans 50 ml de CH₂Cl₂, lave avec 25 ml de HCL 2N, sèche sur MgSO₄ et évapore à sec. On obtient ainsi 548 mg de produit brut que l'on purifie sur silice avec pour éluant (nHexane: 9/AcOEt: 1). On obtient ainsi 312 mg (62%) de produit attendu.

Stade 6 : Acide 5-[1-[[(phénylméthoxy)carbonyl]amino]-3-méthyl-butyl]-2-thiophènecarboxylique

On agite 1 nuit à 40°C une solution de 277 mg de thiophène ester (0,766mM 1 équivalent) obtenu au stade 5 ci-dessus, 48 mg de LiOH (1,148mM 1,5éq) dans 6 ml de THF et 3 ml d'H₂O.

On évapore THF, lave avec 25 ml de CH₂Cl₂, extrait avec 10 ml de NaHCO₃ amène à pH≈1 avec HCl 2N, extrait 3 fois avec 25 ml de AcOEt, lave avec 25 ml d'eau, sèche sur MgS0₄ et évapore à sec. On obtient ainsi 280 mg de produit attendu.

Stade 7 : 2-[(2S)-4-méthyl-1-oxo-2-[[(phénylméthoxy)carbonyl] amino]pentyl]- hydrazide de l'acide 5-[3-méthyl-1-[(phénylméthoxy)carbonyl]amino]butyl]-2-thiophènecarboxylique On opère dans les mêmes conditions que celles déjà décrites au stade 4 de l'exemple 3 à partir de 270 mg de thiophène acide obtenu au stade 6 ci-dessous (0,778mM 1 équivalent), 310 mg (1,1 mM 1,42éq) de Cétohydrazine, 164 mg (0,855mM 1,1éq) de EDC, 115 mg (0,855mM 1,1éq)de HOBT et 10 ml de DMF. On obtient ainsi 360 mg de produit brut que l'on purifie sur silice avec pour éluant CH₂Cl₂:95/MeOH:5. On obtient ainsi 310 mg (67%) de produit attendu sous forme d'un mélange de diastéréoisomères.
RMN (1H,DMSO)
0 , 89 (m) : 12H (CH₃)₂-CH-CH₂
1, 71 (m) : 2H (CH₂)₂-CH-CH₂
1, 54 (m) : 4H (CH₂)₂-CH-CH₂
4,16(ddd): 1H CO-CH-NH
7,53(dl): 1H CO-CH-NH
4,83(ddd): 1H C=C-CH-NH-
7,48(d1): 1H C=C-CH-CH
5,03(s1) : 4H O-CH₂-Ø
7,00(d) et 7,66(d): 2H Ha et Hb
7,27 à 7,39(m): 10H aromatiques
10,05(sl) et 10,32(sl): 2H CO-NH-NH-CO

### Exemple 23 : 2-[(2S)-4-méthyl-1-oxo-2-[[(phénylméthoxy)-carbonyl]amino] pentyl]-hydrazide de l'acide 5-[3-méthyl-1-[[(phénylméthoxy) carbonyl]amino]butyl]-2-thiophènecarboxylique isomère A.

### Exemple 24: [(2S)-4-méthyl-1-oxo-2-[[(phénylméthoxy)-carbonyl]amino] pentyl]-hydrazide de l'acide 5-[3-méthyl-1-[[(phénylméthoxy) carbonyl]amino]butyl]-2-thiophènecarboxylique Isomere B.

Après purification et séparation de 50 mg du produit de l'exemple 22 sur colonne chirale, on obtient l'isomère A qui constitue l'exemple 23 et l'isomère B qui constitue l'exemple 24.
RMN ex 23 isomère A
0,89 (m) 12H : les (CH3)2-CH-CH2
1,43 à 1,78 (m) 6H: les (CH3)2-CH-CH2
4,15 (ddd) 1H: CO-CH-NH
7,52 (dl) 1H: CO-CH-NH
4,83 (ddd) 1H: =C-CH-NH
7,96 (d1) 1H: =C-CH-NH
5,03 (s1) 4H: les O-CH2-phényl
7,34 (m) 10H: les O-CH2-phényl
6,98 (d), 7,63 (d) 2H: Ha et Hb
10,07 (s1) 2H: NH-NH
RMN ex 24 isomère B
0,89 (m) 12H: les (CH3)2-CH-CH2
1,46 à 1,78 (m) 6H: les (CH3)2-CH-CH2
4,13 (ddd) 1H: CO-CH-NH
7,53 (dl) 1H: CO-CH-NH
4,82 (ddd) 1H : =C-CH-NH
7,93 (dl) 1H: =C-CH-NH
5,04 (s1) 4H: les O-CH2-phényl
7,35 (m) 10H: les O-CH2-phényl
6,96 (d), 7,56 (s1) 2H: Ha et Hb
10,02 (s1), 10,37 (s1) 2H: NH-NH

### Exemple 25: COMPOSITION PHARMACEUTTQUE :

On a préparé des comprimés répondant à la formule suivante :

| | |
|---|---|
| Produit de l'exemple 1 | 0,2 g |
| Excipient pour un comprimé terminé à | 1 g |

| | |
|---|---|
| (détail de l'excipient : lactose, talc, amidon, stéarate de magnésium). | |

## Revendications

1. Produits de formule (I): 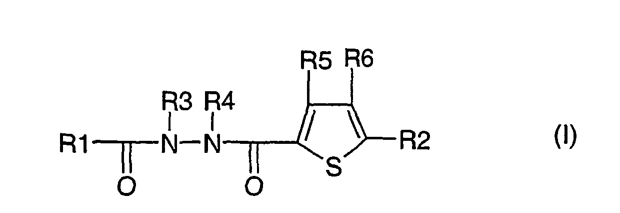 dans laquelle:
R1 représente un radical alkyle ou alcoxy linéaires ou ramifiés renfermant au plus 8 atomes de carbone substitués soit par un radical monocyclique ou constitué de cycles condensés, carbocyclique ou hétérocyclique saturé ou insaturé renfermant au plus 14 chaînons et contenant un ou plusieurs hétéroatomes identiques ou différents, O, N, NH ou S, un tel radical monocyclique ou constitué de cycles condensés étant lui-même éventuellement substitué comme indiqué ci-après, soit par un radical amino lui-même éventuellement substitué par un radical -C(O)-O- R7 ou -C(O)-R7
R2 représente :
a) un radical alkyle linéaire ou ramifié renfermant au plus 8 atomes de carbone éventuellement substitué par un ou plusieurs radicaux choisis parmi les radicaux -NH-C(O)-O-R7, les radicaux -NH-C(O)-alk-NH-C(O)-O-R7 et les radicaux monocycliques ou constitués de cycles condensés, carbocycliques ou hétérocycliques saturés ou insaturés renfermant au plus 14 chaînons et contenant un ou plusieurs hétéroatomes identiques ou différents, O, N, NH ou S, de tels radicaux monocycliques ou constitués de cycles condensés étant eux-mêmes éventuellement substitués come indiqué ci-après,
b) un radical phényle éventuellement substitué par un radical alcoxy linéaire ou ramifié renfermant au plus 6 atomes de carbone ou un radical arylalcoxy, ces radicaux alcoxy et arylalcoxy étant eux-mêmes éventuellement substitués come indiqué ci-après,
c) un radical naphtyle éventuellement substitué come indiqué ci-après,
d) un radical amino éventuellement substitué par un ou deux radicaux identiques ou différents choisis parmi les radicaux alkyles linéaires ou ramifiés renfermant au plus 6 atomes de carbone, les radicaux -C(O)-alk-NH=C(O)-O-R7 et les radicaux arylalkyle eux-mêmes éventuellement substitués par un radical alcoxyphénoxy dans lequel le radical alcoxy linéaire ou ramifié renferme au plus 6 atomes de carbone, ces radicaux alkyle, arylalkyle, alcoxy, et alcoxyphénoxy étant eux-mêmes éventuellement substitués come indiqué ci-après,
e) -(CH3)C=N-O-R8
R3 et R4, identiques ou différents, représentent un atome d'hydrogène, un radical alkyle, arylalkyle ou aryle, dans lesquels les radicaux alkyle sont linéaires ou ramifiés renfermant au plus 6 atomes de carbone et les radicaux aryle renferment au plus 10 atomes de carbone, ces radicaux alkyle, arylalkyle et aryle éventuellement substitués par un atome d'halogène, un radical hydroxyle ou un radical alkyle ou alcoxy linéaires ou ramifiés renfermant au plus 4 atomes de carbone,
R5 et R6, identiques ou différents, sont choisis parmi l'atome d'hydrogène, le radical hydroxyle et les radicaux alkyle et alcoxy linéaires ou ramifiés renfermant au plus 6 atomes de carbone, ces radicaux alkyle et alcoxy étant eux-mêmes éventuellement substitués comme indiqué ci-après
R7 représente un radical alkyle linéaire ou ramifié renfermant au plus 6 atomes de carbone éventuellement substitué par un radical aryle ou hétéroaryle (indazolyle), éventuellement substitués,
R8 représente un atome d'hydrogène ou un radical alkyle linéaire ou ramifié renfermant au plus 6 atomes de carbone éventuellement substitué par un radical aryle (phényle), étant entendu que tous les radicaux alkyle, alcoxy, monocyclique ou constitué de cycles condensés, aryle, hétéroaryle, arylalkyle, arylalcoxy et alcoxyphénoxy indiqués ci-dessus comme étant éventuellement subtitués, sont éventuellement substitués par un ou plusieurs radicaux choisis parmi les atomes d'halogène ; les radicaux hydroxyle; alkyle, alkényle, alkylthio ou alcoxy renfermant au plus 6 atomes de carbone; cycloalkyle renfermant au plus 6 chaînons; acyle renfermant au plus 7 atomes de carbone ; cyano ; nitro ; carboxy libre, salifié ou estérifié ; tétrazolyle; indazolyle ; -NH2, -NH(alk), -N(alk)(alk) ; -SO2-NH-CO-NHR5 dans lequel R5 représente alk ou phényle; -C(O)-NH2 , -C(O)-NH(alk), -C(O)-N(alk)(alk), -NH-C(O)-(alk), -N(alk)-C(O)-(alk); et phényle lui même éventuellement substitué par un radical alkyle ou alcoxy renfermant au plus 4 atomes de carbone,
étant entendu que alk représente un radical alkyle linéaire ou ramifié renfermant au plus 4 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

2. Produits de formule (I) telle que définie à la revendication 1 répondant à la formule (Ia) : 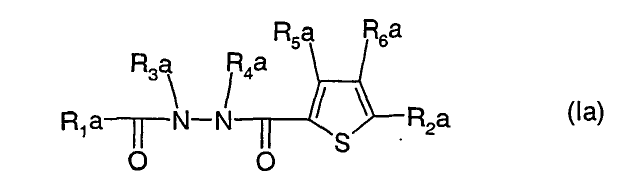 dans laquelle:
R1a représente un radical alkyle ou alcoxy linéaires ou ramifiés renfermant au plus 8 atomes de carbone substitués par un radical phényle, un radical constitué de cycles condensés carbocyclique saturé ou insaturé renfermant au plus 14 chaînons et un radical amino lui-même éventuellement substitué par un radical -C(O)-O- R7a ou -C(O)-R7a, ce radical phényle et ces radicaux constitués de cycles condensés carbocyclique saturé ou insaturé étant éventuellement substitués comme indiqué ci-après,
R2a représente :
a) un radical alkyle linéaire ou ramifié renfermant au plus 6 atomes de carbone éventuellement substitué par un ou plusieurs radicaux choisis parmi les radicaux NH-C(O)-O-R7a et -NH-C(O)-alk-NH-C(O)-O-R7, le radical phényle et les radicaux hétérocycliques saturés ou insaturés renfermant au plus 10 chaînons,
b) un radical phényle éventuellement substitué par un radical alcoxy linéaire ou ramifié renfermant au plus 6 atomes de carbone ou un radical benzyloxy,
c) un radical naphtyle,
d) un radical amino éventuellement substitué par un ou deux radicaux identiques ou différents choisis parmi les radicaux alkyles linéaires ou ramifiés renfermant au plus 4 atomes de carbone, les radicaux -C(O)-alk-NH-C(O)-O-R7a et le radical benzyle lui-même éventuellement substitué par un radical alcoxyphénoxy dans lequel le radical alcoxy renferme au plus 4 atomes de carbone,
e) -(CH3)C=N-O-R8
R3a et R4a, identiques ou différents, représentent un atome d'hydrogène, un radical alkyle linéaire ou ramifié renfermant au plus 6 atomes de carbone ou un radical phényle éventuellement substitué par un radical hydroxyle ou alcoxy renfermant au plus 4 atomes de carbone
R5a et R6a, identiques ou différents, sont choisis parmi l'atome d'hydrogène, le radical hydroxyle et les radicaux alkyle ou alcoxy linéaires ou ramifiés renfermant au plus 6 atomes de carbone éventuellement substitués par un radical phényle ou indazolyle,
R7a représente un radical alkyle linéaire ou ramifié renfermant au plus 4 atomes de carbone éventuellement substitué par un radical phényle ou un radical indazolyle, R8a représente un atome d'hydrogène ou un radical alkyle linéaire ou ramifié renfermant au plus 4 atomes de carbone éventuellement substitué par un radical phényle,
étant entendu que alk représente un radical alkyle linéaire ou ramifié renfermant au plus 4 atomes de carbone,
lesdits produits de formule (Ia) étant sous toutes les formes isomères possibles racémiques, énantiomères et diastéréoisomères, ainsi que les sels d'addition avec les acides minéraux et organiques ou avec les bases minérales et organiques desdits produits de formule (Ia).

3. Produits de formule (I) telle que définie aux revendications 1 et 2 répondant à la formule (Ib) : 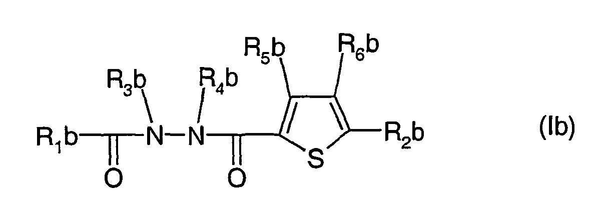 dans laquelle:
R1b représente un radical alkyle ou alcoxy linéaires ou ramifiés renfermant au plus 6 atomes de carbone substitué par un radical choisi parmi les radicaux phényle, fluoren (ex 1 et 20) et amino lui-même éventuellement substitué par un radical -C(O)-O-R7b ou -C(O)-R7b,
R2b représente :
b) un radical alkyle linéaire ou ramifié renfermant au plus 6 atomes de carbone éventuellement substitué par un ou plusieurs radicaux choisis parmi les radicaux -NH-C(O)-O-R7b et -NH-C(O)-alk-NH-C(O)-O-R7b et les radicaux phényle, pyridyle, pyridinyle, pipéridinyle, benzofurannyle, morpholinyle, quinolinyle, indolyle, benzimidazolyle et indazolyle,
b) un radical phényle éventuellement substitué par un radical alcoxy linéaire ou ramifié renfermant au plus 4 atomes de carbone ou un radical benzyloxy,
c) un radical naphtyle,
d) un radical amino éventuellement substitué par un ou deux radicaux identiques ou différents choisis parmi les radicaux alkyles linéaires ou ramifiés renfermant au plus 4 atomes de carbone, les radicaux -C(O)-alk-NH-C(O)-O-benzyle et le radical benzyle lui-même éventuellement substitué par un radical alcoxyphénoxy dans lequel le radical alcoxy renferme au plus 4 atomes de carbone,
e) -(CH3)C=N-O-R8
R3b et R4b représentent un atome d'hydrogène,
R5b et R6b, identiques ou différents, représentent un atome d'hydrogène, un radical hydroxyle, un radical alkyle ou alcoxy linéaires ou ramifiés renfermant au plus 4 atomes de carbone éventuellement substitué par un radical phényle,
R7b représente un radical alkyle linéaire ou ramifié renfermant au plus 4 atomes de carbone éventuellement substitué par un radical phényle ou un radical indazolyle, R8b représente un atome d'hydrogène ou le radical benzyle, étant entendu que alk représente un radical alkyle linéaire ou ramifié renfermant au plus 4 atomes de carbone,
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

4. Produits de formules (I), (Ia) et (Ib) telles que définies aux revendications 1 à 3 dans lesquelles R3 ,R3a, R4, R4a, R5, R5a, R5b, R6, R6a et R6b représentent un atome d'hydrogène,
lesdits produits de formules (I), (Ia) et (Ib) étant sous toutes les formes isomères possibles racémiques, énantiomères et diastéréoisomères, ainsi que les sels d'addition avec les acides minéraux et organiques ou avec les bases minérales et organiques desdits produits de formules (I), (Ia) et (Ib).

5. Produits de formule (I) suivants :
- le 2-[(2S)-4-méthyl-1-oxo-2-[[(phénylméthoxy)carbonyl] amino]pentyl]-hydrazide de l'acide 5-[(2S)-1-[[4-méthyl-1-oxo
- 2-[[(phénylméthoxy)carbonyl]amino]pentyl]amino]éthyl]-2-thiophènecarboxylique
- le 2-[[(9H-fluoren-9-yl)méthoxy]carbonyl]-hydrazide de l'acide 5-[1-(hydroxyimino)éthyl]-2-thiophèneCarboxylique
- le 2-[(2S)-4-méthyl-1-oxo-2-[[(phénylméthoxy)carbonyl] amino]pentyl]-hydrazide de l'acide 5-[2-(phénylméthoxy) phényl]-2-thiophènecarboxylique
- le 2-[(2S)-4-méthyl-1-oxo-2-[[(phénylméthoxy)carbonyl] amino]pentyl]-hydrazide de l'acide 5-[[[3-(4-méthoxyphènoxy) phényl]méthyl] (phénylméthyl)amino]-2-thiophènecarboxylique
- le 2-[(2S)-4-méthyl-1-oxo-2-[[(phénylméthoxy)carbonyl]-amino] pentyl]-hydrazide de l'acide 5-[1-[(phénylméthoxy) imino]éthyl]-2-thiophènecarboxylique
- le 2-[[(9H-fluoren-9-yl)méthoxy]carbonyl]- hydrazide de l'acide 5-[1-[[(phénylméthoxy)carbonyl]amino]éthyl]-2-thiophènecarboxylique
- le 2-[(2S)-4-méthyl-1-oxo-2-[[(phénylméthoxy)carbonyl]-amino] pentyl]-hydrazide de l'acide 5-[1-[[(phénylméthoxy)-carbonyl] amino]éthyl]-2-thiophènecarboxylique
- le 2-[(2S)-4-méthyl-1-oxo-2-[[(phénylméthoxy)carbonyl]-amino] pentyl]- hydrazide de l'acide 5-[3-méthyl-1-[(phénylméthoxy) carbonyl]amino]butyl]-2-thiophènecarboxylique
- le 2-[(2S)-4-méthyl-1-oxo-2-[[(phénylméthoxy)carbonyl]-amino] pentyl]-hydrazide de l'acide 5-[3-méthyl-1-[[(phénylméthoxy) carbonyl]amino]butyl]-2-thiophènecarboxylique isomère A
- le [(2S)-4-méthyl-1-oxo-2-[[(phénylméthoxy)carbonyl]amino] pentyl]-hydrazide de l'acide 5-[3-méthyl-1-[[(phénylméthoxy) carbonyl]amino]butyl]-2-thiophènecarboxylique Isomere B ainsi que les sels d'addition avec les acides minéraux et organiques ou avec les bases minérales et organiques desdits produits de formule (I).

6. Procédé de préparation des produits de formule (I), telle que définie à la revendication 1, **caractérisé en ce que** l'on part d'un composé de formule (II): 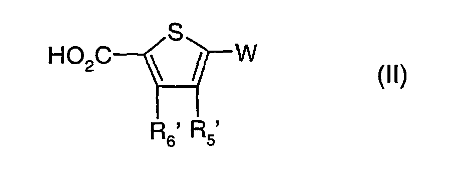 dans lequel W représente un atome d'hydrogène, un atome d'halogène ou un radical nitro, R5' et R6' ont les significations indiquées à la revendication 1 respectivement pour R5 et R6, dans lesquelles les éventuelles fonctions réactives sont éventuellement protégées par des groupements protecteurs,
produit de formule (II) que selon la valeur de W l'on peut soumettre aux réactions suivantes :
a) lorsque W représente un atome d'hydrogène, l'on peut soumettre le produit de formule (II) aux réactions des voies i) ou ii) :
voie i) on peut soumettre le composé de formule (II) dans lequel W représente un atome d'hydrogène à l'action d'un dérivé bromé de formule (III) :
R2'-Br (III)
dans laquelle R2' a la signification indiquée à la revendication 1 pour R2 dans laquelle les éventuelles fonctions réactives sont éventuellement protégées par des groupements protecteurs,
pour obtenir un produit de formule (IV) : 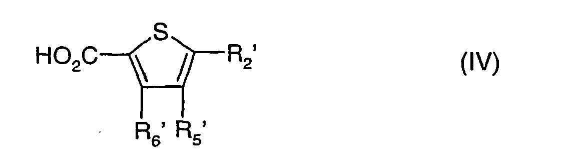 dans laquelle R2', R5' et R6' ont les significations indiquées ci-dessus,
que lorsque R2' représente un radical -CH2- phényle, pour donner le produit de formule (IV1) : 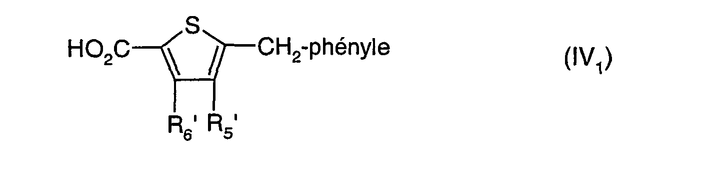 dans laquelle R5' et R6' ont les significations indiquées ci-dessus, l'on peut soumettre à une seconde réaction avec le composé de formule (III) pour obtenir le composé de formule (IV2) : 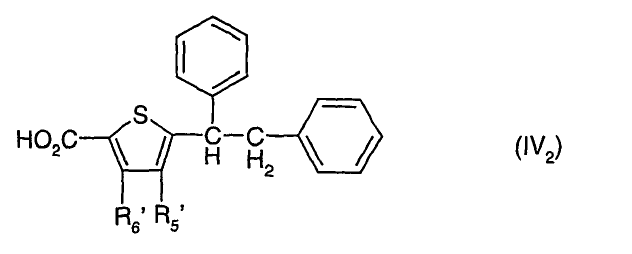 dans laquelle R5' et R6' ont les significations indiquées ci-dessus,
produits de formules (IV), (IV1) et (IV2) que l'on peut soumettre à une réaction d'estérification pour obtenir les produits correspondants de formules (V), (V1) et (V2) : 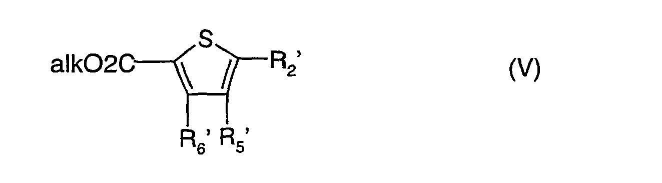 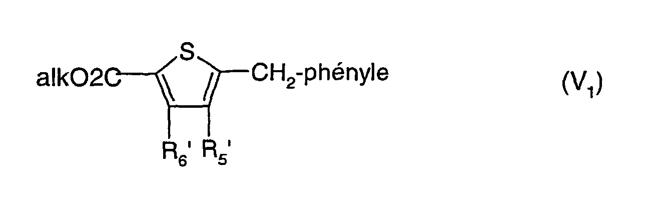 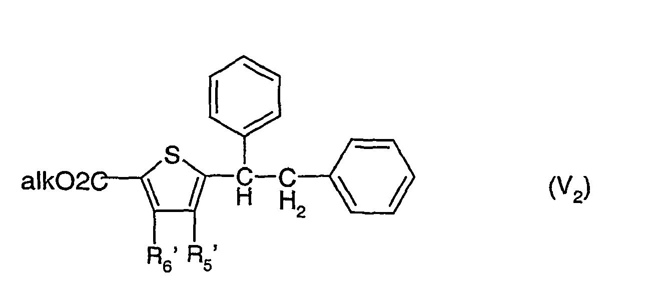 dans laquelle R2', R5' et R6' ont les significations indiquées ci-dessus et alk représente un radical alkyle linéaire ou ramifié renfermant au plus 4 atomes de carbone, voie ii) on peut soumettre le composé de formule (II) dans lequel W représente un atome d'hydrogène à une réaction d'estérification avec un alcool de formule (VI) :
Rc-OH (VI)
dans laquelle Rc représente un radical alkyle linéaire ou ramifié renfermant au plus 6 atomes de carbone,
pour obtenir des produits de formule (VII): 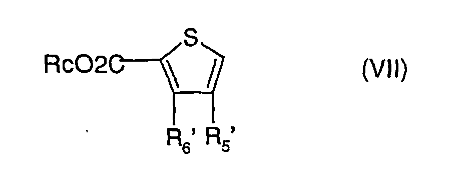 dans laquelle Rc, R5' et R6' ont les significations indiquées ci-dessus,
que l'on soumet à une aldéhyde de formule (VIII) :
Rd-CHO (VIII)
dans laquelle Rd représente un atome d'hydrogène, un radical alkyle tel que défini ci-dessus ou un radical monocyclique ou constitué de cycles condensés, carbocyclique ou hétérocyclique saturé ou insaturé renfermant au plus 14 chaînons et contenant un ou plusieurs hétéroatomes identiques ou différents, O, N, NH ou S, eux-mêmes éventuellement substitués,
pour obtenir des produits de formule (IX) : 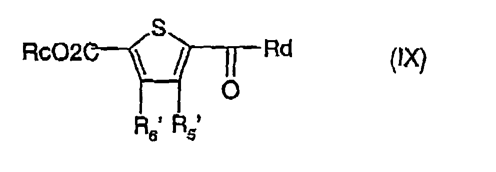 dans laquelle Rc, Rd, R5' et R6' ont les significations indiquées ci-dessus,
produits de formule (IX) que l'on fait réagir avec une amine de formule (X):
H2N-CH2-ORe (X)
dans laquelle Re représente un radical arylalkyle éventuellement substitué,
pour obtenir des produits de formule (XI) : 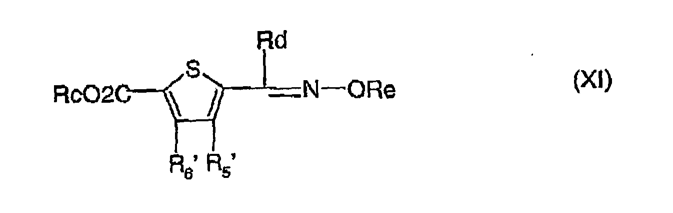 dans laquelle Rc, Rd, Re, R5' et R6' ont les significations indiquées ci-dessus,
produit de formule (XI) que l'on peut soumettre à une réaction de réduction sélective,
pour obtenir des produits de formule (XII) : 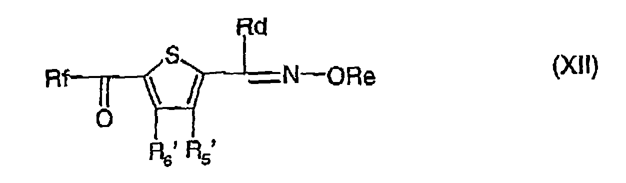 dans laquelle Rd, Re, R5' et R6' ont les significations indiquées ci-dessus,
et Rf représente un groupement de réduction sélective de ORc. produits de formules (XI) et (XII) que l'on peut soumettre à une réaction de réduction pour obtenir les produits correspondants de formule (XIII): 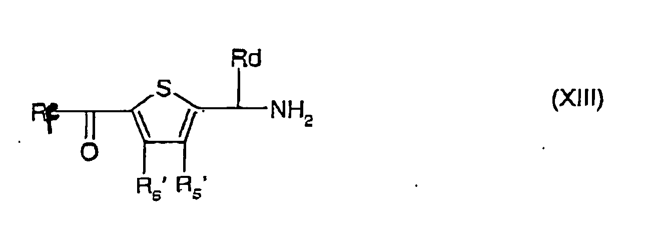 dans laquelle Rd, Rf, R5' et R6' ont les significations indiquées ci-dessus,
produits de formule (XIII) que l'on peut soumettre à une réaction.de saponification pour obtenir les produits correspondants de formule (XIV): 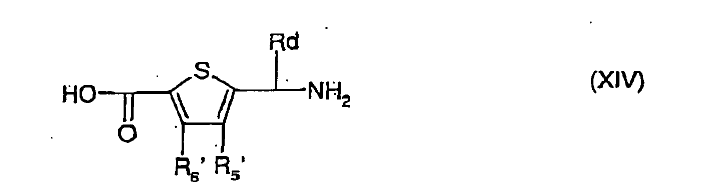 dans laquelle Rd, R5' et R6' ont les significations indiquées ci-dessus,
produit de formule (XIV) que l'on peut faire réagir avec un produit de formule (XV) : 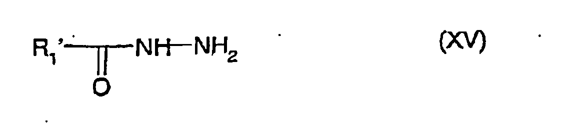 dans laquelle R1' a la signification indiquée à la revendication 1 pour R1 dans laquelle les éventuelles fonctions réactives sont éventuellement protégées par des groupements protecteurs,
pour obtenir des produits de formule (I'x): 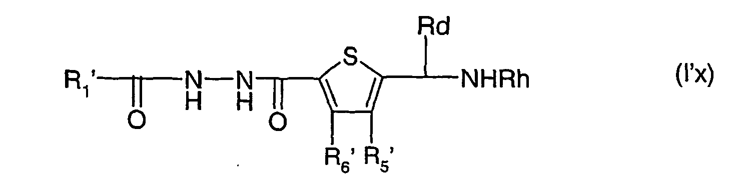 dans laquelle R1', Rd, R5' et R6' ont les significations indiquées ci-dessus et Rh représente -C(O)-O-R7 ou -C(O)-alk-NH-C(O)-O-R7 avec R7 tel que défini ci-dessus,
b) lorsque W représente un atome d'halogène, l'on peut soumettre le produit de formule (II) à une réaction d'estérification avec un alcool de formule (VI) telle que définie ci-dessus pour obtenir des produits de formule (XVI): 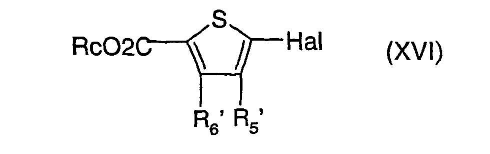 dans laquelle Rc, R5' et R6' ont les significations indiquées ci-dessus et Hal représente un atome d'halogène, produit de formule (XVI) que l'on peut soumettre :
soit à l'action d'un dérivé boré de formule (XVII) :
R2'-B(OH)2 (XVII)
dans laquelle R2' a la signification indiquée ci-dessus, pour obtenir un produit de formule (XVIII) : 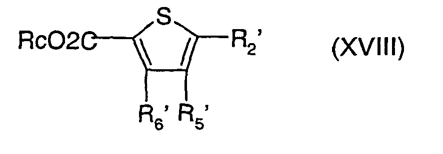 dans laquelle Rc, R2', R5' et R6' ont les significations indiquées ci-dessus,
soit à l'action d'un produit de formule (XIX):
NH-RaRb (XIX)
dans laquelle Ra et Rb identiques ou différents représentent un radical alkyle ou aryle tels que définis ci-dessus éventuellement substitués,
pour obtenir un produit de formule (XX) : 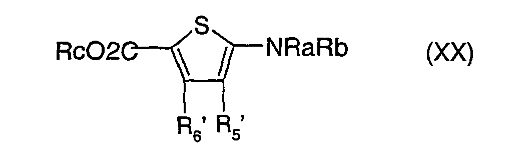 dans laquelle Rc, Ra, Rb, R5' et R6' ont les significations indiquées ci-dessus,
c) lorsque W représente un radical nitro, l'on peut soumettre le produit de formule (II) à une réaction d'estérification avec un alcool de formule (VI) tel que défini ci-dessus pour obtenir des produits de formule (XXI) : 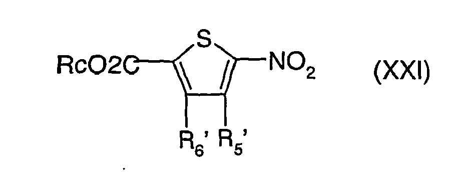 dans laquelle Rc, R5' et R6' ont les significations indiquées ci-dessus,
que l'on peut soumettre à une réaction avec un dérivé halogéné de formule (XXII):
RaRbHal (XXII)
dans laquelle Ra et Rb ont les significations indiquées ci-dessus et Hal représente un atome d'halogène,
pour obtenir un produit de formule (XX) telle que définie ci-dessus,
produits de formules (IV), (IV1), (IV2), (V), (V1), (V2), (schéma I) (XVIII), (XX) telles que définies ci-dessus que l'on peut faire réagir avec la diamine NH2-NH2 pour obtenir un produit de formule (XXIII) : 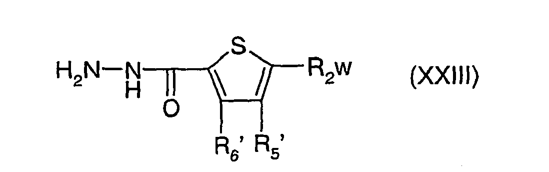 dans laquelle R5' et R6' ont les significations indiquées ci-dessus et R2w représente -CH2-phényle,
-(CH)phényle-CH2(phényle), R2' ou NRaRb telles que définies ci-dessus,
que l'on peut soumettre à une réaction avec un produit de formule (XXIV) :
R1'-COOH (XXIV)
dans laquelle R1' a la signification indiquée ci-dessus, pour obtenir un produit de formule (Iy) : 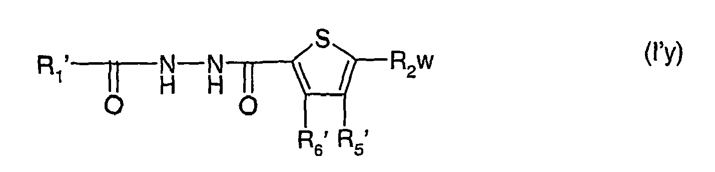
dans laquelle R1', R2w, R5' et R6' ont les significations indiquées ci-dessus,
produits de formule (Ix') et (Iy')qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés,
lesdits produits de formule (I) ainsi obtenus étant sous toutes les formes isomères possibles racémiques, énantiomères et diastéréoisomères.

7. A titre de médicaments, les produits de formule (I) telle que définie à la revendication 1 ainsi que les sels d'addition avec les acides minéraux et organiques ou avec les bases minérales et organiques pharmaceutiquement acceptables desdits produits de formule (I).

8. A titre de médicaments, les produits de formule (I) répondant aux formules (Ia) ou (Ib) telles que définies aux revendications 2 à 4, ainsi que les sels d'addition avec les acides minéraux et organiques ou avec les bases minérales et organiques pharmaceutiquement acceptables desdits produits de formules (Ia) et (Ib).

9. A titre de médicaments, les produits de formule (I) tels que définis à la revendication 5, ainsi que les sels d'addition avec les acides minéraux et organiques ou avec les bases minérales et organiques pharmaceutiquement acceptables desdits produits de formule (I).

10. Les compositions pharmaceutiques contenant à titre de principe actif, l'un au moins des médicaments tels que définis à l'une quelconque des revendications 7 à 9.

11. Utilisation de produits de formule (I) telle que définie à l'une quelconque des revendications 1 à 5 ou de sels pharmaceutiquement acceptables desdits produits de formule (I) pour la préparation de médicaments destinés à la prévention ou au traitement de maladies liées à un comportement physiologique anormal au niveau de la sécrétion et/ou de l'activité de cystéines protéases telles que notamment les cathepsines B, H, J, L, N, S, T, C, V, W, K ou O, 02 ou la papaine.

12. Utilisation telle que définie à la revendication 11 pour la préparation de médicaments destinés à la prévention ou au traitement de maladies liées à un comportement physiologique anormal au niveau de la sécrétion et/ou de l'activité de la cathepsine K.

13. Utilisation des produits de formule (I) telle que définie à l'une quelconque des revendications 1 à 5 ou de sels pharmaceutiquement acceptables desdits produits de formule (I) pour la préparation de médicaments destinés à la prévention ou au traitement de maladies du métabolisme du cartilage et de l'os, les maladies prolifératives, les cancers, les maladies cardiovasculaires, la resténose, les maladies du système nerveux central, les maladies du système immunitaire, les allergies, les maladies infectieuses, inflammatoires et les maladies autoimmunes.

14. Utilisation selon la revendication 13 **caractérisée en ce que** les maladies à prévenir ou à traiter sont choisies parmi les maladies du métabolisme du cartilage et de l'os, les maladies prolifératives, les cancers et les maladies neurodégénératives comme la maladie d'Alzheimer.

15. Utilisation selon la revendication 13 **caractérisée en ce que** les maladies à prévenir ou à traiter sont choisies parmi les maladies du métabolisme du cartilage et de l'os et les cancers osseux.

16. Utilisation selon la revendication 13 **caractérisée en ce que** les maladies à prévenir ou à traiter sont notamment l'ostéoporose, les maladies gingivales incluant gingivite et périodontosis, l'arthrite telle que notamment l'ostéoarthrite et l'arthrite rhumatoïde, la maladie de Paget, l'hypercalcémie et les cancers osseux.

17. Utilisation selon la revendication 13 **caractérisée en ce que** la maladie à prévenir ou à traiter est notamment l'ostéoporose.

## Claims

1. Products of formula (I): 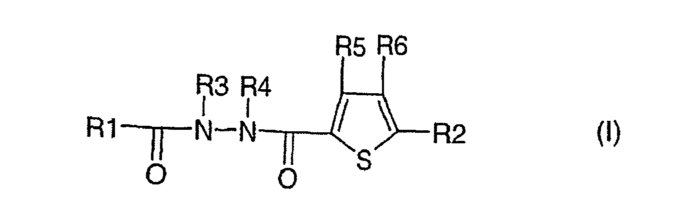 in which:
R1 represents a linear or branched alkyl or alkoxy radical containing up to 8 carbon atoms substituted either with a monocyclic radical or consisting of carbocyclic or heterocyclic, saturated or unsaturated fused rings that are up to 14-membered, containing one or more identical or different heteroatoms, O, N, NH or S, such a monocyclic radical or a radical consisting of fused rings itself being optionally substituted as indicated below,
or with an amino radical that is itself optionally substituted with a radical -C(O)-O-R7 or -C(O)-R7, R2 represents:
a) a linear or branched alkyl radical containing up to 8 carbon atoms optionally substituted with one or more radicals chosen from the radicals -NH-C(O)-O-R7, radicals -NH-C(O)-alk-NH-C(O)-O-R7 and monocyclic radicals or radicals consisting of carbocyclic or heterocyclic, saturated or unsaturated fused rings that are up to 14-membered, containing one or more identical or different heteroatoms, O, N, NH or S, such monocyclic radicals or radicals consisting of fused rings, themselves being optionally substituted as indicated below,
b) a phenyl radical optionally substituted with a linear or branched alkoxy radical containing up to 6 carbon atoms or an arylalkoxy radical, these alkoxy and arylalkoxy radicals themselves being optionally substituted as indicated below,
c) a naphthyl radical optionally substituted as indicated below,
d) an amino radical optionally substituted with one or two radicals, which may be identical or different, chosen from linear or branched alkyl radicals containing up to 6 carbon atoms, radicals -C(O)-alk-NH-C(O)-O-R7 and arylalkyl radicals, themselves optionally substituted with an alkoxyphenoxy radical in which the linear or branched alkoxy radical contains up to 6 carbon atoms, these alkyl, arylalkyl, alkoxy and alkoxyphenoxy radicals themselves being optionally substituted as indicated below,
e) -(CH₃)C=N-O-R8,
R3 and R4, which may be identical or different, represent a hydrogen atom or an alkyl, arylalkyl or aryl radical, in which the alkyl radicals are linear or branched, containing up to 6 carbon atoms, and the aryl radicals contain up to 10 carbon atoms, these alkyl, arylalkyl and aryl radicals being optionally substituted with a halogen atom, a hydroxyl radical or a linear or branched alkyl or alkoxy radical containing up to 4 carbon atoms,
R5 and R6, which may be identical or different, are chosen from a hydrogen atom, a hydroxyl radical and linear or branched alkyl and alkoxy radicals containing up to 6 carbon atoms, these alkyl and alkoxy radicals themselves being optionally substituted as indicated below,
R7 represents a linear or branched alkyl radical containing up to 6 carbon atoms, optionally substituted with an optionally substituted aryl or heteroaryl (indazolyl) radical,
R8 represents a hydrogen atom or a linear or branched alkyl radical containing up to 6 carbon atoms optionally substituted with an aryl (phenyl) radical, it being understood that all alkyl and alkoxy radicals, monocyclic radicals or radicals consisting of fused rings, aryl, heteroaryl, arylalkyl, arylalkoxy and alkoxyphenoxy radicals indicated above as being optionally substituted, are optionally substituted with one or more radicals chosen from halogen atoms; hydroxyl radicals; alkyl, alkenyl, alkylthio or alkoxy containing up to 6 carbon atoms; up to 6-membered cycloalkyl; acyl containing up to 7 carbon atoms; cyano; nitro; free, salified or esterified carboxyl; tetrazolyl; indazolyl; -NH2, -NH(alk) or -N(alk)(alk); -S02-NH-CO-NHR5 in which R5 represents alk or phenyl; -C(O)-NH2, -C(O)-NH(alk), -C (O) -N (alk) (alk), -NH-C (O) - (alk), -N(alk)-C(O)-(alk); and phenyl itself optionally substituted with an alkyl or alkoxy radical containing up to 4 carbon atoms,
it being understood that alk represents a linear or branched alkyl radical containing up to 4 carbon atoms, said products of formula (I) being in any possible racemic, enantiomeric or diastereoisomeric isomer form, and also the addition salts with mineral and organic acids or with mineral and organic bases of said products of formula (I).

2. Products of formula (I) as defined in Claim 1, corresponding to formula (Ia): 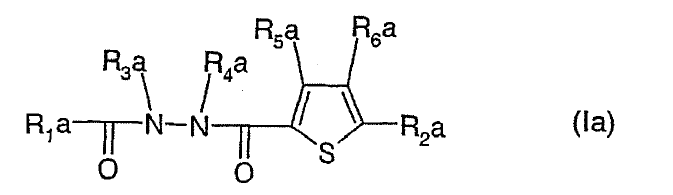 in which:
R1a represents a linear or branched alkyl or alkoxy radical containing up to 8 carbon atoms substituted with a phenyl radical, a radical consisting of saturated or unsaturated carbocyclic fused rings that are up to 14-membered and an amino radical that is itself optionally substituted with a radical -C(O)-OR7a or -C(O)-R7a, this phenyl radical and these radicals consisting of saturated or unsaturated carbocyclic fused rings being optionally substituted as indicated below,
R2a represents:
a) a linear or branched alkyl radical containing up to 6 carbon atoms optionally substituted with one or more radicals chosen from the radicals NH-C(O)-O-R7a and -NH-C(O)-alk-NH-C(O)-O-R7, a phenyl radical and saturated or unsaturated heterocyclic radicals that are up to 10-membered,
b) a phenyl radical optionally substituted with a linear or branched alkoxy radical containing up to 6 carbon atoms or a benzyloxy radical,
c) a naphthyl radical,
d) an amino radical optionally substituted with one or two radicals, which may be identical or different, chosen from linear or branched alkyl radicals containing up to 4 carbon atoms, radicals -C(O)-alk-NH-C(O)-O-R7a and a benzyl radical, itself optionally substituted with an alkoxyphenoxy radical in which the alkoxy radical contains up to 4 carbon atoms,
e) -(CH₃)C=N-O-R8,
R3a and R4a, which may be identical or different, represent a hydrogen atom, a linear or branched alkyl radical containing up to 6 carbon atoms or a phenyl radical optionally substituted with a hydroxyl or alkoxy radical containing up to 4 carbon atoms,
R5a and R6a, which may be identical or different, are chosen from the hydrogen atom, the hydroxyl radical and linear or branched alkyl or alkoxy radicals containing up to 6 carbon atoms optionally substituted with a phenyl or indazolyl radical,
R7a represents a linear or branched alkyl radical containing up to 4 carbon atoms optionally substituted with a phenyl radical or an indazolyl radical,
R8a represents a hydrogen atom or a linear or branched alkyl radical containing up to 4 carbon atoms optionally substituted with a phenyl radical,
it being understood that alk represents a linear or branched alkyl radical containing up to 4 carbon atoms, said products of formula (Ia) being in any possible racemic, enantiomeric or diastereoisomeric isomer form, and also the addition salts with mineral and organic acids or with mineral and organic bases of said products of formula (Ia).

3. Products of formula (I) as defined in Claims 1 and 2, corresponding to formula (Ib): 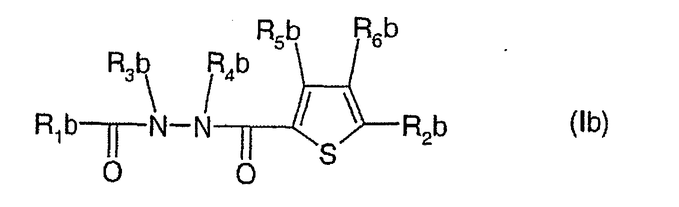 in which:
R1b represents a linear or branched alkyl or alkoxy radical containing up to 6 carbon atoms substituted with a radical chosen from phenyl and fluorene (Ex. 1 and 20) radicals and an amino radical that is itself optionally substituted with a radical -C(O)-O-R7b or -C(O)-R7b,
R2b represents:
a) a linear or branched alkyl radical containing up to 6 carbon atoms optionally substituted with one or more radicals chosen from the radicals -NH-C(O)-O-R7b and -NH-C(O)-alk-NH-C(O)-O-R7b and phenyl, pyridyl, pyridyl, piperidyl, benzofuryl, morpholinyl, quinolyl, indolyl, benzimidazolyl and indazolyl radicals,
b) a phenyl radical optionally substituted with a linear or branched alkoxy radical containing up to 4 carbon atoms or a benzyloxy radical,
c) a naphthyl radical,
d) an amino radical optionally substituted with one or two radicals, which may be identical or different, chosen from linear or branched alkyl radicals containing up to 4 carbon atoms, radicals -C(O)-alk-NH-C(O)-O-benzyl and a benzyl radical, itself optionally substituted with an alkoxyphenoxy radical in which the alkoxy radical contains up to 4 carbon atoms,
e) -(CH₃)C=N-O-R8,
R3b and R4b represent a hydrogen atom,
R5b and R6b, which may be identical or different, represent a hydrogen atom, a hydroxyl radical, a linear or branched alkyl or alkoxy radical containing up to 4 carbon atoms optionally substituted with a phenyl radical,
R7b represents a linear or branched alkyl radical containing up to 4 carbon atoms optionally substituted with a phenyl radical or an indazolyl radical,
R8b represents a hydrogen atom or a benzyl radical,
it being understood that alk represents a linear or branched alkyl radical containing up to 4 carbon atoms, said products of formula (Ib) being in any possible racemic, enantiomeric or diastereoisomeric isomer form, and also the addition salts with mineral and organic acids or with mineral and organic bases of said products of formula (Ib).

4. Products of formulae (I), (Ia) and (Ib) as defined in Claims 1 to 3, in which R3, R3a, R4, R4a, R5, R5a, R5b, R6, R6a and R6b represent a hydrogen atom, said products of formulae (I), (Ia) and (Ib) being in any possible racemic, enantiomeric or diastereoisomeric isomer form, and also the addition salts with mineral and organic acids or with mineral and organic bases of said products of formulae (I), (Ia) and (Ib).

5. Products of formula (I) below:
- 5-[(2S)-1-[[4-methyl-1-oxo-2-[[(phenylmethoxy)-carbonyl]amino]pentyl]amino]ethyl]-2-thiophenecarboxylic acid 2-[(2S)-4-methyl-1-oxo-2-[[(phenylmethoxy)carbonyl]amino]pentyl]hydrazide
- 5-[1-(hydroxyimino)ethyl]-2-thiophenecarboxylic acid 2-[[(9H-fluoren-9-yl)methoxy]carbonyl]hydrazide
- 5-[2-(phenylmethoxy)phenyl]-2-thiophenecarboxylic acid 2-[(2S)-4-methyl-1-oxo-2-[[(phenylmethoxy)-carbonyl]amino]pentyl]hydrazide
- 5-[[[3-(4-methoxyphenoxy)phenyl]methyl](phenylmethyl)amino]-2-thiophenecarboxylic acid 2-[(2S)-4-methyl-1-oxo-2-[[(phenylmethoxy)carbonyl]amino]pentyl]-hydrazide
- 5-[1-[(phenylmethoxy)imino]ethyl]-2-thiophenecarboxylic acid 2-[(2S)-4-methyl-1-oxo-2-[[(phenylmethoxy)carbonyl]amino]pentyl]hydrazide
- 5-[1-[[(phenylmethoxy)carbonyl]amino]ethyl]-2-thiophenecarboxylic acid 2-[[(9H-fluoren-9-yl)methoxy]-carbonyl]hydrazide
- 5-[1-[[(phenylmethoxy)carbonyl]amino]ethyl]-2-thiophenecarboxylic acid 2-[(2S)-4-methyl-1-oxo-2-[[(phenylmethoxy)carbonyl]amino]pentyl]hydrazide
- 5-[3-methyl-1-[(phenylmethoxy)carbonyl]amino]butyl]-2-thiophenecarboxylic acid 2-[(2S)-4-methyl-1-oxo-2-[[(phenylmethoxy)carbonyl]amino]pentyl]hydrazide
- 5-[3-methyl-1-[[(phenylmethoxy)carbonyl]amino]butyl]-2-thiophenecarboxylic acid 2-[(2S)-4-methyl-1-oxo-2-[[(phenylmethoxy)carbonyl]amino]pentyl]hydrazide isomer A
- 5-[3-methyl-1-[[(phenylmethoxy)carbonyl]amino]butyl]-2-thiophenecarboxylic acid [(2S)-4-methyl-1-oxo-2-[[(phenylmethoxy)carbonyl]amino]pentyl]hydrazide isomer B,
and also the addition salts with mineral and organic acids or with mineral and organic bases of said products of formula (I).

6. Process for preparing the products of formula (I) as defined in Claim 1, **characterized in that** the starting material used is a compound of formula (II): 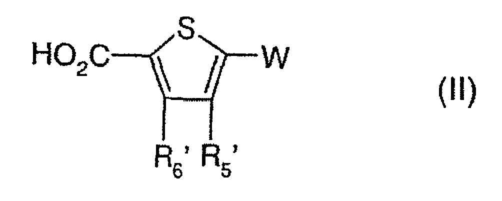 in which W represents a hydrogen atom, a halogen atom or a nitro radical, R5' and R6' have the meanings given in claim 1, respectively, for R5 and R6, in which the optional reactive functions are optionally protected with protecting groups,
which product of formula (II), depending on the value of W, may be subjected to the following reactions:
a) when W represents a hydrogen atom, the product of formula (II) may be subjected to the reactions of route i) or ii):
route i) the compound of formula (II) in which W represents a hydrogen atom may be subjected to the action of a bromo derivative of formula (III):
R2'-Br (III)
in which R2' has the meaning given in claim 1 for R2 in which the optional reactive functions are optionally protected with protecting groups, to give a product of formula (IV): 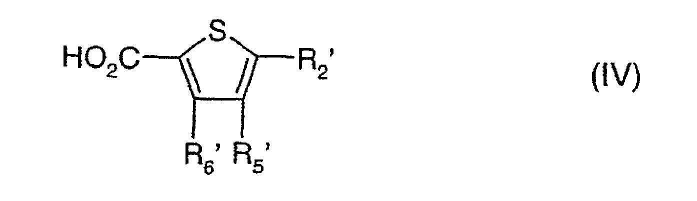 in which R2', R5' and R6' have the meanings given above,
when R2' represents a -CH2-phenyl radical, to give the product of formula (IV1): 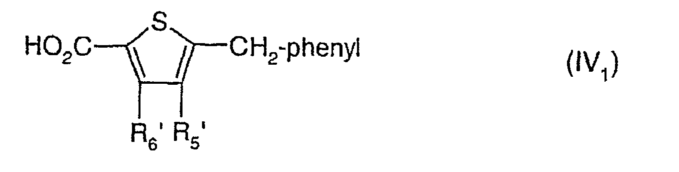 in which R5' and R6' have the meanings given above, which product may be subjected to a second reaction with the compound of formula (III) to give the compound of formula (IV2): 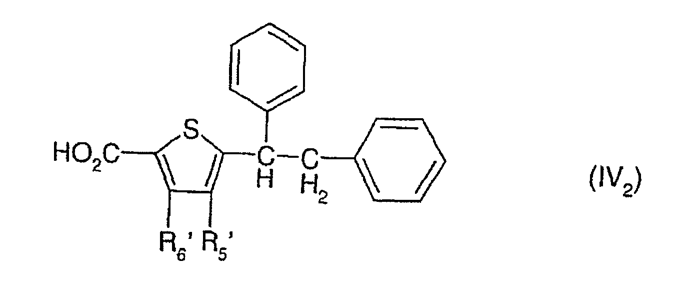 in which R5' and R6' have the meanings given above, which products of formulae (IV), (IV1) and (IV2) may be subjected to an esterification reaction to give the corresponding products of formulae (V), (V1) and (V2): 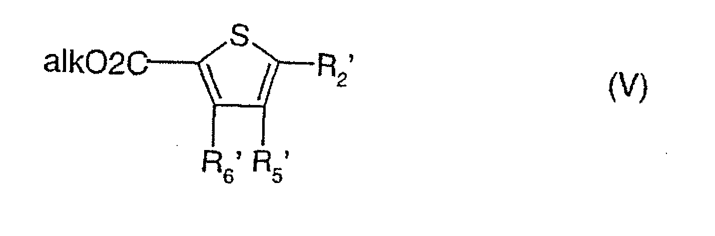 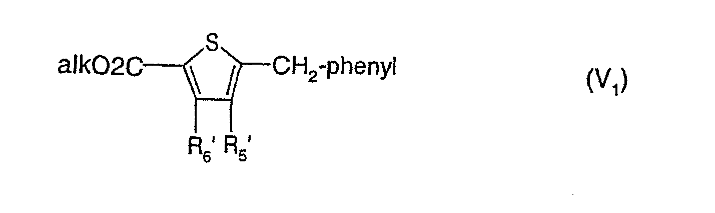 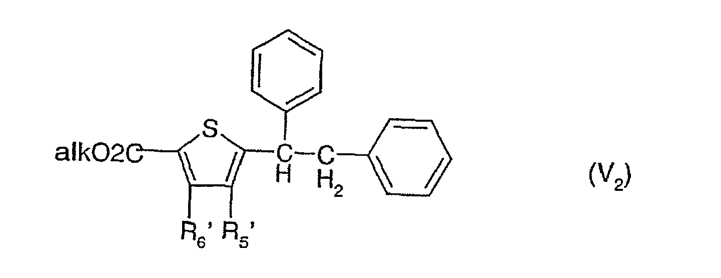 in which R2', R5' and R6' have the meanings given above and alk represents a linear or branched alkyl radical containing up to 4 carbon atoms,
route ii) the compound of formula (II) in which W represents a hydrogen atom may be subjected to an esterification reaction with an alcohol of formula (VI):
RC-OH (VI)
in which Rc represents a linear or branched alkyl radical containing up to 6 carbon atoms, to give products of formula (VII): 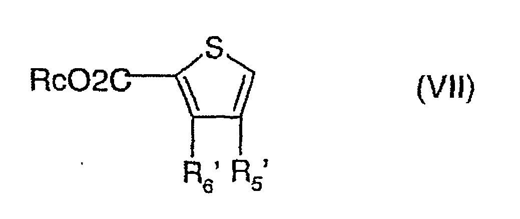 in which Rc, R5' and R6' have the meanings given above, which products are subjected to an aldehyde of formula (VIII):
Rd-CHO (VIII)
in which Rd represents a hydrogen atom, an alkyl radical as defined above or a monocyclic radical or a radical consisting of saturated or unsaturated carbocyclic or heterocyclic fused rings that are up to 14-membered, containing one or more identical or different heteroatoms O, N, NH or S, which are themselves optionally substituted, to give products of formula (IX): 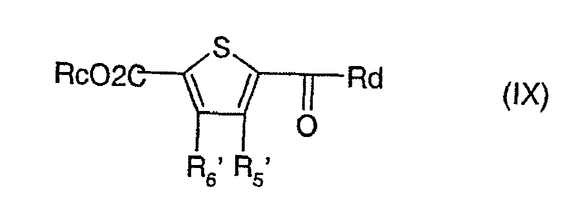 in which Rc, Rd, R5' and R6' have the meanings given above,
which products of formula (IX) are reacted with an amine of formula (X):
H2N-CH2-ORe (X)
in which Re represents an optionally substituted arylalkyl radical, to give products of formula (XI): 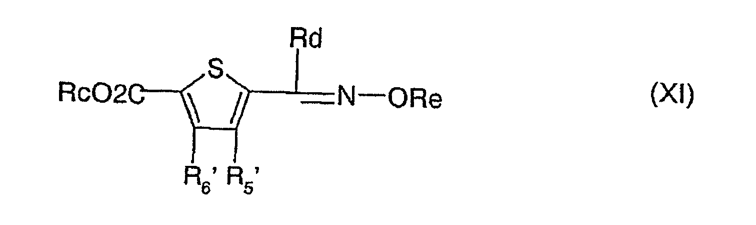 in which Rc, Rd, Re, R5' and R6' have the meanings given above,
which product of formula (XI) may be subjected to a selective reduction reaction,
to give products of formula (XII): 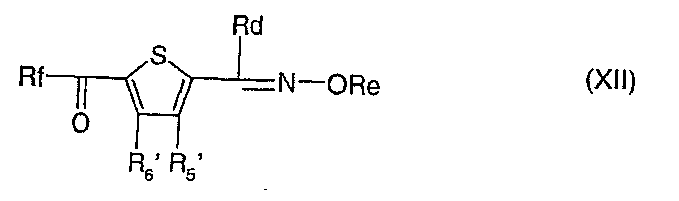 in which Rd, Re, R5' and R6' have the meanings given above,
and Rf represents a selective reduction group of ORc, which products of formulae (XI) and (XII) may be subjected to a reduction reaction to give the corresponding products of formula (XIII): 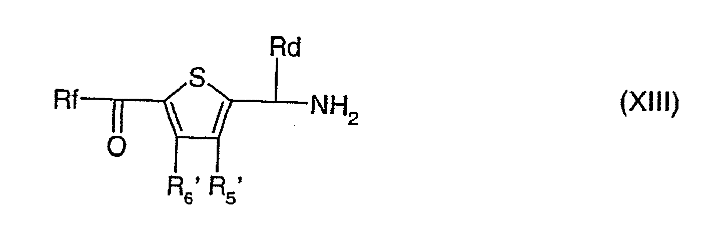 in which Rd, Rf, R5' and R6' have the meanings given above,
which products of formula (XIII) may be subjected to a saponification reaction to give the corresponding products of formula (XIV): 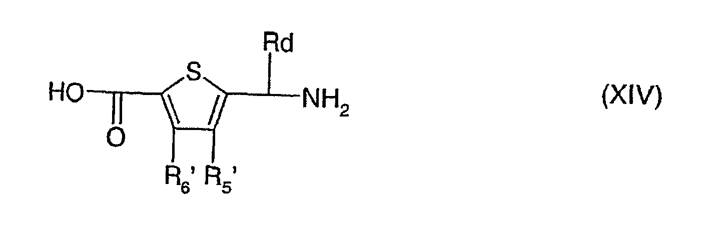 in which Rd, R5' and R6' have the meanings given above, which product of formula (XIV) may be reacted with a product of formula (XV): 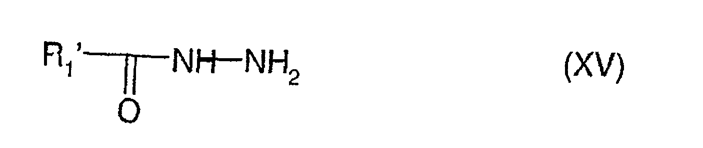 in which R1' has the meaning given in claim 1 for R1 in which the optional reactive functions are optionally protected with protecting groups, to give products of formula (I'x): 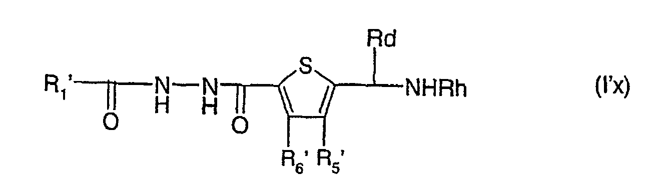 in which R1', Rd, R5' and R6' have the meanings given above and Rh represents -C(O)-O-R7 or -C(O)-alk-NH-C(O)-O-R7 with R7 as defined above,
b) when W represents a halogen atom, the product of formula (II) may be subjected to an esterification reaction with an alcohol of formula (VI) as defined above, to give products of formula (XVI): 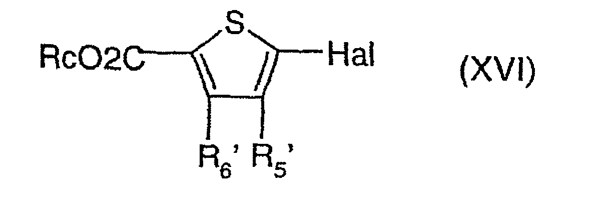 in which Rc, R5' and R6' have the meanings given above and Hal represents a halogen atom,
which product of formula (XVI) may be subjected:
either to the action of a boron derivative of formula (XVII):
R2'-B (OH) 2 (XVII)
in which R2' has the meaning given above, to give a product of formula (XVIII): 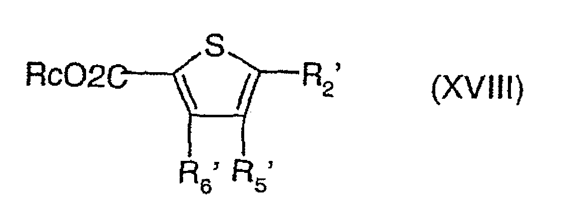 in which Rc, R2', R5' and R6' have the meanings given above,
or to the action of a product of formula (XIX):
NH-RaRb (XIX)
in which Ra and Rb, which may be identical or different, represent an optionally substituted alkyl or aryl radical as defined above,
to give a product of formula (XX): 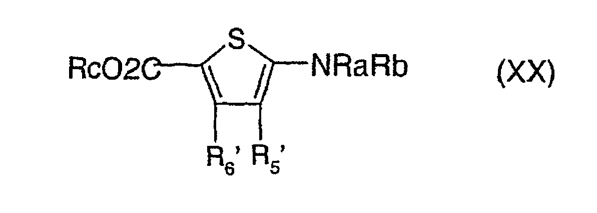 in which Rc, Ra, Rb, R5' and R6' have the meanings given above,
c) when W represents a nitro radical, the product of formula (II) may be subjected to an esterification reaction with an alcohol of formula (VI) as defined above, to give products of formula (XXI): 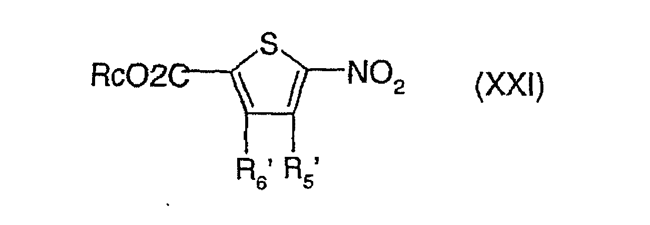 in which Rc, R5' and R6' have the meanings given above, which products may be subjected to a reaction with a halo derivative of formula (XXII):
RaRbHal (XXII)
in which Ra and Rb have the meanings given above and Hal represents a halogen atom,
to give a product of formula (XX) as defined above, which products of formulae (IV), (IV1), (IV2), (V), (VI), (V2), (scheme I) (XVIII) and (XX) as defined above may be reacted with a diamine NH2-NH2 to give a product of formula (XXIII): 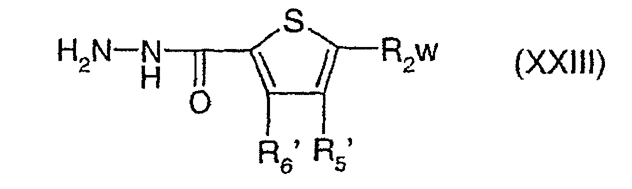 in which R5' and R6' have the meanings given above and R2w represents -CH2-phenyl, -(CH)phenyl-CH2(phenyl), R2' or NRaRb as defined above,
which product may be subjected to a reaction with a product of formula (XXIV):
R1'-COOH (XXIV)
in which R1' has the meaning given above, to give a product of formula (Iy): 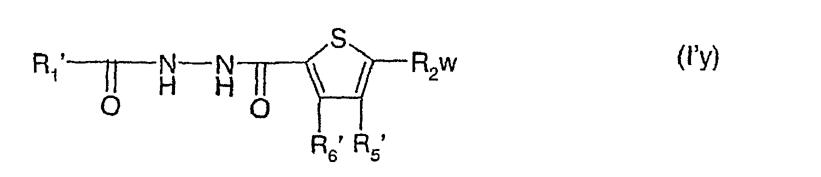 in which R1', R2w, R5' and R6' have the meanings given above,
which products of formulae (Ix') and (Iy') may be products of formula (I) and which, in order to obtain products or other products of formula (I), may be subjected, if desired and if necessary, to one or more of the following conversion reactions, in any order:
a) an esterification reaction of an acid function,
b) a saponification reaction of an ester function to an acid function,
c) an oxidation reaction of an alkylthio group to the corresponding sulfoxide or sulfone,
d) a reaction for conversion of a ketone function to an oxime function,
e) a reduction reaction of the free or esterified carboxyl function to an alcohol function,
f) a reaction for conversion of an alkoxy function to a hydroxyl function, or alternatively of a hydroxyl function to an alkoxy function,
g) an oxidation reaction of an alcohol function to an aldehyde, acid or ketone function,
h) a reaction for conversion of a nitrile radical to a tetrazolyl,
i) a reaction for removal of the protecting groups possibly borne by the protected reactive functions,
j) a salification reaction with a mineral or organic acid or with a base, to give the corresponding salt,
k) a reaction for resolving the racemic forms into resolved products,
said products of formula (I) thus obtained being in any possible racemic, enantiomeric or diastereoisomeric isomer form.

7. As medicaments, the products of formula (I) as defined in Claim 1, and also the pharmaceutically acceptable addition salts with mineral and organic acids or with mineral and organic bases of said products of formula (I).

8. As medicaments, the products of formula (I) corresponding to formulae (Ia) or (Ib) as defined in Claims 2 to 4, and also the pharmaceutically acceptable addition salts with mineral and organic acids or with mineral and organic bases of said products of formulae (Ia) and (Ib).

9. As medicaments, the products of formula (I) as defined in Claim 5, and also the pharmaceutically acceptable addition salts with mineral and organic acids or with mineral and organic bases of said products of formula (I).

10. Pharmaceutical compositions containing, as active principle, at least one of the medicaments as defined in any one of Claims 7 to 9.

11. Use of products of formula (I) as defined in any one of Claims 1 to 5, or of pharmaceutically acceptable salts of said products of formula (I), for the preparation of medicaments for preventing or treating diseases associated with an abnormal physiological behavior in the secretion and/or the activity of cysteine proteases such as, especially, the cathepsins B, H, J, L, N, S, T, C, V, W, K or O, 02 or papain.

12. Use as defined in Claim 11, for the preparation of medicaments for preventing or treating diseases associated with an abnormal physiological behavior in the secretion and/or the activity of cathepsin K.

13. Use of the products of formula (I) as defined in any one of Claims 1 to 5, or of pharmaceutically acceptable salts of said products of formula (I), for the preparation of medicaments for preventing or treating cartilage and bone metabolism disorders, proliferative diseases, cancers, cardiovascular diseases, restenosis, central nervous system diseases, immune system diseases, allergies, infectious and inflammatory diseases and autoimmune diseases.

14. Use according to Claim 13, **characterized in that** the diseases to be prevented or treated are chosen from cartilage and bone metabolism diseases, proliferative diseases, cancers and neurodegenerative diseases, for instance Alzheimer's disease.

15. Use according to Claim 13, **characterized in that** the diseases to be prevented or treated are chosen from cartilage and bone metabolism diseases and bone cancers.

16. Use according to Claim 13, **characterized in that** the diseases to be prevented or treated are especially osteoporosis, gingival diseases including gingivitis and periodontosis, arthritis such as, especially, osteoarthritis and rheumatoid arthritis, Paget's disease, hypercalcemia and bone cancers.

17. Use according to Claim 13, **characterized in that** the disease to be prevented or treated is especially osteoporosis.

## Patentansprüche

1. Produkte der Formel (I): 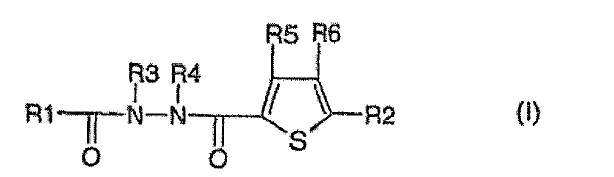 worin:
R1 einen geraden oder verzweigten Alkyl- oder Alkoxyrest darstellt, der höchstens 8 Kohlenstoffatome enthält und entweder mit einem monocyclischen oder einem aus kondensierten carbocyclischen oder heterocyclischen, gesättigten oder ungesättigten Ringen, die höchstens 14 Ringglieder umfassen und ein oder mehrere gleiche oder unterschiedliche Heteroatome O, N, NH oder S enthalten, bestehenden Rest, wobei ein derartiger monocyclischer oder aus kondensierten Ringen bestehender Rest selbst gegebenenfalls wie nachstehend angegeben substituiert ist,
oder mit einem Aminorest substituiert ist, der selbst gegebenenfalls mit einem Rest -C(O)-O-R7 oder -C(O)-R7 substituiert ist,
R2 Folgendes darstellt:
a) einen geraden oder verzweigten Alkylrest, der höchstens 8 Kohlenstoffatome umfasst und gegebenenfalls mit einem oder mehreren Resten substituiert ist, die aus den Resten -NH-C(O)-O-R7, den Resten -NH-C(O)-alk-NH-C(O)-O-R7 und den monocyclischen oder aus kondensierten carbocyclischen oder heterocyclischen, gesättigten oder ungesättigten Ringen, die höchstens 14 Ringglieder umfassen und ein oder mehrere gleiche oder unterschiedliche Heteroatome O, N, NH oder S enthalten, bestehenden Resten ausgewählt sind, wobei derartige monocyclische oder aus kondensierten Ringen bestehende Reste selbst gegebenenfalls wie nachstehend angegeben substituiert sind,
b) einen Phenylrest, der gegebenenfalls mit einem geraden oder verzweigten Alkoxyrest, der höchstens 6 Kohlenstoffatome enthält, oder einem Arylalkoxyrest substituiert ist, wobei diese Alkoxy- und Arylalkoxyreste selbst gegebenenfalls wie nachstehend angegeben substituiert sind,
c) einen Naphthylrest, der gegebenenfalls wie nachstehend angegeben substituiert ist,
d) einen Aminorest, der gegebenenfalls mit einem oder zwei gleichen oder unterschiedlichen Resten substituiert ist, die aus geraden oder verzweigten Alkylresten, die höchstens 6 Kohlenstoffatome enthalten, den Resten -C(O)-alk-NH-C(O)-O-R7 und Arylalkylresten ausgewählt sind, die selbst gegebenenfalls mit einem Alkoxyphenoxyrest substituiert sind, wobei der gerade oder verzweigte Alkoxyrest höchstens 6 Kohlenstoffatome umfasst, wobei diese Alkyl-, Arylalkyl-, Alkoxy- und Alkoxyphenoxyreste selbst gegebenenfalls wie nachstehend angegeben substituiert sind,
e) -(CH3)C=N-O-R8,
R3 und R4, die gleich oder unterschiedlich sind, ein Wasserstoffatom, einen Alkyl-, Arylalkyl- oder Arylrest darstellen, in denen die Alkylreste gerade oder verzweigt sind und höchstens 6 Kohlenstoffatome umfassen und die Arylreste höchstens 10 Kohlenstoffatome enthalten, wobei diese Alkyl-, Arylalkyl- und Arylreste gegebenenfalls mit einem Halogenatom, einem Hydroxyl- oder einem geraden oder verzweigten Alkyl- oder Alkoxyrest, der höchstens 4 Kohlenstoffatome enthält, substituiert sind,
R5 und R6, die gleich oder unterschiedlich sind, aus einem Wasserstoffatom, einem Hydroxylrest und geraden oder verzweigten Alkyl- und Alkoxyresten, die höchstens 6 Kohlenstoffatome enthalten, ausgewählt sind, wobei diese Alkyl- und Alkoxyreste selbst gegebenenfalls wie nachstehend angegeben substituiert sind,
R7 einen geraden oder verzweigten Alkylrest darstellt, der höchstens 6 Kohlenstoffatome enthält und gegebenenfalls mit einem gegebenenfalls substituierten Aryl- oder Heteroaryl- (Indazolyl-) rest substituiert ist,
R8 ein Wasserstoffatom oder einen geraden oder verzweigten Alkylrest darstellt, der höchstens 6 Kohlenstoffatome enthält und gegebenenfalls mit einem Arylrest (Phenylrest) substituiert ist, wobei es sich versteht, dass alle Alkyl-, Alkoxy-, monocyclischen oder aus kondensierten Ringen bestehen-den, Aryl-, Heteroaryl-, Arylalkyl-, Arylalkoxy- und Alkoxyphenoxyreste, von denen vorstehend angegeben ist, dass sie gegebenenfalls substituiert sind, gegebenenfalls mit einem oder mehreren Resten substituiert sind, die ausgewählt sind aus Halogenatomen; Hydroxyl-; Alkyl-, Alkenyl-, Alkylthio- oder Alkoxyresten, die höchstens 6 Kohlenstoffatome enthalten; höchstens 6 Ringglieder enthaltenden Cycloalkyl-; höchstens 7 Kohlenstoffatome enthaltenden Acylresten; Cyano-; Nitro-; freien, versalzten oder veresterten Carboxy-; Tetrazolyl-; Indazolylgruppen; -NH2, -NH(Alk), -N(Alk)(alk); -S02-NH-CO-NHR5, worin R5 für Alk oder Phenyl steht; -C(O)-NH2, -C(O)-NH(Alk), -C(O) -N (Alk) (alk), -NH-C(O)-(Alk), -N (Alk) -C (O) - (alk); und Phenyl, das selbst gegebenenfalls mit einem Alkyl- oder Alkoxyrest, die höchstens 4 Kohlenstoffatome enthalten, substituiert ist,
wobei es sich versteht, dass Alk einen geraden oder verzweigten Alkylrest, der höchstens 4 Kohlenstoffatome enthält, darstellt,
wobei die Produkte der Formel (I) in allen möglichen racemischen, enantiomeren und diastereoisomeren isomeren Formen vorliegen, sowie die Additions-salze der Produkte der Formel (I) mit anorganischen und organischen Säuren oder mit anorganischen und orga-nischen Basen.

2. Produkte der Formel (I) nach Anspruch 1, die der Formel (Ia) entsprechen: 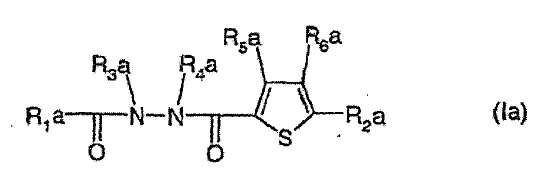 worin:
R1a einen geraden oder verzweigten Alkyl- oder Alkoxyrest darstellt, der höchstens 8 Kohlenstoffatome enthält und mit einem Phenylrest, einem aus kondensierten, gesättigten oder ungesättigten carbocyclischen Ringen, die höchstens 14 Ringglieder umfassen, bestehenden Rest und einem Aminorest substituiert ist, der selbst gegebenenfalls mit einem Rest -C(O)-O-R7a oder -C(O)-R7a substituiert ist, wobei der Phenylrest und die aus kondensierten, gesättigten oder ungesättigten carbocyclischen Ringen bestehenden Reste gegebenenfalls wie nachstehend angegeben substituiert sind,
R2a Folgendes darstellt:
a) einen geraden oder verzweigten Alkylrest, der höchstens 6 Kohlenstoffatome umfasst und gegebenenfalls mit einem oder mehreren Resten substituiert ist, die aus den Resten NH-C(O)-O-R7a und -NH-C(O)-alk-NH-C(O)-O-R7, einem Phenylrest und gesättigten oder ungesättigten heterocyclischen Resten mit höchstens 10 Ringgliedern ausgewählt sind,
b) einen Phenylrest, der gegebenenfalls mit einem geraden oder verzweigten Alkoxyrest, der höchstens 6 Kohlenstoffatome enthält, oder einem Benzyloxyrest substituiert ist,
c) einen Naphthylrest,
d) einen Aminorest, der gegebenenfalls mit einem oder zwei gleichen oder unterschiedlichen Resten substituiert ist, die aus geraden oder verzweigten Alkylresten, die höchstens 4 Kohlenstoffatome enthalten, den Resten -C(O)-alk-NH-C(O)-O-R7a und dem Benzylrest ausgewählt sind, der selbst gegebenenfalls mit einem Alkoxyphenoxyrest substituiert ist, wobei der Alkoxyrest höchstens 4 Kohlenstoffatome umfasst,
e) -(CH3)C=N-O-R8,
R3a und R4a, die gleich oder unterschiedlich sind, ein Wasserstoffatom, einen geraden oder verzweigten Alkylrest, der höchstens 6 Kohlenstoffatome enthält, oder einen Phenylrest darstellen, der gegebenenfalls mit einem Hydroxyl- oder Alkoxyrest, der höchstens 4 Kohlenstoffatome enthält, substituiert ist,
R5a und R6a, die gleich oder unterschiedlich sind, aus einem Wasserstoffatom, einem Hydroxylrest und geraden oder verzweigten Alkyl- oder Alkoxyresten ausgewählt sind, die höchstens 6 Kohlenstoffatome enthalten und gegebenenfalls mit einem Phenyl- oder Indazolylrest substituiert sind,
R7a einen geraden oder verzweigten Alkylrest darstellt, der höchstens 4 Kohlenstoffatome enthält und gegebenenfalls mit einem Phenylrest oder einem Indazolylrest substituiert ist,
R8a ein Wasserstoffatom oder einen geraden oder verzweigten Alkylrest darstellt, der höchstens 4 Kohlenstoffatome enthält und gegebenenfalls mit einem Phenylrest substituiert ist,
wobei es sich versteht, dass Alk einen geraden oder verzweigten Alkylrest, der höchstens 4 Kohlenstoffatome enthält, darstellt,
wobei die Produkte der Formel (Ia) in allen möglichen racemischen, enantiomeren und diaste-reoisomeren isomeren Formen vorliegen, sowie die Additionssalze der Produkte der Formel (Ia) mit anorganischen und organischen Säuren oder mit anorganischen und organischen Basen.

3. Produkte der Formel (I) nach Anspruch 1 und 2, die der Formel (Ib) entsprechen: 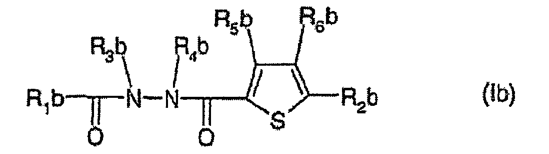 worin:
R1b einen geraden oder verzweigten Alkyl- oder Alkoxyrest darstellt, der höchstens 6 Kohlenstoffatome enthält und mit einem Rest substituiert ist, der ausgewählt ist aus den Resten Phenyl, Fluoren (Bsp. 1 und 20) und Amino, der selbst gegebenenfalls mit einem Rest -C(O)-O-R7b oder -C(O)-R7b substituiert ist,
R2b Folgendes darstellt:
a) einen geraden oder verzweigten Alkylrest, der höchstens 6 Kohlenstoffatome umfasst und gegebenenfalls mit einem oder mehreren Resten substituiert ist, die aus den Resten -NH-C(O)-O-R7b und -NH-C(O)-alk-NH-C(O)-O-R7b und den Resten Phenyl, Pyridyl, Pyridinyl, Piperidinyl, Benzofuranyl, Morpholinyl, Chinolinyl, Indolyl, Benzimidazolyl und Indazolyl ausgewählt sind,
b) einen Phenylrest, der gegebenenfalls mit einem geraden oder verzweigten Alkoxyrest, der höchstens 4 Kohlenstoffatome enthält, oder einem Benzyloxyrest substituiert ist,
c) einen Naphthylrest,
d) einen Aminorest, der gegebenenfalls mit einem oder zwei gleichen oder unterschiedlichen Resten substituiert ist, die aus geraden oder verzweigten Alkylresten, die höchstens 4 Kohlenstoffatome enthalten, den Resten -C(O)-alk-NH-C(O)-O-Benzyl und dem Benzylrest ausgewählt sind, der selbst gegebenenfalls mit einem Alkoxyphenoxyrest substituiert ist, wobei der Alkoxyrest höchstens 4 Kohlenstoffatome umfasst,
e) -(CH3)C=N-O-R8,
R3b und R4b ein Wasserstoffatom darstellen,
R5b und R6b, die gleich oder unterschiedlich sind, ein Wasserstoffatom, einen Hydroxylrest, einen geraden oder verzweigten Alkyl- oder Alkoxyrest darstellen, der höchstens 4 Kohlenstoffatome enthält und gegebenenfalls mit einem Phenylrest substituiert ist,
R7b einen geraden oder verzweigten Alkylrest darstellt, der höchstens 4 Kohlenstoffatome enthält und gegebenenfalls mit einem Phenylrest oder einem Indazolylrest substituiert ist,
R8b ein Wasserstoffatom oder den Benzylrest darstellt,
wobei es sich versteht, dass Alk einen geraden oder verzweigten Alkylrest, der höchstens 4 Kohlenstoffatome enthält, darstellt,
wobei die Produkte der Formel (Ib) in allen möglichen racemischen, enantiomeren und diastereoisomeren isomeren Formen vorliegen, sowie die Additionssalze der Produkte der Formel (Ib) mit anorganischen und organischen Säuren oder mit anorganischen und organischen Basen.

4. Produkte der Formeln (I), (Ia) und (Ib) nach den Ansprüchen 1 bis 3, worin R3, R3a, R4, R4a, R5, R5a, R5b, R6, R6a, R6b ein Wasserstoffatom darstellen,
wobei die Produkte der Formeln (I), (Ia) und (Ib) in allen möglichen racemischen, enantiomeren und diastereoisomeren isomeren Formen vorliegen, sowie die Additionssalze der Produkte der Formeln (I), (Ia) und (Ib) mit anorganischen und organischen Säuren oder mit anorganischen und organischen Basen.

5. Produkte der Formel (I) wie folgt:
- 5-[(2S)-1-[[4-Methyl-1-oxo-2-[[(phenylmethoxy)-carbonyl]amino]pentyl]amino]ethyl]-2-thiophencarbonsäure-2-[(2S)-4-methyl-1-oxo-2-[[(phenylmethoxy)carbonyl]amino]pentyl]hydrazid
- 5-[1-(Hydroxyimino)ethyl]-2-thiophencarbonsäure-2-[[(9H-fluoren-9-yl)methoxy]carbonyl]hydrazid
- 5-[2-(Phenylmethoxy)phenyl]-2-thiophencarbonsäure-2-[(2S)-4-methyl-1-oxo-2-[[(phenylmethoxy)-carbonyl]-amino]pentyl]hydrazid
- 5-[[[3-(4-Methoxyphenoxy)phenyl]methyl](phenylmethyl)amino]-2-thiophencarbonsäure-2-[(2S)-4-methyl-1-oxo-2-[[(phenylmethoxy)carbonyl]amino]pentyl]-hydrazid
- 5-[1-[(Phenylmethoxy)imino]ethyl]-2-thiophencarbonsäure-2-[(2S)-4-methyl-1-oxo-2-[[(phenylmethoxy)carbonyl]amino]pentyl]hydrazid
- 5-[1-[[(Phenylmethoxy)carbonyl]amino]ethyl]-2-thiophencarbonsäure-2-[[(9H-fluoren-9-yl)methoxy]-carbonyl]hydrazid
- 5-[1-[[(Phenylmethoxy)carbonyl]amino]ethyl]-2-thiophencarbonsäure-2-[(2S)-4-methyl-1-oxo-2-[[(phenylmethoxy)carbonyl]amino]pentyl]hydrazid
- 5-[3-Methyl-1-[(phenylmethoxy)carbonyl]amino]-butyl]-2-thiophencarbonsäure-2-[(2S)-4-methyl-1-oxo-2-[[(phenylmethoxy)carbonyl]amino]pentyl]hydrazid
- 5-[3-Methyl-1-[[(phenylmethoxy)carbonyl]amino]-butyl]-2-thiophencarbonsäure-2-[(2S)-4-methyl-1-oxo-2-[[(phenylmethoxy)carbonyl]amino]pentyl]hydrazid Isomer A und
- 5-[3-Methyl-1-[[(phenylmethoxy)carbonyl]amino]-butyl]-2-thiophencarbonsäure-[(2S)-4-methyl-1-oxo-2-[[(phenylmethoxy)carbonyl]amino]pentyl]hydrazid Isomer B
sowie die Additionssalze der Produkte der Formel (I) mit anorganischen und organischen Säuren oder mit anorganischen und organischen Basen.

6. Verfahren zur Herstellung der Produkte der Formel (I) nach Anspruch 1, **dadurch gekennzeichnet, dass** man von einer Verbindung der Formel (II): 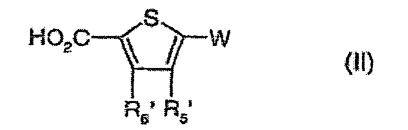 ausgeht, worin W ein Wasserstoffatom, ein Halogenatom oder einen Nitrorest darstellt, R5' und R6' die in Anspruch 1 für R5 beziehungsweise R6 angegebenen Bedeutungen haben, wobei die eventuellen reaktiven Funktionen gegebenenfalls mit Schutzgruppen geschützt werden,
wobei man das Produkt der Formel (II) je nach dem Wert von W folgenden Reaktionen unterwerfen kann:
a) wenn W ein Wasserstoffatom darstellt, kann man das Produkt der Formel (II) den Reaktionen der Wege i) oder ii) unterwerfen:
Weg i) man kann das Produkt der Formel (II), worin W ein Wasserstoffatom darstellt, der Wirkung eines Bromderivats der Formel (III) unterwerfen:
R2'-Br (III)
worin R2' die in Anspruch 1 für R2 angegebene Bedeutung hat, wobei die eventuellen reaktiven Funktionen gegebenenfalls mit Schutzgruppen geschützt werden,
um ein Produkt der Formel (IV) zu erhalten: 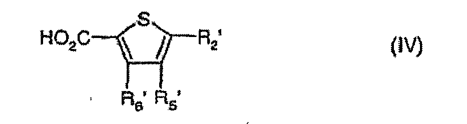 worin R2', R5' und R6' die vorstehend angegebenen Bedeutungen haben,
das man, wenn R2' einen Rest -CH2-Phenyl darstellt, so dass das Produkt der Formel (IV1): 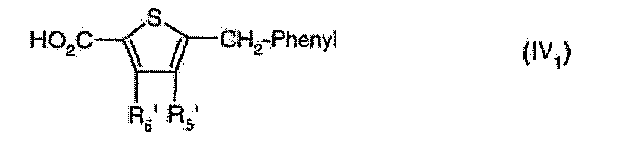 worin R5' und R6' die vorstehend angegebenen Bedeutungen haben, erhalten wird, einer zweiten Reaktion mit der Verbindung der Formel (III) unterwerfen kann, um die Verbindung der Formel (IV2) zu erhalten: 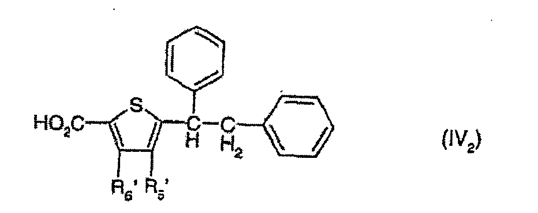 worin R5' und R6' die vorstehend angegebenen Bedeutungen haben
wobei man die Produkt der Formeln (IV), (IV1) und (IV2) einer Veresterungsreaktion unterwerfen kann, um die entsprechenden Produkte der Formeln (V), (V1) und (V2) zu erhalten: 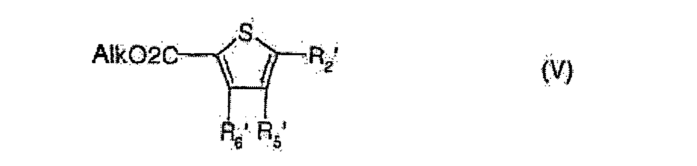 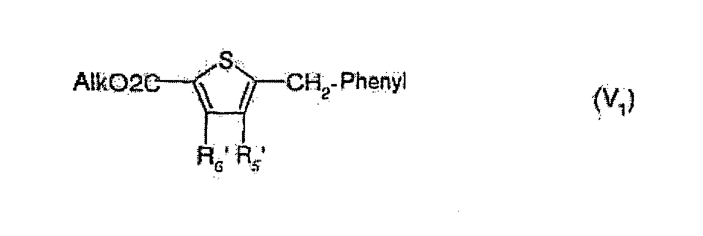 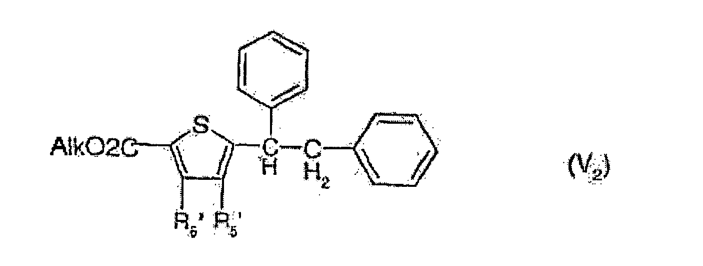 worin R2', R5' und R6' die vorstehend angegebenen Bedeutungen haben und Alk einen geraden oder verzweigten Alkylrest darstellt, der höchstens 4 Kohlenstoffatome enthält,
Weg ii) man kann die Verbindung der Formel (II), worin W ein Wasserstoffatom darstellt, einer Veresterungsreaktion mit einem Alkohol der Formel (VI) unterwerfen:
Rc-OH- (VI)
worin Rc einen geraden oder verzweigten Alkylrest darstellt, der höchstens 6 Kohlenstoffatome enthält,
um die Produkte der Formel (VII) zu erhalten: 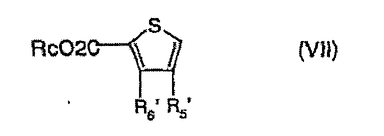 worin Rc, R5' und R6' die vorstehend angegebenen Bedeutungen haben,
die man einem Aldehyd der Formel (VIII) unterwirft:
Rd-CHO (VIII)
worin Rd ein Wasserstoffatom, einen Alkylrest, wie vorstehend definiert, oder einen monocyclischen oder einen aus kondensierten, carbocyclischen oder heterocyclischen, gesättigten oder ungesättigten Ringen, die höchstens 14 Ringglieder umfassen und ein oder mehrere gleiche oder unterschiedliche Heteroatome O, N, NH oder S enthalten, bestehenden Rest darstellt, die selbst gegebenenfalls substituiert sind,
um Produkte der Formel (IX) zu erhalten: 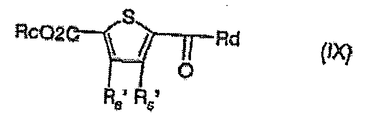 worin Rc, Rd, R5' und R6' die vorstehend angegebenen Bedeutungen haben,
wobei man die Produkte der Formel (IX) mit einem Amin der Formel (X) umsetzt:
H2N-CH2-ORe (X)
worin Re einen gegebenenfalls substituierten Arylalkylrest darstellt,
um Produkte der Formel (XI) zu erhalten: 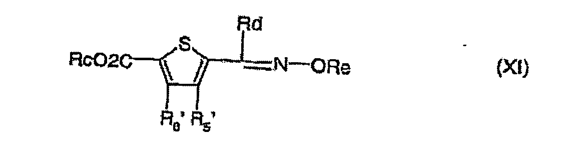 worin Rc, Rd, Re, R5' und R6' die vorstehend angegebenen Bedeutungen haben,
wobei man das Produkt der Formel (XI) einer selektiven Reduktionsreaktion unterwerfen kann,
um Produkte der Formel (XII) zu erhalten: 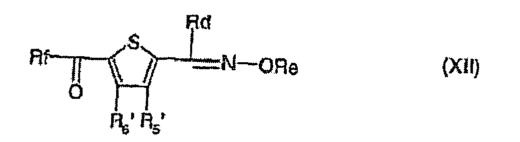 worin Rd, Re, R5' und R6' die vorstehend angegebenen Bedeutungen haben,
und Rf eine Gruppe zur selektiven Reduktion von ORc darstellt,
wobei man die Produkte der Formeln (XI) und (XII) einer Reduktionsreaktion unterwerfen kann, um die entsprechenden Produkte der Formel (XIII) zu erhalten: 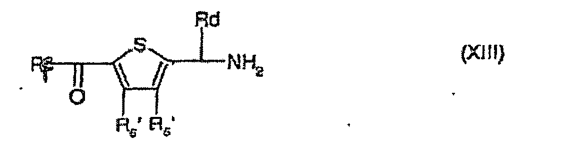 worin Rd, Rf, R5' und R6' die vorstehend angegebenen Bedeutungen haben,
man die Produkte der Formel (XIII) einer Verseifungsreaktion unterwerfen kann, um die entsprechenden Produkte der Formel (XIV) zu erhalten: 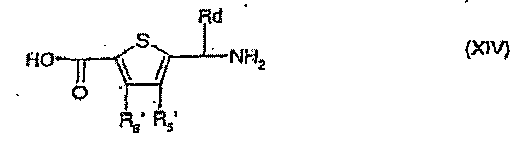 worin Rd, R5' und R6' die vorstehend angegebenen Bedeutungen haben,
man die Produkte der Formel (XIV) mit einem Produkt der Formel (XV) umsetzen kann: 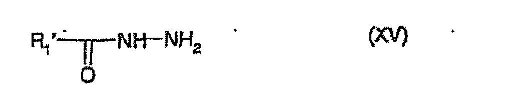 worin R1' die in Anspruch 1 für R1 angegebene Bedeutung hat, wobei die eventuellen reaktiven Funktionen gegebenenfalls durch Schutzgruppen geschützt werden,
um Produkte der Formel (I'x) zu erhalten: 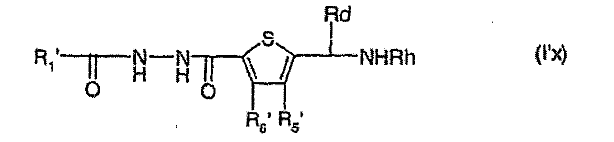 worin R1', Rd, R5' und R6' die vorstehend angegebenen Bedeutungen haben und Rh für -C(O)-O-R7 oder -C(O)-alk-NH-C(O)-O-R7 steht, mit R7 wie vorstehend definiert,
b) wenn W ein Halogenatom darstellt, kann man das Produkt der Formel (II) einer Veresterungsreaktion mit einem Alkohol der Formel (VI), wie vorstehend definiert, unterwerfen, um Produkte der Formel (XVI) zu erhalten: 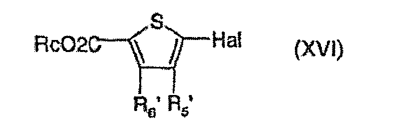 worin Rc, R5' und R6' die vorstehend angegebenen Bedeutungen haben und Hal für ein Halogenatom steht,
wobei man das Produkt der Formel (XVI) Folgendem unterwerfen kann:
entweder der Einwirkung eines Borderivats der Formel (XVII):
R2'-B(OH)2 (XVII)
worin R2' die vorstehend angegebene Bedeutung hat, um ein Produkt der Formel (XVIII) zu erhalten: 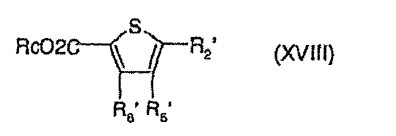 worin Rc, R2', R5' und R6' die vorstehend angegebenen Bedeutungen haben,
oder der Einwirkung eines Produkts der Formel (XIX) :
NH-RaRb (XIX)
worin Ra und Rb gleich oder unterschiedlich sind und einen gegebenenfalls substituierten Alkyl- oder Arylrest, wie vorstehend definiert, darstellen
um ein Produkt der Formel (XX) zu erhalten: 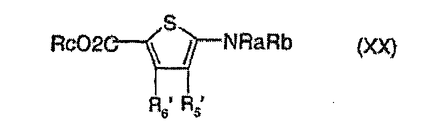 worin Rc, Ra, Rb, R5' und R6' die vorstehend angegebenen Bedeutungen haben,
c) wenn W einen Nitrorest darstellt, kann man das Produkt der Formel (II) einer Veresterungsreaktion mit einem Alkohol der Formel (VI), wie vorstehend definiert, unterwerfen, um Produkte der Formel (XXI) zu erhalten: 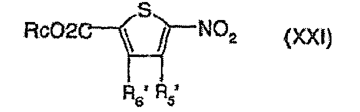 worin Rc, R5' und R6' die vorstehend angegebenen Bedeutungen haben,
die man einer Umsetzung mit einem Halogenderivat der Formel (XXII) unterwerfen kann:
RaRbHal (XXII)
worin Ra und Rb die vorstehend angegebenen Bedeutungen haben und Hal für ein Halogenatom steht,
um ein Produkt der Formel (XX), wie oben definiert, zu erhalten,
wobei man die Produkte der Formeln (IV), (IV1), (IV2), (V), (V1), (V2), (Schema I) (XVIII), (XX), wie vorstehend definiert, mit einem Diamin NH2-NH2 umsetzen kann, um ein Produkt der Formel (XXIII) zu erhalten: 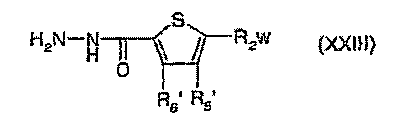 worin R5' und R6' die vorstehend angegebenen Bedeutungen haben und R2w für -CH2-Phenyl, -(CH)-Phenyl-CH2(Phenyl), R2' oder NRaRb, wie oben definiert, steht,
das man einer Reaktion mit einem Produkt der Formel (XXIV) unterwerfen kann:
R1'-COOH (XXIV)
worin R1 die vorstehend angegebene Bedeutung hat, um ein Produkt der Formel (Iy) zu erhalten: 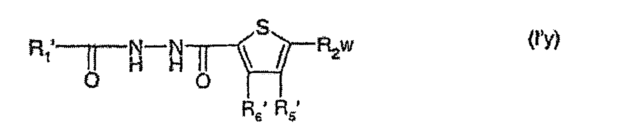
worin R1', R2w, R5' und R6' die vorstehend angegebenen Bedeutungen haben,
wobei die Produkte der Formel (Ix') und (Iy') Produkte der Formel (I) sein können und man sie, um andere Produkte der Formel (I) zu erhalten, wenn gewünscht und wenn notwendig, einer oder mehreren der folgenden Umwandlungsreaktionen in beliebiger Reihenfolge unterwerfen kann:
a) einer Veresterungsreaktion der Säurefunktion,
b) einer Verseifungsreaktion der Esterfunktion in eine Säurefunktion,
c) einer Oxidationsreaktion der Alkylthiogruppe in das entsprechende Sulfoxid oder Sulfon,
d) einer Reaktion zur Umwandlung der Ketonfunktion in eine Oximfunktion,
e) einer Reduktionsreaktion der freien oder veresterten Carboxyfunktion in eine Alkoholfunktion,
f) einer Reaktion zur Umwandlung der Alkoxyfunktion in eine Hydroxylfunktion oder auch der Hydroxylfunktion in eine Alkoxyfunktion,
g) einer Oxidationsreaktion der Alkoholfunktion in eine Aldehyd-, Säure- oder Ketonfunktion,
h) einer Reaktion zur Umwandlung des Nitrilrests in ein Tetrazolyl,
i) einer Reaktion zur Entfernung der Schutzgruppen, die die geschützten reaktiven Funktionen tragen können,
j) einer Salzbildungsreaktion mit einer anorganischen oder organischen Säure oder einer Base, um das entsprechende Salz zu erhalten,
k) einer Reaktion zur Aufspaltung der racemischen Formen in aufgespaltene Produkte,
wobei die so erhaltenen Produkte der Formel (I) in allen möglichen isomeren racemischen, enantiomeren oder diastereoisomeren Formen vorliegen.

7. Produkte der Formel (I) nach Anspruch 1 sowie die pharmazeutisch annehmbaren Additionssalze der Produkte der Formel (I) mit anorganischen und organischen Säuren oder mit anorganischen und organischen Basen als Medikamente.

8. Produkte der Formel (I) entsprechend den Formeln (Ia) oder (Ib) nach den Ansprüchen 2 bis 4 sowie die pharmazeutisch annehmbaren Additionssalze der Produkte der Formeln (Ia) und (Ib) mit anorganischen und organischen Säuren oder mit anorganischen und organischen Basen als Medikamente.

9. Produkte der Formel (I) nach Anspruch 5 sowie die pharmazeutisch annehmbaren Additionssalze der Produkte der Formel (I) mit anorganischen und organischen Säuren oder mit anorganischen und organischen Basen als Medikamente.

10. Pharmazeutische Zusammensetzungen, die als Wirkstoff mindestens eines der Arzneimittel nach einem der Ansprüche 7 bis 9 enthalten.

11. Verwendung der Produkte der Formel (I) nach einem der Ansprüche 1 bis 5 oder der pharmazeutisch annehmbaren Salze der Produkte der Formel (I) zur Herstellung von Arzneimitteln zur Vorbeugung oder Behandlung von Erkrankungen, die mit einem physiologisch anomalen Verhalten auf der Ebene der Sekretion und/oder der Aktivität von Cysteinproteasen, wie insbesondere den Kathepsinen B, H, J, L, N, S, T, C, V, W, K oder O, 02, oder Papain einhergehen.

12. Verwendung nach Anspruch 11 zur Herstellung von Arzneimitteln zur Vorbeugung oder Behandlung von Erkrankungen, die mit einem physiologisch anomalen Verhalten auf der Ebene der Sekretion und/oder der Aktivität von Kathepsin K einhergehen.

13. Verwendung der Produkte der Formel (I) nach einem der Ansprüche 1 bis 5 oder der pharmazeutisch annehmbaren Salze der Produkte der Formel (I) zur Herstellung von Arzneimitteln zur Vorbeugung oder Behandlung von Stoffwechselerkrankungen des Knorpels oder Knochens, proliferativen Erkrankungen, Krebserkrankungen, Kardiovaskulärerkrankungen, Restenose, Erkrankungen des zentralen Nervensystems, Erkrankungen des Immunsystems, Allergien, Infektionskrankheiten, entzündlichen Erkrankungen und Autoimmunkrankheiten.

14. Verwendung nach Anspruch 13, **dadurch gekennzeichnet, dass** die Erkrankungen, denen vorgebeugt wird oder die behandelt werden, aus Stoffwechselerkrankungen des Knorpels oder Knochens, proliferativen Erkrankungen, Krebserkrankungen und neurodegenerativen Erkrankungen, wie Alzheimer-Krankheit, ausgewählt sind.

15. Verwendung nach Anspruch 13, **dadurch gekennzeichnet, dass** die Erkrankungen, denen vorgebeugt wird oder die behandelt werden, aus Stoffwechselerkrankungen des Knorpels oder Knochens und Knochenkrebserkrankungen ausgewählt sind.

16. Verwendung nach Anspruch 13, **dadurch gekennzeichnet, dass** es sich bei den Erkrankungen, denen vorgebeugt wird oder die behandelt werden, insbesondere um Osteoporose, Zahnfleischerkrankungen, einschließlich Gingivitis und Periodontose, Arthritis, wie insbesondere Osteoarthritis und rheumatoide Arthritis, Paget-Krankheit, Hyperkalzämie und Knochenkrebserkrankungen handelt.

17. Verwendung nach Anspruch 13, **dadurch gekennzeichnet, dass** es sich bei der Erkrankung, der vorgebeugt oder die behandelt wird, insbesondere um Osteoporose handelt.
